# EUROPEAN PATENT SPECIFICATION

(11) **EP 1 928 899 B1**
(45) Date of publication and mention of the grant of the patent: **24.10.2012**
(21) Application number: 06811161.6
(22) Date of filing: 27.09.2006
(51) Int. Cl.: C12P 13/22, C07K 14/24, C12P 13/08, C07K 14/195

(54) **AN L-AMINO ACID-PRODUCING BACTERIUM AND A METHOD FOR PRODUCING AN L-AMINO ACID**
L-AMINOSÄURE-PRODUZIERENDES BAKTERIUM UND VERFAHREN ZUR HERSTELLUNG EINER L-AMINOSÄURE
BACTÉRIE PRODUCTRICE D'ACIDE L-AMINÉ ET PROCÉDÉ DE PRODUCTION D'UN ACIDE L-AMINÉ

(30) Priority: 27.09.2005 JP 2005279027; 06.10.2005 US 723936 P; 04.08.2006 JP 2006213584
(43) Date of publication of application: 11.06.2008
(73) Proprietor: Ajinomoto Co., Inc., Tokyo 104-8315 (JP)
(72) Inventor: IWATANI, Shintaro, Kanagawa, 2108681 (JP); IMAIZUMI, Akira, Kamagawa, 2108681 (JP); USUDA, Yoshihiro, Kanagawa, 2108681 (JP); MATSUI, Kazuhiko, Kanagawa, 2108681 (JP)
(74) Representative: HOFFMANN EITLE
(86) International application number: PCT/JP2006/319821
(87) International publication number: WO 2007/037503

(56) References cited:
- WO-A-03/076637
- WO-A2-02/081722
- WO-A2-03/076636
- NISHINO K. EZ AL: "Global analyses of genes regulated by EvgA of the two-component regulatory system in Escherichia coli" JOURNAL OF BACTERIOLOGY, vol. 185, no. 8, April 2003 (2003-04), pages 2667-2672, XP009075766
- MA Z. ET AL.: "Characterization of EvgAS-YdeO-GadE branched regulatory circuit governing glutamate-dependent acid resistance in Escherichia coli" JOURNAL OF BACTERIOLOGY, vol. 186, no. 21, November 2004 (2004-11), pages 7378-7389, XP009075765

### SEQUENCE LISTING

<110> Ajinomoto Co., Inc.
<120> An L-Amino Acid Producing Bacterium and a Method for Producing an L-Amino Acid
<130> C557-C6193
<150> JP2005-279027
   <151> 2005-09-27
<150> US60/723936
   <151> 2005-10-06
<150> Jap2006-213584
   <151> 2006-08-04
<160> 38
<170> Patently version 3.1
<210> 1
   <211> 20
   <212> DNA
   <213> Artificial
<220>
   <223> primer
<400> 1
   gggttaattg tttttctgcg 20
<210> 2
   <211> 20
   <212> DNA
   <213> Artificial
<220>
   <223> primer
<400> 2
   cgcatctcga ctgcacggtg 20
<210> 3
   <211> 27
   <212> DNA
   <213> Artificial
<220>
   <223> primer
<400> 3
   gccgaattca agtgtgtgaa gtgtatg 27
<210> 4
   <211> 27
   <212> DNA
   <213> Artificial
<220>
   <223> primer
<400>. 4
   gccaagcttc cgacacgctc aacccag 27
<210> 5
   <211> 864
   <212> DNA
   <213> Escherichia coli
<220>
   <221> CDS
   <222> (1).. (864)
<400> 5
   atg aaa atg att tcc acg ata cag aaa aaa gag act gtc atg gtc gca     48
   Met Lys Met Ile Ser Thr Ile Gln Lys Lys Glu Thr Val Met Val Ala
   1               5                   10                  15
   ccc att ccc gcg aaa cgc ggc aga aaa ccc gcc gtt gcc acc gca cca     96
   Pro Ile Pro Ala Lys Arg Gly Arg Lys Pro Ala Val Ala Thr Ala Pro
               20                  25                  30
   gcg act gga cag gtt cag tct tta acg cgt ggc ctg aaa tta ctg gag     144
   Ala Thr Gly Gln Val Gln Ser Leu Thr Arg Gly Leu Lys Leu Leu Glu
           35                  40                  45
   tgg att gcc gaa tcc aat ggc agt gtg gca ctc acg gaa ctg gcg caa     192
   Trp Ile Ala Glu Ser Asn Gly Ser Val Ala Leu Thr Glu Leu Ala Gln
   50 55 60
   caa gcc ggg tta ccc aat tcc acg acc cac cgc ctg cta acc acg atg 240
   Gln Ala Gly Leu Pro Asn Ser Thr Thr His Arg Leu Leu Thr Thr Met
   65 70 75 80
   caa cag cag ggt ttc gtg cgt cag gtt ggc gaa ctg gga cat tgg gca 288
   Gln Gln Gln Gly Phe Val Arg Gln Val Gly Glu Leu Gly His Trp Ala
   85 90 95
   atc ggc gca cat gcc ttt atg gtc ggc agc agc ttt ctc cag agc cgt 336
   Ile Gly Ala His Ala Phe Met Val Gly Ser Ser Phe Leu Gln Ser Arg
   100 105 110
   aat ttg tta gcg att gtt cac cct atc ctg cgc aat cta atg gaa gag 384
   Asn Leu Leu Ala Ile Val His Pro Ile Leu Arg Asn Leu Met Glu Glu
   115 120 125
   tct ggc gaa acg gtc aat atg gcg gtg ctt gat caa agc gat cac gaa 432
   Ser Gly Glu Thr Val Asn Met Ala Val Leu Asp Gln Ser Asp His Glu
   130 135 140
   gcg att att atc gac cag gta cag tgt acg cat ctg atg cga atg tcc 480
   Ala Ile Ile Ile Asp Gln Val Gln Cys Thr His Leu Met Arg Met Ser
   145 150 155 160
   gcg cct atc ggc ggt aaa ttg ccg atg cac gct tcc ggt gcg ggt aaa 528
   Ala Pro Ile Gly Gly Lys Leu Pro Met His Ala Ser Gly Ala Gly Lys
   165 170 175
   gcc ttt tta gcc caa ctg agc gaa gaa cag gtg acg aag ctg ctg cac 576
   Ala Phe Leu Ala Gln Leu Ser Glu Glu Gln Val Thr Lys Leu Leu His
   180 185 190
   cgc aaa ggg tta cat gcc tat acc cac gca acg ctg gtg tct cct gtg 624
   Arg Lys Gly Leu His Ala Tyr Thr His Ala Thr Leu Val Ser Pro Val
   195 200 205
   cat tta aaa gaa gat ctc gcc caa acg cgc aaa cgg ggt tat tca ttt 672
   His Leu Lys Glu Asp Leu Ala Gln Thr Arg Lys Arg Gly Tyr Ser Phe
   210 215 220
   gac gat gag gaa cat gca ctg ggg cta cgt tgc ctt gca gcg tgt att 720
   Asp Asp Glu Glu His Ala Leu Gly Leu Arg Cys Leu Ala Ala Cys Ile
   225 230 235 240
   ttc gat gag cac cgt gaa ccg ttt gcc gca att tct att tcc gga ccg 768
   Phe Asp Glu His Arg Glu Pro Phe Ala Ala Ile Ser Ile Ser Gly Pro
   245 250 255
   att tca cgt att acc gat gac cgc gtg acc gag ttt ggc gcg atg gtg 816
   Ile Ser Arg Ile Thr Asp Asp Arg Val Thr Glu Phe Gly Ala Met Val
   260 265 270
   att aaa gcg gcg aag gaa gtg acg ctg gcg tac ggt gga atg cgc tga 864
   Ile Lys Ala Ala Lys Glu Val Thr Leu Ala Tyr Gly Gly Met Arg
   275 280 285
<210> 6
   <211> 287
   <212> PRT
   <213> Escherichia coli
<400> 6
   Met Lys Met Ile Ser Thr Ile Gln Lys Lys Glu Thr Val Met Val Ala
   1 5 10 15
   Pro Ile Pro Ala Lys Arg Gly Arg Lys Pro Ala Val Ala Thr Ala Pro
   20 25 30
   Ala Thr Gly Gln Val Gln Ser Leu Thr Arg Gly Leu Lys Leu Leu Glu
   35 40 45
   Trp Ile Ala Glu Ser Asn Gly Ser Val Ala Leu Thr Glu Leu Ala Gln
   50 55 60
   Gln Ala Gly Leu Pro Asn Ser Thr Thr His Arg Leu Leu Thr Thr Met
   65 70 75 80
   Gln Gln Gln Gly Phe Val Arg Gln Val Gly Glu Leu Gly His Trp Ala
   85 90 95
   Ile Gly Ala His Ala Phe Met Val Gly Ser Ser Phe Leu Gln Ser Arg
   100 105 110
   Asn Leu Leu Ala Ile Val His Pro Ile Leu Arg Asn Leu Met Glu Glu
   115 120 125
   Ser Gly Glu Thr Val Asn Met Ala Val Leu Asp Gln Ser Asp His Glu
   130 135 140
   Ala Ile Ile Ile Asp Gln Val Gln Cys Thr His Leu Met Arg Met Ser
   145 150 155 160
   Ala Pro Ile Gly Gly Lys Leu Pro Met His Ala Ser Gly Ala Gly Lys
   165 170 175
   Ala Phe Leu Ala Gln Leu Ser Glu Glu Gln Val Thr Lys Leu Leu His
   180 185 190
   Arg Lys Gly Leu His Ala Tyr Thr His Ala Thr Leu Val Ser Pro Val
   195 200 205
   His Leu Lys Glu Asp Leu Ala Gln Thr Arg Lys Arg Gly Tyr Ser Phe
   210 215 220
   Asp Asp Glu Glu His Ala Leu Gly Leu Arg Cys Leu Ala Ala Cys Ile
   225 230 235 240
   Phe Asp Glu His Arg Glu Pro Phe Ala Ala Ile Ser Ile Ser Gly Pro
   245 250 255
   Ile Ser Arg Ile Thr Asp Asp Arg Val Thr Glu Phe Gly Ala Met Val
   260 265 270
   Ile Lys Ala Ala Lys Glu Val Thr Leu Ala Tyr Gly Gly Met Arg
   275 280 285
<210> 7
   <211> 1305
   <212> DNA
   <213> Escherichia coli
<220>
   <221> CDS
   <222> (1)..(1305)
<400> 7
   atg aaa acc cgt aca caa caa att gaa gaa tta cag aaa gag tgg act 48
   Met Lys Thr Arg Thr Gln Gln Ile Glu Glu Leu Gln Lys Glu Trp Thr
   1 5 10 15
   caa ccg cgt tgg gaa ggc att act cgc cca tac agt gcg gaa gat gtg 96
   Gln Pro Arg Trp Glu Gly Ile Thr Arg Pro Tyr Ser Ala Glu Asp Val
   20 25 30
   gtg aaa tta cgc ggt tca gtc aat cct gaa tgc acg ctg gcg caa ctg 144
   Val Lys Leu Arg Gly Ser Val Asn Pro Glu Cys Thr Leu Ala Gln Leu
   35 40 45
   ggc gca gcg aaa atg tgg cgt ctg ctg cac ggt gag tcg aaa aaa ggc 192
   Gly Ala Ala Lys Met Trp Arg Leu Leu His Gly Glu Ser Lys Lys Gly
   50 55 60
   tac atc aac agc ctc ggc gca ctg act ggc ggt cag gcg ctg caa cag 240
   Tyr Ile Asn Ser Leu Gly Ala Leu Thr Gly Gly Gln Ala Leu Gln Gln
   65 70 75 80
   gcg aaa gcg ggt att gaa gca gtc tat ctg tcg gga tgg cag gta gcg 288
   Ala Lys Ala Gly Ile Glu Ala Val Tyr Leu Ser Gly Trp Gln Val Ala
   85 90 95
   gcg gac gct aac ctg gcg gcc agc atg tat ccg gat cag tcg ctc tat 336
   Ala Asp Ala Asn Leu Ala Ala Ser Met Tyr Pro Asp Gln Ser Leu Tyr
   100 105 110
   ccg gca aac tcg gtg cca gct gtg gtg gag cgg atc aac aac acc ttc     384
   Pro Ala Asn Ser Val Pro Ala Val Val Glu Arg Ile Asn Asn Thr Phe
           115                 120                 125
   cgt cgt gcc gat cag atc caa tgg tcc gcg ggc att gag ccg ggc gat     432
   Arg Arg Ala Asp Gln Ile Gln Trp Ser Ala Gly Ile Glu Pro Gly Asp
       130                 135                 140
   ccg cgc tat gtc gat tac ttc ctg ccg atc gtt gcc gat gcg gaa gcc     480
   Pro Arg Tyr Val Asp Tyr Phe Leu Pro Ile Val Ala Asp Ala Glu Ala
   145                 150                 155                 160
   ggt ttt ggc ggt gtc ctg aat gcc ttt gaa ctg atg aaa gcg atg att     528
   Gly Phe Gly Gly Val Leu Asn Ala Phe Glu Leu Met Lys Ala Met Ile
                   165                 170                 175
   gaa gcc ggt gca gcg gca gtt cac ttc gaa gat cag ctg gcg tca gtg     576
   Glu Ala Gly Ala Ala Ala Val His Phe Glu Asp Gln Leu Ala Ser Val
               180                 185                 190
   aag aaa tgc ggt cac atg ggc ggc aaa gtt tta gtg cca act cag gaa     624
   Lys Lys Cys Gly His Met Gly Gly Lys Val Leu Val Pro Thr Gln Glu
           195                 200                 205
   gct att cag aaa ctg gtc gcg gcg cgt ctg gca gct gac gtg acg ggc     672
   Ala Ile Gln Lys Leu Val Ala Ala Arg Leu Ala Ala Asp Val Thr Gly
       210                 215                 220
   gtt cca acc ctg ctg gtt gcc cgt acc gat gct gat gcg gcg gat ctg     720
   Val Pro Thr Leu Leu Val Ala Arg Thr Asp Ala Asp Ala Ala Asp Leu
   225                 230                 235                 240
   atc acc tcc gat tgc gac ccg tat gac agc gaa ttt att acc ggc gag     768
   Ile Thr Ser Asp Cys Asp Pro Tyr Asp Ser Glu Phe Ile Thr Gly Glu
                   245                 250                 255
   cgt acc agt gaa ggc ttc ttc cgt act cat gcg ggc att gag caa gcg     816
   Arg Thr Ser Glu Gly Phe Phe Arg Thr His Ala Gly Ile Glu Gln Ala
               260                 265                 270
   atc agc cgt ggc ctg gcg tat gcg cca tat gct gac ctg gtc tgg tgt     864
   Ile Ser Arg Gly Leu Ala Tyr Ala Pro Tyr Ala Asp Leu Val Trp Cys
           275                 280                 285
   gaa acc tcc acg ccg gat ctg gaa ctg gcg cgt cgc ttt gca caa gct     912
   Glu Thr Ser Thr Pro Asp Leu Glu Leu Ala Arg Arg Phe Ala Gln Ala
       290                 295                 300
   atc cac gcg aaa tat ccg ggc aaa ctg ctg gct tat aac tgc tcg ccg      960
   Ile His Ala Lys Tyr Pro Gly Lys Leu Leu Ala Tyr Asn Cys Ser Pro
   305                 310                 315                 320
   tcg ttc aac tgg cag aaa aac ctc gac gac aaa act att gcc agc ttc      1008
   Ser Phe Asn Trp Gln Lys Asn Leu Asp Asp Lys Thr Ile Ala Ser Phe
                   325                 330                 335
   cag cag cag ctg tcg gat atg ggc tac aag ttc cag ttc atc acc ctg      1056
   Gln Gln Gln Leu Ser Asp Met Gly Tyr Lys Phe Gln Phe Ile Thr Leu
               340                 345                 350
   gca ggt atc cac agc atg tgg ttc aac atg ttt gac ctg gca aac gcc      1104
   Ala Gly Ile His Ser Met Trp Phe Asn Met Phe Asp Leu Ala Asn Ala
           355                 360                 365
   tat gcc cag ggc gag ggt atg aag cac tac gtt gag aaa gtg cag cag      1152
   Tyr Ala Gln Gly Glu Gly Met Lys His Tyr Val Glu Lys Val Gln Gln
       370                 375                 380
   ccg gaa ttt gcc gcc gcg aaa gat ggc tat acc ttc gta tct cac cag      1200
   Pro Glu Phe Ala Ala Ala Lys Asp Gly Tyr Thr Phe Val Ser His Gln
   385                 390                 395                 400
   cag gaa gtg ggt aca ggt tac ttc gat aaa gtg acg act att att cag      1248
   Gln Glu Val Gly Thr Gly Tyr Phe Asp Lys Val Thr Thr Ile Ile Gln
                   405                 410                 415
   ggc ggc acg tct tca gtc acc gcg ctg acc ggc tcc act gaa gaa tcg      1296
   Gly Gly Thr Ser Ser Val Thr Ala Leu Thr Gly Ser Thr Glu Glu Ser
               420                 425                 430
   cag ttc taa                                                          1305
   Gln Phe
<210> 8
   <211> 434
   z<212> PRT
<213> Escherichia coli
<400> 8
   Met Lys Thr Arg Thr Gln Gln Ile Glu Glu Leu Gln Lys Glu Trp Thr
   1               5                   10                  15
   Gln Pro Arg Trp Glu Gly Ile Thr Arg Pro Tyr Ser Ala Glu Asp Val
   20 25 30
   Val Lys Leu Arg Gly Ser Val Asn Pro Glu Cys Thr Leu Ala Gln Leu
   35 40 45
   Gly Ala Ala Lys Met Trp Arg Leu Leu His Gly Glu Ser Lys Lys Gly
   50 55 60
   Tyr Ile Asn Ser Leu Gly Ala Leu Thr Gly Gly Gln Ala Leu Gln Gln
   65 70 75 80
   Ala Lys Ala Gly Ile Glu Ala Val Tyr Leu Ser Gly Trp Gln Val Ala
   85 90 95
   Ala Asp Ala Asn Leu Ala Ala Ser Met Tyr Pro Asp Gln Ser Leu Tyr
   100 105 110
   Pro Ala Asn Ser Val Pro Ala Val Val Glu Arg Ile Asn Asn Thr Phe
   115 120 125
   Arg Arg Ala Asp Gln Ile Gln Trp Ser Ala Gly Ile Glu Pro Gly Asp
   130 135 140
   Pro Arg Tyr Val Asp Tyr Phe Leu Pro Ile Val Ala Asp Ala Glu Ala
   145 150 155 160
   Gly Phe Gly Gly Val Leu Asn Ala Phe Glu Leu Met Lys Ala Met Ile
   165 170 175
   Glu Ala Gly Ala Ala Ala Val His Phe Glu Asp Gln Leu Ala Ser Val
   180 185 190
   Lys Lys Cys Gly His Met Gly Gly Lys Val Leu Val Pro Thr Gln Glu
   195 200 205
   Ala Ile Gln Lys Leu Val Ala Ala Arg Leu Ala Ala Asp Val Thr Gly
   210 215 220
   Val Pro Thr Leu Leu Val Ala Arg Thr Asp Ala Asp Ala Ala Asp Leu
   225 230 235 240
   Ile Thr Ser Asp Cys Asp Pro Tyr Asp Ser Glu Phe Ile Thr Gly Glu
   245 250 255
   Arg Thr Ser Glu Gly Phe Phe Arg Thr His Ala Gly Ile Glu Gln Ala
   260 265 270
   Ile Ser Arg Gly Leu Ala Tyr Ala Pro Tyr Ala Asp Leu Val Trp Cys
   275 280 285
   Glu Thr Ser Thr Pro Asp Leu Glu Leu Ala Arg Arg Phe Ala Gln Ala
   290 295 300
   Ile His Ala Lys Tyr Pro Gly Lys Leu Leu Ala Tyr Asn Cys Ser Pro
   305 310 315 320
   Ser Phe Asn Trp Gln Lys Asn Leu Asp Asp Lys Thr Ile Ala Ser Phe
   325 330 335
   Gln Gln Gln Leu Ser Asp Met Gly Tyr Lys Phe Gln Phe Ile Thr Leu
   340 345 350
   Ala Gly Ile His Ser Met Trp Phe Asn Met Phe Asp Leu Ala Asn Ala
   355 360 365
   Tyr Ala Gln Gly Glu Gly Met Lys His Tyr Val Glu Lys Val Gln Gln
   370 375 380
   Pro Glu Phe Ala Ala Ala Lys Asp Gly Tyr Thr Phe Val Ser His Gln
   385 390 395 400
   Gln Glu Val Gly Thr Gly Tyr Phe Asp Lys Val Thr Thr Ile Ile Gln
   405 410 415
   Gly Gly Thr Ser Ser Val Thr Ala Leu Thr Gly Ser Thr Glu Glu Ser
   420 425 430
   Gln Phe
<210> 9
   <211> 1602
   <212> DNA
   <213> Escherichia coli
<220>
   <221> CDS
   <222> (1)..(1602)
<400> 9
   atg act gaa cag gca aca aca acc gat gaa ctg gct ttc aca agg ccg 48
   Met Thr Glu Gln Ala Thr Thr Thr Asp Glu Leu Ala Phe Thr Arg Pro
   1 5 10 15
   tat ggc gag cag gag aag caa att ctt act gcc gaa gcg gta gaa ttt 96
   Tyr Gly Glu Gln Glu Lys Gln Ile Leu Thr Ala Glu Ala Val Glu Phe
   20 25 30
   ctg act gag ctg gtg acg cat ttt acg cca caa cgc aat aaa ctt ctg 144
   Leu Thr Glu Leu Val Thr His Phe Thr Pro Gln Arg Asn Lys Leu Leu
   35 40 45
   gca gcg cgc att cag cag cag caa gat att gat aac gga acg ttg cct 192
   Ala Ala Arg Ile Gln Gln Gln Gln Asp Ile Asp Asn Gly Thr Leu Pro
   50 55 60
   gat ttt att tcg gaa aca gct tcc att cgc gat gct gat tgg aaa att 240
   Asp Phe Ile Ser Glu Thr Ala Ser Ile Arg Asp Ala Asp Trp Lys Ile
   65 70 75 80
   cgc ggg att cct gcg gac tta gaa gac cgc cgc gta gag ata act ggc 288
   Arg Gly Ile Pro Ala Asp Leu Glu Asp Arg Arg Val Glu Ile Thr Gly
   85 90 95
   ccg gta gag cgc aag atg gtg atc aac gcg ctc aac gcc aat gtg aaa 336
   Pro Val Glu Arg Lys Met Val Ile Asn Ala Leu Asn Ala Asn Val Lys
   100 105 110
   gtc ttt atg gcc gat ttc gaa gat tca ctg gca cca gac tgg aac aaa 384
   Val Phe Met Ala Asp Phe Glu Asp Ser Leu Ala Pro Asp Trp Asn Lys
   115 120 125
   gtg atc gac ggg caa att aac ctg cgt gat gcg gtt aac ggc acc atc 432
   Val Ile Asp Gly Gln Ile Asn Leu Arg Asp Ala Val Asn Gly Thr Ile
   130 135 140
   agt tac acc aat gaa gca ggc aaa att tac cag ctc aag ccc aat cca 480
   Ser Tyr Thr Asn Glu Ala Gly Lys Ile Tyr Gln Leu Lys Pro Asn Pro
   145 150 155 160
   gcg gtt ttg att tgt cgg gta cgc ggt ctg cac ttg ccg gaa aaa cat 528
   Ala Val Leu Ile Cys Arg Val Arg Gly Leu His Leu Pro Glu Lys His
   165 170 175
   gtc acc tgg cgt ggt gag gca atc ccc ggc agc ctg ttt gat ttt gcg 576
   Val Thr Trp Arg Gly Glu Ala Ile Pro Gly Ser Leu Phe Asp Phe Ala
   180 185 190
   ctc tat ttc ttc cac aac tat cag gca ctg ttg gca aag ggc agt ggt 624
   Leu Tyr Phe Phe His Asn Tyr Gln Ala Leu Leu Ala Lys Gly Ser Gly
   195 200 205
   ccc tat ttc tat ctg ccg aaa acc cag tcc tgg cag gaa gcg gcc tgg 672
   Pro Tyr Phe Tyr Leu Pro Lys Thr Gln Ser Trp Gln Glu Ala Ala Trp
   210 215 220
   tgg agc gaa gtc ttc agc tat gca gaa gat cgc ttt aat ctg ccg cgc 720
   Trp Ser Glu Val Phe Ser Tyr Ala Glu Asp Arg Phe Asn Leu Pro Arg
   225 230 235 240
   ggc acc atc aag gcg acg ttg ctg att gaa acg ctg ccc gcc gtg ttc 768
   Gly Thr Ile Lys Ala Thr Leu Leu Ile Glu Thr Leu Pro Ala Val Phe
   245 250 255
   cag atg gat gaa atc ctt cac gcg ctg cgt gac cat att gtt ggt ctg 816
   Gln Met Asp Glu Ile Leu His Ala Leu Arg Asp His Ile Val Gly Leu
   260 265 270
   aac tgc ggt cgt tgg gat tac atc ttc agc tat atc aaa acg ttg aaa 864
   Asn Cys Gly Arg Trp Asp Tyr Ile Phe Ser Tyr Ile Lys Thr Leu Lys
   275 280 285
   aac tat ccc gat cgc gtc ctg cca gac aga cag gca gtg acg atg gat 912
   Asn Tyr Pro Asp Arg Val Leu Pro Asp Arg Gln Ala Val Thr Met Asp
   290 295 300
   aaa cca ttc ctg aat gct tac tca cgc ctg ttg att aaa acc tgc cat 960
   Lys Pro Phe Leu Asn Ala Tyr Ser Arg Leu Leu Ile Lys Thr Cys His
   305 310 315 320
   aaa cgc ggt gct ttt gcg atg ggc ggc atg gcg gcg ttt att ccg agc 1008
   Lys Arg Gly Ala Phe Ala Met Gly Gly Met Ala Ala Phe Ile Pro Ser
   325 330 335
   aaa gat gaa gag cac aat aac cag gtg ctc aac aaa gta aaa gcg gat 1056
   Lys Asp Glu Glu His Asn Asn Gln Val Leu Asn Lys Val Lys Ala Asp
   340 345 350
   aaa tcg ctg gaa gcc aat aac ggt cac gat ggc aca tgg atc gct cac 1104
   Lys Ser Leu Glu Ala Asn Asn Gly His Asp Gly Thr Trp Ile Ala His
   355 360 365
   cca ggc ctt gcg gac acg gca atg gcg gta ttc aac gac att ctc ggc 1152
   Pro Gly Leu Ala Asp Thr Ala Met Ala Val Phe Asn Asp Ile Leu Gly
   370 375 380
   tcc cgt aaa aat cag ctt gaa gtg atg cgc gaa caa gac gcg ccg att 1200
   Ser Arg Lys Asn Gln Leu Glu Val Met Arg Glu Gln Asp Ala Pro Ile
   385 390 395 400
   act gcc gat cag ctg ctg gca cct tgt gat ggt gaa cgc acc gaa gaa 1248
   Thr Ala Asp Gln Leu Leu Ala Pro Cys Asp Gly Glu Arg Thr Glu Glu
   405 410 415
   ggt atg cgc gcc aac att cgc gtg gct gtg cag tac atc gaa gcg tgg 1296
   Gly Met Arg Ala Asn Ile Arg Val Ala Val Gln Tyr Ile Glu Ala Trp
   420 425 430
   atc tct ggc aac ggc tgt gtg ccg att tat ggc ctg atg gaa gat gcg 1344
   Ile Ser Gly Asn Gly Cys Val Pro Ile Tyr Gly Leu Met Glu Asp Ala
   435 440 445
   gcg acg gct gaa att tcc cgt acc tcg atc tgg cag tgg atc cat cat 1392
   Ala Thr Ala Glu Ile Ser Arg Thr Ser Ile Trp Gln Trp Ile His His
   450 455 460
   caa aaa acg ttg agc aat ggc aaa ccg gtg acc aaa gcc ttg ttc cgc 1440
   Gln Lys Thr Leu Ser Asn Gly Lys Pro Val Thr Lys Ala Leu Phe Arg
   465 470 475 480
   cag atg ctg ggc gaa gag atg aaa gtc att gcc agc gaa ctg ggc gaa 1488
   Gln Met Leu Gly Glu Glu Met Lys Val Ile Ala Ser Glu Leu Gly Glu
   485 490 495
   gaa cgt ttc tcc cag ggg cgt ttt gac gat gcc gca cgc ttg atg gaa 1536
   Glu Arg Phe Ser Gln Gly Arg Phe Asp Asp Ala Ala Arg Leu Met Glu
   500 505 510
   cag atc acc act tcc gat gag tta att gat ttc ctg acc ctg cca ggc 1584
   Gln Ile Thr Thr Ser Asp Glu Leu Ile Asp Phe Leu Thr Leu Pro Gly
   515 520 525
   tac cgc ctg tta gcg taa 1602
   Tyr Arg Leu Leu Ala
   530
<210> 10
   <211> 533
   <212> PRT
   <213> Escherichia coli
<400> 10
   Met Thr Glu Gln Ala Thr Thr Thr Asp Glu Leu Ala Phe Thr Arg Pro
   1 5 10 15
   Tyr Gly Glu Gln Glu Lys Gln Ile Leu Thr Ala Glu Ala Val Glu Phe
   20 25 30
   Leu Thr Glu Leu Val Thr His Phe Thr Pro Gln Arg Asn Lys Leu Leu
   35 40 45
   Ala Ala Arg Ile Gln Gln Gln Gln Asp Ile Asp Asn Gly Thr Leu Pro
   50 55 60
   Asp Phe Ile Ser Glu Thr Ala Ser Ile Arg Asp Ala Asp Trp Lys Ile
   65 70 75 80
   Arg Gly Ile Pro Ala Asp Leu Glu Asp Arg Arg Val Glu Ile Thr Gly
   85 90 95
   Pro Val Glu Arg Lys Met Val Ile Asn Ala Leu Asn Ala Asn Val Lys
   100 105 110
   Val Phe Met Ala Asp Phe Glu Asp Ser Leu Ala Pro Asp Trp Asn Lys
   115 120 125
   Val Ile Asp Gly Gln Ile Asn Leu Arg Asp Ala Val Asn Gly Thr Ile
   130 135 140
   Ser Tyr Thr Asn Glu Ala Gly Lys Ile Tyr Gln Leu Lys Pro Asn Pro
   145 150 155 160
   Ala Val Leu Ile Cys Arg Val Arg Gly Leu His Leu Pro Glu Lys His
   165 170 175
   Val Thr Trp Arg Gly Glu Ala Ile Pro Gly Ser Leu Phe Asp Phe Ala
   180 185 190
   Leu Tyr Phe Phe His Asn Tyr Gln Ala Leu Leu Ala Lys Gly Ser Gly
   195 200 205
   Pro Tyr Phe Tyr Leu Pro Lys Thr Gln Ser Trp Gln Glu Ala Ala Trp
   210 215 220
   Trp Ser Glu Val Phe Ser Tyr Ala Glu Asp Arg Phe Asn Leu Pro Arg
   225 230 235 240
   Gly Thr Ile Lys Ala Thr Leu Leu Ile Glu Thr Leu Pro Ala Val Phe
   245 250 255
   Gln Met Asp Glu Ile Leu His Ala Leu Arg Asp His Ile Val Gly Leu
   260 265 270
   Asn Cys Gly Arg Trp Asp Tyr Ile Phe Ser Tyr Ile Lys Thr Leu Lys
   275 280 285
   Asn Tyr Pro Asp Arg Val Leu Pro Asp Arg Gln Ala Val Thr Met Asp
   290 295 300
   Lys Pro Phe Leu Asn Ala Tyr Ser Arg Leu Leu Ile Lys Thr Cys His
   305 310 315 320
   Lys Arg Gly Ala Phe Ala Met Gly Gly Met Ala Ala Phe Ile Pro Ser
   325 330 335
   Lys Asp Glu Glu His Asn Asn Gln Val Leu Asn Lys Val Lys Ala Asp
   340 345 350
   Lys Ser Leu Glu Ala Asn Asn Gly His Asp Gly Thr Trp Ile Ala His
   355 360 365
   Pro Gly Leu Ala Asp Thr Ala Met Ala Val Phe Asn Asp Ile Leu Gly
   370 375 380
   Ser Arg Lys Asn Gln Leu Glu Val Met Arg Glu Gln Asp Ala Pro Ile
   385 390 395 400
   Thr Ala Asp Gln Leu Leu Ala Pro Cys Asp Gly Glu Arg Thr Glu Glu
   405 410 415
   Gly Met Arg Ala Asn Ile Arg Val Ala Val Gln Tyr Ile Glu Ala Trp
   420 425 430
   Ile Ser Gly Asn Gly Cys Val Pro Ile Tyr Gly Leu Met Glu Asp Ala
   435 440 445
   Ala Thr Ala Glu Ile Ser Arg Thr Ser Ile Trp Gln Trp Ile His His
   450 455 460
   Gln Lys Thr Leu Ser Asn Gly Lys Pro Val Thr Lys Ala Leu Phe Arg
   465 470 475 480
   Gln Met Leu Gly Glu Glu Met Lys Val Ile Ala Ser Glu Leu Gly Glu
   485 490 495
   Glu Arg Phe Ser Gln Gly Arg Phe Asp Asp Ala Ala Arg Leu Met Glu
   500 505 510
   Gln Ile Thr Thr Ser Asp Glu Leu Ile Asp Phe Leu Thr Leu Pro Gly
   515 520 525
   Tyr Arg Leu Leu Ala
   530
<210> 11
   <211> 1737
   <212> DNA
   <213> Escherichia coli
<220>
   <221> CDS
   <222> (1)..(1737)
<400> 11
   atg ccg cgt ggc ctg gaa tta ttg att gct caa acc att ttg caa ggc      48
   Met Pro Arg Gly Leu Glu Leu Leu Ile Ala Gln Thr Ile Leu Gln Gly
   1               5                   10                  15
   ttc gat gct cag tat ggt cga ttc ctc gaa gtg acc tcc ggt gcg cag      96
   Phe Asp Ala Gln Tyr Gly Arg Phe Leu Glu Val Thr Ser Gly Ala Gln
               20                  25                  30
   cag cgt ttc gaa cag gcc gac tgg cat gct gtc cag cag gcg atg aaa      144
   Gln Arg Phe Glu Gln Ala Asp Trp His Ala Val Gln Gln Ala Met Lys
           35                  40                  45
   aac cgt atc cat ctt tac gat cat cac gtt ggt ctg gtc gtg gag caa      192
   Asn Arg Ile His Leu Tyr Asp His His Val Gly Leu Val Val Glu Gln
       50                  55                  60
   ctg cgc tgc att act aac ggc caa agt acg gac gcg gca ttt tta cta      240
   Leu Arg Cys Ile Thr Asn Gly Gln Ser Thr Asp Ala Ala Phe Leu Leu
   65                  70                  75                  80
   cgt gtt aaa gag cat tac acc cgg ctg ttg ccg gat tac ccg cgc ttc      288
   Arg Val Lys Glu His Tyr Thr Arg Leu Leu Pro Asp Tyr Pro Arg Phe
                   85                  90                  95
   gag att gcg gag agc ttt ttt aac tcc gtg tac tgt cgg tta ttt gac      336
   Glu Ile Ala Glu Ser Phe Phe Asn Ser Val Tyr Cys Arg Leu Phe Asp
               100                 105                 110
   cac cgc tcg ctt act ccc gag cgg ctt ttt atc ttt agc tct cag cca      384
   His Arg Ser Leu Thr Pro Glu Arg Leu Phe Ile Phe Ser Ser Gln Pro
           115                 120                 125
   gag cgc cgc ttt cgt acc att ccc cgc ccg ctg gcg aaa gac ttt cac      432
   Glu Arg Arg Phe Arg Thr Ile Pro Arg Pro Leu Ala Lys Asp Phe His
       130                 135                 140
   ccc gat cac ggc tgg gaa tct cta ctg atg cgc gtt atc agc gac cta      480
   Pro Asp His Gly Trp Glu Ser Leu Leu Met Arg Val Ile Ser Asp Leu
   145                 150                 155                 160
   ccg ctg cgc ctg cgc tgg cag aat aaa agc cgt gac atc cat tac att      528
   Pro Leu Arg Leu Arg Trp Gln Asn Lys Ser Arg Asp Ile His Tyr Ile
   165 170 175
   att cgc cat ctg acg gaa acg ctg ggg aca gac aac ctc gcg gaa agt 576
   Ile Arg His Leu Thr Glu Thr Leu Gly Thr Asp Asn Leu Ala Glu Ser
   180 185 190
   cat tta cag gtg gcg aac gaa ctg ttt tac cgc aat aaa gcc gcc tgg 624
   His Leu Gln Val Ala Asn Glu Leu Phe Tyr Arg Asn Lys Ala Ala Trp
   195 200 205
   ctg gta ggc aaa ctg atc aca cct tcc ggc aca ttg cca ttt ttg ctg 672
   Leu Val Gly Lys Leu Ile Thr Pro Ser Gly Thr Leu Pro Phe Leu Leu
   210 215 220
   ccg atc cac cag acg gac gac ggc gag tta ttt att gat acc tgc ctg 720
   Pro Ile His Gln Thr Asp Asp Gly Glu Leu Phe Ile Asp Thr Cys Leu
   225 230 235 240
   acg acg acc gcc gaa gcg agc att gtt ttt ggc ttt gcg cgt tct tat 768
   Thr Thr Thr Ala Glu Ala Ser Ile Val Phe Gly Phe Ala Arg Ser Tyr
   245 250 255
   ttt atg gtt tat gcg ccg ctg ccc gca gcg ctg gtc gag tgg cta cgg 816
   Phe Met Val Tyr Ala Pro Leu Pro Ala Ala Leu Val Glu Trp Leu Arg
   260 265 270
   gaa att ctg cca ggt aaa acc acc gct gaa ttg tat atg gct atc ggc 864
   Glu Ile Leu Pro Gly Lys Thr Thr Ala Glu Leu Tyr Met Ala Ile Gly
   275 280 285
   tgc cag aag cac gcc aaa acc gaa agc tac cgc gaa tat ctc gtt tat 912
   Cys Gln Lys His Ala Lys Thr Glu Ser Tyr Arg Glu Tyr Leu Val Tyr
   290 295 300
   cta cag ggc tgt aat gag cag ttc att gaa gcg ccg ggt att cgt gga 960
   Leu Gln Gly Cys Asn Glu Gln Phe Ile Glu Ala Pro Gly Ile Arg Gly
   305 310 315 320
   atg gtg atg ttg gtg ttt acg ctg ccg ggc ttt gat cgg gta ttc aaa 1008
   Met Val Met Leu Val Phe Thr Leu Pro Gly Phe Asp Arg Val Phe Lys
   325 330 335
   gtc atc aaa gac agg ttc gcg ccg cag aaa gag atg tct gcc gct cac 1056
   Val Ile Lys Asp Arg Phe Ala Pro Gln Lys Glu Met Ser Ala Ala His
   340 345 350
   gtt cgt gcc tgc tat caa ctg gtg aaa gag cac gat cgc gtg ggc cga 1104
   Val Arg Ala Cys Tyr Gln Leu Val Lys Glu His Asp Arg Val Gly Arg
   355 360 365
   atg gcg gac acc cag gag ttt gaa aac ttt gtg ctg gag aag cgg cat 1152
   Met Ala Asp Thr Gln Glu Phe Glu Asn Phe Val Leu Glu Lys Arg His
   370 375 380
   att tcc ccg gca tta atg gaa tta ctg ctt cag gaa gca gcg gaa aaa 1200
   Ile Ser Pro Ala Leu Met Glu Leu Leu Leu Gln Glu Ala Ala Glu Lys
   385 390 395 400
   atc acc gat ctc ggc gaa caa att gtg att cgc cat ctt tat att gag 1248
   Ile Thr Asp Leu Gly Glu Gln Ile Val Ile Arg His Leu Tyr Ile Glu
   405 410 415
   cgg cgg atg gtg ccg ctc aat atc tgg ctg gaa caa gtg gaa ggt cag 1296
   Arg Arg Met Val Pro Leu Asn Ile Trp Leu Glu Gln Val Glu Gly Gln
   420 425 430
   cag ttg cgc gac gcc att gaa gaa tac ggt aac gct att cgc cag ctt 1344
   Gln Leu Arg Asp Ala Ile Glu Glu Tyr Gly Asn Ala Ile Arg Gln Leu
   435 440 445
   gcc gct gct aac att ttc cct ggc gac atg ctg ttt aaa aac ttc ggt 1392
   Ala Ala Ala Asn Ile Phe Pro Gly Asp Met Leu Phe Lys Asn Phe Gly
   450 455 460
   gtc acc cgt cac ggg cgt gtg gtt ttt tat gat tac gat gaa att tgc 1440
   Val Thr Arg His Gly Arg Val Val Phe Tyr Asp Tyr Asp Glu Ile Cys
   465 470 475 480
   tac atg acg gaa gtg aat ttc cgc gac atc ccg ccg ccg cgc tat ccg 1488
   Tyr Met Thr Glu Val Asn Phe Arg Asp Ile Pro Pro Pro Arg Tyr Pro
   485 490 495
   gaa gac gaa ctt gcc agc gaa ccg tgg tac agc gtc tcg ccg ggc gat 1536
   Glu Asp Glu Leu Ala Ser Glu Pro Trp Tyr Ser Val Ser Pro Gly Asp
   500 505 510
   gtt ttc ccg gaa gag ttt cgc cac tgg cta tgc gcc gac ccg cgt att 1584
   Val Phe Pro Glu Glu Phe Arg His Trp Leu Cys Ala Asp Pro Arg Ile
   515 520 525
   ggt ccg ctg ttt gaa gag atg cac gcc gac ctg ttc cgc gct gat tac 1632
   Gly Pro Leu Phe Glu Glu Met His Ala Asp Leu Phe Arg Ala Asp Tyr
   530 535 540
   tgg cgc gca cta caa aac cgc ata cgt gaa ggg cat gtg gaa gat gtt     1680
   Trp Arg Ala Leu Gln Asn Arg Ile Arg Glu Gly His Val Glu Asp Val
   545                 550                 555                 560
   tat gcg tat cgg cgc agg caa aga ttt agc gta cgg tat ggg gag atg     1728
   Tyr Ala Tyr Arg Arg Arg Gln Arg Phe Ser Val Arg Tyr Gly Glu Met
                   565                 570                 575
   ctt ttt tga                                                         1737
   Leu Phe
<210> 12
   <211> 578
   <212> PRT
   <213> Escherichia coli
<400> 12
   Met Pro Arg Gly Leu Glu Leu Leu Ile Ala Gln Thr Ile Leu Gln Gly
   1               5                   10                  15
   Phe Asp Ala Gln Tyr Gly Arg Phe Leu Glu Val Thr Ser Gly Ala Gln
               20                  25                  30
   Gln Arg Phe Glu Gln Ala Asp Trp His Ala Val Gln Gln Ala Met Lys
           35                  40                  45
   Asn Arg Ile His Leu Tyr Asp His His Val Gly Leu Val Val Glu Gln
       50                  55                  60
   Leu Arg Cys Ile Thr Asn Gly Gln Ser Thr Asp Ala Ala Phe Leu Leu
   65                  70                  75                  80
   Arg Val Lys Glu His Tyr Thr Arg Leu Leu Pro Asp Tyr Pro Arg Phe
                   85                  90                  95
   Glu Ile Ala Glu Ser Phe Phe Asn Ser Val Tyr Cys Arg Leu Phe Asp
               100                 105                 110
   His Arg Ser Leu Thr Pro Glu Arg Leu Phe Ile Phe Ser Ser Gln Pro
           115                 120                 125
   Glu Arg Arg Phe Arg Thr Ile Pro Arg Pro Leu Ala Lys Asp Phe His
       130                 135                 140
   Pro Asp His Gly Trp Glu Ser Leu Leu Met Arg Val Ile Ser Asp Leu
   145                 150                 155                 160
   Pro Leu Arg Leu Arg Trp Gln Asn Lys Ser Arg Asp Ile His Tyr Ile
                   165                 170                 175
   Ile Arg His Leu Thr Glu Thr Leu Gly Thr Asp Asn Leu Ala Glu Ser
               180                 185                 190
   His Leu Gln Val Ala Asn Glu Leu Phe Tyr Arg Asn Lys Ala Ala Trp
           195                 200                 205
   Leu Val Gly Lys Leu Ile Thr Pro Ser Gly Thr Leu Pro Phe Leu Leu
       210                 215                 220
   Pro Ile His Gln Thr Asp Asp Gly Glu Leu Phe Ile Asp Thr Cys Leu
   225 230 235 240
   Thr Thr Thr Ala Glu Ala Ser Ile Val Phe Gly Phe Ala Arg Ser Tyr
   245 250 255
   Phe Met Val Tyr Ala Pro Leu Pro Ala Ala Leu Val Glu Trp Leu Arg
   260 265 270
   Glu Ile Leu Pro Gly Lys Thr Thr Ala Glu Leu Tyr Met Ala Ile Gly
   275 280 285
   Cys Gln Lys His Ala Lys Thr Glu Ser Tyr Arg Glu Tyr Leu Val Tyr
   290 295 300
   Leu Gln Gly Cys Asn Glu Gln Phe Ile Glu Ala Pro Gly Ile Arg Gly
   305 310 315 320
   Met Val Met Leu Val Phe Thr Leu Pro Gly Phe Asp Arg Val Phe Lys
   325 330 335
   Val Ile Lys Asp Arg Phe Ala Pro Gln Lys Glu Met Ser Ala Ala His
   340 345 350
   Val Arg Ala Cys Tyr Gln Leu Val Lys Glu His Asp Arg Val Gly Arg
   355 360 365
   Met Ala Asp Thr Gln Glu Phe Glu Asn Phe Val Leu Glu Lys Arg His
   370 375 380
   Ile Ser Pro Ala Leu Met Glu Leu Leu Leu Gln Glu Ala Ala Glu Lys
   385 390 395 400
   Ile Thr Asp Leu Gly Glu Gln Ile Val Ile Arg His Leu Tyr Ile Glu
   405 410 415
   Arg Arg Met Val Pro Leu Asn Ile Trp Leu Glu Gln Val Glu Gly Gln
   420 425 430
   Gln Leu Arg Asp Ala Ile Glu Glu Tyr Gly Asn Ala Ile Arg Gln Leu
   435 440 445
   Ala Ala Ala Asn Ile Phe Pro Gly Asp Met Leu Phe Lys Asn Phe Gly
   450 455 460
   Val Thr Arg His Gly Arg Val Val Phe Tyr Asp Tyr Asp Glu Ile Cys
   465 470 475 480
   Tyr Met Thr Glu Val Asn Phe Arg Asp Ile Pro Pro Pro Arg Tyr Pro
   485 490 495
   Glu Asp Glu Leu Ala Ser Glu Pro Trp Tyr Ser Val Ser Pro Gly Asp
   500 505 510
   Val Phe Pro Glu Glu Phe Arg His Trp Leu Cys Ala Asp Pro Arg Ile
   515 520 525
   Gly Pro Leu Phe Glu Glu Met His Ala Asp Leu Phe Arg Ala Asp Tyr
   530 535 540
   Trp Arg Ala Leu Gln Asn Arg Ile Arg Glu Gly His Val Glu Asp Val
   545 550 555 560
   Tyr Ala Tyr Arg Arg Arg Gln Arg Phe Ser Val Arg Tyr Gly Glu Met
   565 570 575
   Leu Phe
<210> 13
   <211> 807
   <212> DNA
   <213> Escherichia coli
<220>
   <221> CDS
   <222> (1)..(807)
<400> 13
   atg gaa cgc tac gaa tct ctg ttt gcc cag ttg aag gag cgc aaa gaa      48
   Met Glu Arg Tyr Glu Ser Leu Phe Ala Gln Leu Lys Glu Arg Lys Glu
   1               5                   10                  15
   ggc gca ttc gtt cct ttc gtc acg ctc ggt gat ccg ggc att gag cag      96
   Gly Ala Phe Val Pro Phe Val Thr Leu Gly Asp Pro Gly Ile Glu Gln
               20                  25                  30
   tca ttg aaa att atc gat acg cta att gaa gcc ggt gct gac gcg ctg      144
   Ser Leu Lys Ile Ile Asp Thr Leu Ile Glu Ala Gly Ala Asp Ala Leu
           35                  40                  45
   gag tta ggt atc ccc ttc tcc gac cca ctg gcg gat ggc ccg acg att      192
   Glu Leu Gly Ile Pro Phe Ser Asp Pro Leu Ala Asp Gly Pro Thr Ile
       50                  55                  60
   caa aac gcc act ctg cgc gcc ttt gcg gca ggt gtg act ccg gca caa      240
   Gln Asn Ala Thr Leu Arg Ala Phe Ala Ala Gly Val Thr Pro Ala Gln
   65                  70                  75                  80
   tgt ttt gaa atg ctg gca ctg att cgc cag aaa cac ccg acc att ccc      288
   Cys Phe Glu Met Leu Ala Leu Ile Arg Gln Lys His Pro Thr Ile Pro
                   85                  90                  95
   att ggc ctg ttg atg tat gcc aat ctg gtg ttt aac aaa ggc att gat      336
   Ile Gly Leu Leu Met Tyr Ala Asn Leu Val Phe Asn Lys Gly Ile Asp
               100                 105                 110
   gag ttt tat gcc cag tgc gaa aaa gtc ggc gtc gat tcg gtg ctg gtt      384
   Glu Phe Tyr Ala Gln Cys Glu Lys Val Gly Val Asp Ser Val Leu Val
           115                 120                 125
   gcc gat gtg cca gtt gaa gag tcc gcg ccc ttc cgc cag gcc gcg ttg      432
   Ala Asp Val Pro Val Glu Glu Ser Ala Pro Phe Arg Gln Ala Ala Leu
       130                 135                 140
   cgt cat aat gtc gca cct atc ttc atc tgc ccg cca aat gcc gat gac      480
   Arg His Asn Val Ala Pro Ile Phe Ile Cys Pro Pro Asn Ala Asp Asp
   145 150 155 160
   gac ctg ctg cgc cag ata gcc tct tac ggt cgt ggt tac acc tat ttg 528
   Asp Leu Leu Arg Gln Ile Ala Ser Tyr Gly Arg Gly Tyr Thr Tyr Leu
   165 170 175
   ctg tca cga gca ggc gtg acc ggc gca gaa aac cgc gcc gcg tta ccc 576
   Leu Ser Arg Ala Gly Val Thr Gly Ala Glu Asn Arg Ala Ala Leu Pro
   180 185 190
   ctc aat cat ctg gtt gcg aag ctg aaa gag tac aac gct gca cct cca 624
   Leu Asn His Leu Val Ala Lys Leu Lys Glu Tyr Asn Ala Ala Pro Pro
   195 200 205
   ttg cag gga ttt ggt att tcc gcc ccg gat cag gta aaa gca gcg att 672
   Leu Gln Gly Phe Gly Ile Ser Ala Pro Asp Gln Val Lys Ala Ala Ile
   210 215 220
   gat gca gga gct gcg ggc gcg att tct ggt tcg gcc att gtt aaa atc 720
   Asp Ala Gly Ala Ala Gly Ala Ile Ser Gly Ser Ala Ile Val Lys Ile
   225 230 235 240
   atc gag caa cat att aat gag cca gag aaa atg ctg gcg gca ctg aaa 768
   Ile Glu Gln His Ile Asn Glu Pro Glu Lys Met Leu Ala Ala Leu Lys
   245 250 255
   gtt ttt gta caa ccg atg aaa gcg gcg acg cgc agt taa 807
   Val Phe Val Gln Pro Met Lys Ala Ala Thr Arg Ser
   260 265
<210> 14
   <211> 268
   <212> PRT
   <213> Escherichia coli
<400> 14
   Met Glu Arg Tyr Glu Ser Leu Phe Ala Gln Leu Lys Glu Arg Lys Glu
   1 5 10 15
   Gly Ala Phe Val Pro Phe Val Thr Leu Gly Asp Pro Gly Ile Glu Gln
   20 25 30
   Ser Leu Lys Ile Ile Asp Thr Leu Ile Glu Ala Gly Ala Asp Ala Leu
   35 40 45
   Glu Leu Gly Ile Pro Phe Ser Asp Pro Leu Ala Asp Gly Pro Thr Ile
   50 55 60
   Gln Asn Ala Thr Leu Arg Ala Phe Ala Ala Gly Val Thr Pro Ala Gln
   65 70 75 80
   Cys Phe Glu Met Leu Ala Leu Ile Arg Gln Lys His Pro Thr Ile Pro
                       85                  90                  95
   Ile Gly Leu Leu Met Tyr Ala Asn Leu Val Phe Asn Lys Gly Ile Asp
                   100                 105                 110
   Glu Phe Tyr Ala Gln Cys Glu Lys Val Gly Val Asp Ser Val Leu Val
               115                 120                 125
   Ala Asp Val Pro Val Glu Glu Ser Ala Pro Phe Arg Gln Ala Ala Leu
       130                 135                 140
   Arg His Asn Val Ala Pro Ile Phe Ile Cys Pro Pro Asn Ala Asp Asp
   145                 150                 155                 160
   Asp Leu Leu Arg Gln Ile Ala Ser Tyr Gly Arg Gly Tyr Thr Tyr Leu
                   165                 170                 175
   Leu Ser Arg Ala Gly Val Thr Gly Ala Glu Asn Arg Ala Ala Leu Pro
               180                 185                 190
   Leu Asn His Leu Val Ala Lys Leu Lys Glu Tyr Asn Ala Ala Pro Pro
           195                 200                 205
   Leu Gln Gly Phe Gly Ile Ser Ala Pro Asp Gln Val Lys Ala Ala Ile
       210                 215                 220
   Asp Ala Gly Ala Ala Gly Ala Ile Ser Gly Ser Ala Ile Val Lys Ile
   225                 230                 235                 240
   Ile Glu Gln His Ile Asn Glu Pro Glu Lys Met Leu Ala Ala Leu Lys
                   245                 250                 255
   Val Phe Val Gln Pro Met Lys Ala Ala Thr Arg Ser
               260                 265
<210> 15
   <211> 1194
   <212> DNA
   <213> Escherichia coli
<220>
   <221> CDS
   <222> (1)..(1194)
<400> 15
   atg aca aca tta ctt aac ccc tat ttt ggt gag ttt ggc ggc atg tac      48
   gtg cca caa atc ctg atg cct gct ctg cgc cag ctg gaa gaa gct ttt      96
   gtc agt gcg caa aaa gat cct gaa ttt cag gct cag ttc aac gac ctg     144
   ctg aaa aac tat gcc ggg cgt cca acc gcg ctg acc aaa tgc cag aac     192
   att aca gcc ggg acg aac acc acg ctg tat ctc aag cgt gaa gat ttg 240
   ctg cac ggc ggc gcg cat aaa act aac cag gtg ctg ggg cag gcg ttg 288
   ctg gcg aag cgg atg ggt aaa acc gaa atc atc gcc gaa acc ggt gcc 336
   ggt cag cat ggc gtg gcg tcg gcc ctt gcc agc gcc ctg ctc ggc ctg 384
   aaa tgc cgt att tat atg ggt gcc aaa gac gtt gaa cgc cag tcg cct 432
   aac gtt ttt cgt atg cgc tta atg ggt gcg gaa gtg atc ccg gtg cat 480
   agc ggt tcc gcg acg ctg aaa gat gcc tgt aac gag gcg ctg cgc gac 528
   tgg tcc ggt agt tac gaa acc gcg cac tat atg ctg ggc acc gca gct 576
   ggc ccg cat cct tat ccg acc att gtg cgt gag ttt cag cgg atg att 624
   ggc gaa gaa acc aaa gcg cag att ctg gaa aga gaa ggt cgc ctg ccg 672
   gat gcc gtt atc gcc tgt gtt ggc ggc ggt tcg aat gcc atc ggc atg 720
   ttt gct gat ttc atc aat gaa acc aac gtc ggc ctg att ggt gtg gag     768
   cca ggt ggt cac ggt atc gaa act ggc gag cac ggc gca ccg cta aaa     816
   cat ggt cgc gtg ggt atc tat ttc ggt atg aaa gcg ccg atg atg caa     864
   acc gaa gac ggg cag att gaa gaa tct tac tcc atc tcc gcc gga ctg     912
   gat ttc ccg tct gtc ggc cca caa cac gcg tat ctt aac agc act gga     960
   cgc gct gat tac gtg tct att acc gat gat gaa gcc ctt gaa gcc ttc     1008
   aaa acg ctg tgc ctg cac gaa ggg atc atc ccg gcg ctg gaa tcc tcc     1056
   cac gcc ctg gcc cat gcg ttg aaa atg atg cgc gaa aac ccg gat aaa     1104
   gag cag cta ctg gtg gtt aac ctt tcc ggt cgc ggc gat aaa gac atc     1152
   ttc acc gtt cac gat att ttg aaa gca cga ggg gaa atc tga             1194
<210> 16
   <211> 397
   <212> PRT
   <213> Escherichia coli
<400> 16
<210> 17
   <211> 44
   <212> DNA
   <213> Artificial
<220>
   <223> 5' -primer-evgAS
<400> 17
   gggaattcac gcctgtagga ttagtaagaa gacttatagt gcca 44
<210> 18
   <211> 44
   <212> DNA
   <213> Artificial
<220>
   <223> 3' -primer-evgAS
<400> 18
   ggaagcttcc acatttgaac attgtgggag ccgctattta gtca 44
<210> 19
   <211> 44
   <212> DNA
   <213> Artificial
<220>
   <223> 5' -primer-gadE
<400> 19
   ggggatccaa agtgaacaaa gagttccgta agcgttgatg ctat 44
<210> 20
   <211> 44
   <212> DNA
   <213> Artificial
<220>
   <223> 3' -primer-gadE
<400> 20
   ggaagcttat gccagccatc aatttcagtt gcttatgtcc tgac 44
<210> 21
   <211> 47
   <212> DNA
   <213> Artificial
<220>
   <223> 5' -primer-yde0
<400> 21
   ggaagcttaa cgcggggcag ggaatggctg ccccatttaa-ttcttac 47
<210> 22
   <211> 46
   <212> DNA
   <213> Artificial
<220>
   <223> 3' -primer-yde0
<400> 22
   gggaattcgc tgagtatttc agaaatgggt cgcattgcaa gagatc 46
<210> 23
   <211> 615
   <212> DNA
   <213> Escherichia coli
<220>
   <221> CDS
   <222> (1)..(615)
<400> 23
   atg aac gca ata att att gat gac cat cct ctt gct atc gca gca att        48
   Met Asn Ala Ile Ile Ile Asp Asp His Pro Leu Ala Ile Ala Ala Ile
   1               5                   10                  15
   cgt aat tta ttg atc aaa aac gat att gaa atc tta gca gag ttg act        96
   Arg Asn Leu Leu Ile Lys Asn Asp Ile Glu Ile Leu Ala Glu Leu Thr
               20                  25                  30
   gaa ggc gga agt gcc gtt cag cgg gtg gaa aca ctt aag cct gat atc      144
   Glu Gly Gly Ser Ala Val Gln Arg Val Glu Thr Leu Lys Pro Asp Ile
           35                  40                  45
   gtc atc att gat gtc gat atc ccc gga gtt aac ggt atc cag gtg tta      192
   Val Ile Ile Asp Val Asp Ile Pro Gly Val Asn Gly Ile Gln Val Leu
       50                  55                  60
   gaa acg ctg agg aag cgc caa tat agc gga att att att atc gtc tcc      240
   Glu Thr Leu Arg Lys Arg Gln Tyr Ser Gly Ile Ile Ile Ile Val Ser
   65                  70                  75                  80
   gct aaa aat gac cat ttt tac ggg aaa cat tgt gct gat gct ggc gct      288
   Ala Lys Asn Asp His Phe Tyr Gly Lys His Cys Ala Asp Ala Gly Ala
                   85                  90                  95
   aat ggt ttc gtg agt aaa aaa gaa ggc atg aac aat atc att gcg gct      336
   Asn Gly Phe Val Ser Lys Lys Glu Gly Met Asn Asn Ile Ile Ala Ala
               100                 105                 110
   att gaa gct gca aaa aat ggc tac tgc tat ttc ccc ttc tct ctc aac      384
   Ile Glu Ala Ala Lys Asn Gly Tyr Cys Tyr Phe Pro Phe Ser Leu Asn
           115                 120                 125
   cgg ttt gtt gga agt tta acg tcc gac cag caa aaa ctc gac tcc tta      432
   Arg Phe Val Gly Ser Leu Thr Ser Asp Gln Gln Lys Leu Asp Ser Leu
       130                 135                 140
   tcg aaa caa gaa att agt gtc atg cgg tat att ctt gat ggc aag gat      480
   Ser Lys Gln Glu Ile Ser Val Met Arg Tyr Ile Leu Asp Gly Lys Asp
   145                 150                 155                 160
   aat aat gac att gct gaa aaa atg ttc atc agc aac aaa act gtc agc      528
   Asn Asn Asp Ile Ala Glu Lys Met Phe Ile Ser Asn Lys Thr Val Ser
                   165                 170                 175
   act tat aaa agt cgc ctg atg gaa aaa tta gaa tgt aaa tca ctg atg      576
   Thr Tyr Lys Ser Arg Leu Met Glu Lys Leu Glu Cys Lys Ser Leu Met
               180                 185                 190
   gat ctt tac aca ttc gca caa cgt aac aaa atc ggc taa                  615
   Asp Leu Tyr Thr Phe Ala Gln Arg Asn Lys Ile Gly
           195                 200
<210> 24
   <211> 204
   <212> PRT
   <213> Escherichia coli
<400> 24
   Met Asn Ala Ile Ile Ile Asp Asp His Pro Leu Ala Ile Ala Ala Ile
   1 5 10 15
   Arg Asn Leu Leu Ile Lys Asn Asp Ile Glu Ile Leu Ala Glu Leu Thr
   20 25 30
   Glu Gly Gly Ser Ala Val Gln Arg Val Glu Thr Leu Lys Pro Asp Ile
   35 40 45
   Val Ile Ile Asp Val Asp Ile Pro Gly Val Asn Gly Ile Gln Val Leu
   50 55 60
   Glu Thr Leu Arg Lys Arg Gln Tyr Ser Gly Ile Ile Ile Ile Val Ser
   65 70 75 80
   Ala Lys Asn Asp His Phe Tyr Gly Lys His Cys Ala Asp Ala Gly Ala
   85 90 95
   Asn Gly Phe Val Ser Lys Lys Glu Gly Met Asn Asn Ile Ile Ala Ala
   100 105 110
   Ile Glu Ala Ala Lys Asn Gly Tyr Cys Tyr Phe Pro Phe Ser Leu Asn
   115 120 125
   Arg Phe Val Gly Ser Leu Thr Ser Asp Gln Gln Lys Leu Asp Ser Leu
   130 135 140
   Ser Lys Gln Glu Ile Ser Val Met Arg Tyr Ile Leu Asp Gly Lys Asp
   145 150 155 160
   Asn Asn Asp Ile Ala Glu Lys Met Phe Ile Ser Asn Lys Thr Val Ser
   165 170 175
   Thr Tyr Lys Ser Arg Leu Met Glu Lys Leu Glu Cys Lys Ser Leu Met
   180 185 190
   Asp Leu Tyr Thr Phe Ala Gln Arg Asn Lys Ile Gly
   195 200
<210> 25
   <211> 3594
   <212> DNA
   <213> Escherichia coli
<220>
   <221> CDS
   <222> (1)..(3594)
<400> 25
   atg aag ttt tta ccc tat att ttt ctt ctc tgt tgt ggt ctt tgg tcg 48
   Met Lys Phe Leu Pro Tyr Ile Phe Leu Leu Cys Cys Gly Leu Trp Ser
   1 5 10 15
   acc ata agt ttc gca gac gaa gat tac atc gaa tat cgt ggc atc agt      96
   Thr Ile Ser Phe Ala Asp Glu Asp Tyr Ile Glu Tyr Arg Gly Ile Ser
                   20                  25                  30
   agt aac aac cgt gtc aca ctt gat cca cta cgt ctg agc aac aag gaa     144
   Ser Asn Asn Arg Val Thr Leu Asp Pro Leu Arg Leu Ser Asn Lys Glu
               35                  40                  45
   tta cgt tgg tta gcg agc aaa aaa aat ctt gtg att gca gta cat aag     192
   Leu Arg Trp Leu Ala Ser Lys Lys Asn Leu Val Ile Ala Val His Lys
           50                  55                  60
   tcc caa acg gct acg ttg ttg cat acc gat tcg cag caa cgg gtt cgt     240
   Ser Gln Thr Ala Thr Leu Leu His Thr Asp Ser Gln Gln Arg Val Arg
   65                  70                  75                  80
   ggt att aat gct gat tat tta aat ctt tta aaa aga gcg tta aat atc     288
   Gly Ile Asn Ala Asp Tyr Leu Asn Leu Leu Lys Arg Ala Leu Asn Ile
                   85                  90                  95
   aaa tta aca ctc cgg gaa tac gca gat cat caa aaa gca atg gac gcg     336
   Lys Leu Thr Leu Arg Glu Tyr Ala Asp His Gln Lys Ala Met Asp Ala
               100                 105                 110
   ctt gca gaa ggt gaa gtc gat ata gtg tta tca cat tta gtt act tcg     384
   Leu Ala Glu Gly Glu Val Asp Ile Val Leu Ser His Leu Val Thr Ser
           115                 120                 125
   ccg cct ctt aat aat gac att gct gca acc aaa cca ttg ata att acc     432
   Pro Pro Leu Asn Asn Asp Ile Ala Ala Thr Lys Pro Leu Ile Ile Thr
       130                 135                 140
   ttt ccg gcg ctg gta acc acc ctt cac gac tca atg cga ccg ctt acc     480
   Phe Pro Ala Leu Val Thr Thr Leu His Asp Ser Met Arg Pro Leu Thr
   145                 150                 155                 160
   tca cca aaa cca gta aat att gct cgg gta gca aat tac ccc cca gac     528
   Ser Pro Lys Pro Val Asn Ile Ala Arg Val Ala Asn Tyr Pro Pro Asp
                   165                 170                 175
   gag gta att cat caa tca ttt cca aaa gca aca att atc tct ttt aca     576
   Glu Val Ile His Gln Ser Phe Pro Lys Ala Thr Ile Ile Ser Phe Thr
               180                 185                 190
   aat tta tat cag gca tta gca tcc gtc tca gct ggg cac aat gat tac     624
   Asn Leu Tyr Gln Ala Leu Ala Ser Val Ser Ala Gly His Asn Asp Tyr
           195                 200                 205
   ttt att ggt agt aac atc att acc agc agt atg att tcc cgc tat ttc     672
   Phe Ile Gly Ser Asn Ile Ile Thr Ser Ser Met Ile Ser Arg Tyr Phe
       210                 215                 220
   act cac tcc tta aat gta gtg aaa tat tat aac tcg ccg cgt caa tat     720
   Thr His Ser Leu Asn Val Val Lys Tyr Tyr Asn Ser Pro Arg Gln Tyr
   225                 230                 235                 240
   aat ttt ttc ttg acc aga aaa gaa tct gtc att ctt aat gaa gta ctc     768
   Asn Phe Phe Leu Thr Arg Lys Glu Ser Val Ile Leu Asn Glu Val Leu
                   245                 250                 255
   aat aga ttt gtt gat gct tta aca aat gaa gtt cgc tat gaa gta tca     816
   Asn Arg Phe Val Asp Ala Leu Thr Asn Glu Val Arg Tyr Glu Val Ser
               260                 265                 270
   caa aat tgg ctt gat aca gga aac ctg gcc ttt ctg aac aaa cca tta     864
   Gln Asn Trp Leu Asp Thr Gly Asn Leu Ala Phe Leu Asn Lys Pro Leu
           275                 280                 285
   gaa ctc act gaa cat gaa aaa cag tgg att aag cag cat ccc aat tta     912
   Glu Leu Thr Glu His Glu Lys Gln Trp Ile Lys Gln His Pro Asn Leu
       290                 295                 300
   aag gtg ctg gaa aat cct tac tcg ccc ccc tat tct atg acg gat gaa     960
   Lys Val Leu Glu Asn Pro Tyr Ser Pro Pro Tyr Ser Met Thr Asp Glu
   305                 310                 315                 320
   aat ggc tcg gtt cgg ggc gtt atg ggg gac att ctt aat att att acc    1008
   Asn Gly Ser Val Arg Gly Val Met Gly Asp Ile Leu Asn Ile Ile Thr
                   325                 330                 335
   ttg caa aca ggt tta aat ttt tct ccg atc acc gtt tca cac aat atc    1056
   Leu Gln Thr Gly Leu Asn Phe Ser Pro Ile Thr Val Ser His Asn Ile
               340                 345                 350
   cat gct gga aca cag ctt agc ccc gga gga tgg gat ata ata cct ggc    1104
   His Ala Gly Thr Gln Leu Ser Pro Gly Gly Trp Asp Ile Ile Pro Gly
           355                 360                 365
   gct att tat agt gaa gat cga gaa aat aat gtt tta ttt gct gaa gcc    1152
   Ala Ile Tyr Ser Glu Asp Arg Glu Asn Asn Val Leu Phe Ala Glu Ala
       370                 375                 380
   ttc ata aca acg cct tac gtt ttt gtc atg caa aaa gcg cct gac agt    1200
   Phe Ile Thr Thr Pro Tyr Val Phe Val Met Gln Lys Ala Pro Asp Ser
   385                 390                 395                 400
   gaa caa aca tta aaa aaa gga atg aaa gtt gcc att cca tat tat tat     1248
   Glu Gln Thr Leu Lys Lys Gly Met Lys Val Ala Ile Pro Tyr Tyr Tyr
                   405                 410                 415
   gag ctg cat tcg caa tta aaa gag atg tat ccg gag gtt gaa tgg ata     1296
   Glu Leu His Ser Gln Leu Lys Glu Met Tyr Pro Glu Val Glu Trp Ile
               420                 425                 430
   cag gtc gat aat gcc agc gct gca ttt cac aag gtt aag gaa ggt gaa     1344
   Gln Val Asp Asn Ala Ser Ala Ala Phe His Lys Val Lys Glu Gly Glu
           435                 440                 445
   ctt gat gct ctg gtc gcg aca cag cta aat tcg cgt tac atg atc gat     1392
   Leu Asp Ala Leu Val Ala Thr Gln Leu Asn Ser Arg Tyr Met Ile Asp
       450                 455                 460
   cat tac tat cct aat gaa ctt tat cat ttt ctt att cct ggc gtt ccg     1440
   His Tyr Tyr Pro Asn Glu Leu Tyr His Phe Leu Ile Pro Gly Val Pro
   465                 470                 475                 480
   aat gca tcg ctt tcg ttc gct ttt cct cgc gga gaa ccg gaa ctt aag     1488
   Asn Ala Ser Leu Ser Phe Ala Phe Pro Arg Gly Glu Pro Glu Leu Lys
                   485                 490                 495
   gat att att aat aaa gca ctg aat gca att ccc cca agc gaa gtt ctg     1536
   Asp Ile Ile Asn Lys Ala Leu Asn Ala Ile Pro Pro Ser Glu Val Leu
               500                 505                 510
   cgc ctg acg gaa aaa tgg att aaa atg ccc aat gtg acc att gac aca     1584
   Arg Leu Thr Glu Lys Trp Ile Lys Met Pro Asn Val Thr Ile Asp Thr
           515                 520                 525
   tgg gac cta tat agc gag caa ttt tat att gtt acg aca tta tcc gtt     1632
   Trp Asp Leu Tyr Ser Glu Gln Phe Tyr Ile Val Thr Thr Leu Ser Val
       530                 535                 540
   tta tta gtt ggc agt agc ctt tta tgg gga ttc tac ctg tta cgc tca     1680
   Leu Leu Val Gly Ser Ser Leu Leu Trp Gly Phe Tyr Leu Leu Arg Ser
   545                 550                 555                 560
   gtt cgt cgt cgt aaa gtc att cag ggt gat tta gaa aac caa ata tca     1728
   Val Arg Arg Arg Lys Val Ile Gln Gly Asp Leu Glu Asn Gln Ile Ser
                   565                 570                 575
   ttc cga aaa gca ctc tcg gat tcc tta ccg aat cca act tat gtt gta 1776
   Phe Arg Lys Ala Leu Ser Asp Ser Leu Pro Asn Pro Thr Tyr Val Val
   580 585 590
   aac tgg caa ggt aat gtc att agt cat aat agt gct ttt gaa cat tat 1824
   Asn Trp Gln Gly Asn Val Ile Ser His Asn Ser Ala Phe Glu His Tyr
   595 600 605
   ttc act gcg gat tac tac aaa aat gca atg tta cca tta gaa aac agt 1872
   Phe Thr Ala Asp Tyr Tyr Lys Asn Ala Met Leu Pro Leu Glu Asn Ser
   610 615 620
   gac tca ccc ttt aaa gat gtt ttt tct aat gcg cat gaa gtc aca gca 1920
   Asp Ser Pro Phe Lys Asp Val Phe Ser Asn Ala His Glu Val Thr Ala
   625 630 635 640
   gaa acg aaa gaa aat cga aca ata tac aca cag gta ttt gaa att gat 1968
   Glu Thr Lys Glu Asn Arg Thr Ile Tyr Thr Gln Val Phe Glu Ile Asp
   645 650 655
   aat ggc atc gag aaa aga tgc att aat cac tgg cat aca tta tgc aat 2016
   Asn Gly Ile Glu Lys Arg Cys Ile Asn His Trp His Thr Leu Cys Asn
   660 665 670
   ctt cct gca agt gac aat gca gta tat att tgt ggt tgg caa gat att 2064
   Leu Pro Ala Ser Asp Asn Ala Val Tyr Ile Cys Gly Trp Gln Asp Ile
   675 680 685
   act gaa acg cgt gat cta att aat gca ctc gag gta gaa aaa aat aaa 2112
   Thr Glu Thr Arg Asp Leu Ile Asn Ala Leu Glu Val Glu Lys Asn Lys
   690 695 700
   gcg ata aag gct acc gta gca aaa agt cag ttt ctg gca acg atg agt 2160
   Ala Ile Lys Ala Thr Val Ala Lys Ser Gln Phe Leu Ala Thr Met Ser
   705 710 715 720
   cac gaa ata aga aca cca ata agc tct att atg ggc ttc ctg gaa ctt 2208
   His Glu Ile Arg Thr Pro Ile Ser Ser Ile Met Gly Phe Leu Glu Leu
   725 730 735
   ctg tcg ggt tct ggt ctt agc aag gag caa cgg gtg gag gcg att tca 2256
   Leu Ser Gly Ser Gly Leu Ser Lys Glu Gln Arg Val Glu Ala Ile Ser
   740 745 750
   ctt gcc tac gcc acc gga caa tca ctc ctc ggc tta att ggt gaa atc 2304
   Leu Ala Tyr Ala Thr Gly Gln Ser Leu Leu Gly Leu Ile Gly Glu Ile
   755 760 765
   ctt gat gtc gac aaa att gaa tcg ggt aac tat caa ctt caa cca caa 2352
   Leu Asp Val Asp Lys Ile Glu Ser Gly Asn Tyr Gln Leu Gln Pro Gln
   770 775 780
   tgg gtc gat atc cct act tta gtc cag aac act tgt cac tct ttc ggt 2400
   Trp Val Asp Ile Pro Thr Leu Val Gln Asn Thr Cys His Ser Phe Gly
   785 790 795 800
   gcg att gct gca agc aaa tcg atc gca tta agt tgc agc agt acg ttt 2448
   Ala Ile Ala Ala Ser Lys Ser Ile Ala Leu Ser Cys Ser Ser Thr Phe
   805 810 815
   cct gaa cat tac ctg gtt aag atc gac cct cag gcg ttt aag cag gtc 2496
   Pro Glu His Tyr Leu Val Lys Ile Asp Pro Gln Ala Phe Lys Gln Val
   820 825 830
   tta tca aat tta ctg agt aat gct ctc aaa ttt acc acc gag ggg gca 2544
   Leu Ser Asn Leu Leu Ser Asn Ala Leu Lys Phe Thr Thr Glu Gly Ala
   835 840 845
   gta aaa att acg acc tcc ctg ggt cac att gat gac aac cac gct gtt 2592
   Val Lys Ile Thr Thr Ser Leu Gly His Ile Asp Asp Asn His Ala Val
   850 855 860
   atc aaa atg acg att atg gat tct gga agt gga tta tcg cag gaa gaa 2640
   Ile Lys Met Thr Ile Met Asp Ser Gly Ser Gly Leu Ser Gln Glu Glu
   865 870 875 880
   caa caa caa ctg ttt aaa cgc tac agc caa aca agt gca ggt cgt cag 2688
   Gln Gln Gln Leu Phe Lys Arg Tyr Ser Gln Thr Ser Ala Gly Arg Gln
   885 890 895
   caa aca ggt tct ggt tta ggc tta atg atc tgc aaa gaa tta att aaa 2736
   Gln Thr Gly Ser Gly Leu Gly Leu Met Ile Cys Lys Glu Leu Ile Lys
   900 905 910
   aat atg cag ggc gat ttg tca tta gaa agt cat cca ggc ata gga aca 2784
   Asn Met Gln Gly Asp Leu Ser Leu Glu Ser His Pro Gly Ile Gly Thr
   915 920 925
   aca ttt acg atc aca atc ccg gta gaa att agc cag caa gtg gcg act 2832
   Thr Phe Thr Ile Thr Ile Pro Val Glu Ile Ser Gln Gln Val Ala Thr
   930 935 940
   gtc gag gca aaa gca gaa caa ccc atc aca cta cct gaa aag ttg agc 2880
   Val Glu Ala Lys Ala Glu Gln Pro Ile Thr Leu Pro Glu Lys Leu Ser
   945 950 955 960
   ata tta atc gcg gat gat cat ccg acc aac agg cta tta ctc aaa cgc 2928
   Ile Leu Ile Ala Asp Asp His Pro Thr Asn Arg Leu Leu Leu Lys Arg
   965 970 975
   cag cta aat cta tta gga tat gat gtt gat gaa gcc act gat ggt gtg 2976
   Gln Leu Asn Leu Leu Gly Tyr Asp Val Asp Glu Ala Thr Asp Gly Val
   980 985 990
   caa gcg cta cac aaa gtc agt atg caa cat tat gat ctg ctt att act 3024
   Gln Ala Leu His Lys Val Ser Met Gln His Tyr Asp Leu Leu Ile Thr
   995 1000 1005
   gac gtt aat atg ccg aat atg gat ggt ttt gag ttg act cgc aaa 3069
   Asp Val Asn Met Pro Asn Met Asp Gly Phe Glu Leu Thr Arg Lys
   1010 1015 1020
   ctc cgt gag caa aat tct tcc tta ccc atc tgg ggg ctt aca gcc 3114
   Leu Arg Glu Gln Asn Ser Ser Leu Pro Ile Trp Gly Leu Thr Ala
   1025 1030 1035
   aac gca cag gct aac gaa cgt gaa aaa ggg tta agt tgc ggc atg 3159
   Asn Ala Gln Ala Asn Glu Arg Glu Lys Gly Leu Ser Cys Gly Met
   1040 1045 1050
   aac tta tgt ttg ttc aaa ccg ttg acc ctg gat gta ctg aaa aca 3204
   Asn Leu Cys Leu Phe Lys Pro Leu Thr Leu Asp Val Leu Lys Thr
   1055 1060 1065
   cat tta agt cag tta cac caa gtt gcg cat att gca cct cag tat 3249
   His Leu Ser Gln Leu His Gln Val Ala His Ile Ala Pro Gln Tyr
   1070 1075 1080
   cgc cac ctt gat atc gaa gcc ctg aaa aat aat acg gcg aac gat 3294
   Arg His Leu Asp Ile Glu Ala Leu Lys Asn Asn Thr Ala Asn Asp
   1085 1090 1095
   cta caa ctg atg cag gag att ctc atg act ttc cag cat gaa acg 3339
   Leu Gln Leu Met Gln Glu Ile Leu Met Thr Phe Gln His Glu Thr
   1100 1105 1110
   cat aaa gat cta ccc gct gcg ttt caa gca cta gaa gct ggc gat 3384
   His Lys Asp Leu Pro Ala Ala Phe Gln Ala Leu Glu Ala Gly Asp
   1115 1120 1125
   aac aga act ttc cat cag tgt att cat cgc atc cac ggt gcg gct 3429
   Asn Arg Thr Phe His Gln Cys Ile His Arg Ile His Gly Ala Ala
   1130 1135 1140
   aac atc ctg aat ttg caa aag ttg att aat att agc cat cag tta 3474
   Asn Ile Leu Asn Leu Gln Lys Leu Ile Asn Ile Ser His Gln Leu
   1145 1150 1155
   gaa ata aca cct gtt tca gat gac agt aag cct gaa att ctt cag 3519
   Glu Ile Thr Pro Val Ser Asp Asp Ser Lys Pro Glu Ile Leu Gln
   1160 1165 1170
   ttg ctg aac tct gta aaa gaa cac att gca gag ctg gac cag gag 3564
   Leu Leu Asn Ser Val Lys Glu His Ile Ala Glu Leu Asp Gln Glu
   1175 1180 1185
   att gct gtt ttc tgt cag aaa aat gac taa 3594
   Ile Ala Val Phe Cys Gln Lys Asn Asp
   1190 1195
<210> 26
   <211> 1197
   <212> PRT
   <213> Escherichia coli
<400> 26
   Met Lys Phe Leu Pro Tyr Ile Phe Leu Leu Cys Cys Gly Leu Trp Ser
   1 5 10 15
   Thr Ile Ser Phe Ala Asp Glu Asp Tyr Ile Glu Tyr Arg Gly Ile Ser
   20 25 30
   Ser Asn Asn Arg Val Thr Leu Asp Pro Leu Arg Leu Ser Asn Lys Glu
   35 40 45
   Leu Arg Trp Leu Ala Ser Lys Lys Asn Leu Val Ile Ala Val His Lys
   50 55 60
   Ser Gln Thr Ala Thr Leu Leu His Thr Asp Ser Gln Gln Arg Val Arg
   65 70 75 80
   Gly Ile Asn Ala Asp Tyr Leu Asn Leu Leu Lys Arg Ala Leu Asn Ile
   85 90 95
   Lys Leu Thr Leu Arg Glu Tyr Ala Asp His Gln Lys Ala Met Asp Ala
   100 105 110
   Leu Ala Glu Gly Glu Val Asp Ile Val Leu Ser His Leu Val Thr Ser
   115 120 125
   Pro Pro Leu Asn Asn Asp Ile Ala Ala Thr Lys Pro Leu Ile Ile Thr
   130 135 140
   Phe Pro Ala Leu Val Thr Thr Leu His Asp Ser Met Arg Pro Leu Thr
   145 150 155 160
   Ser Pro Lys Pro Val Asn Ile Ala Arg Val Ala Asn Tyr Pro Pro Asp
   165 170 175
   Glu Val Ile His Gln Ser Phe Pro Lys Ala Thr Ile Ile Ser Phe Thr
   180 185 190
   Asn Leu Tyr Gln Ala Leu Ala Ser Val Ser Ala Gly His Asn Asp Tyr
   195 200 205
   Phe Ile Gly Ser Asn Ile Ile Thr Ser Ser Met Ile Ser Arg Tyr Phe
   210 215 220
   Thr His Ser Leu Asn Val Val Lys Tyr Tyr Asn Ser Pro Arg Gln Tyr
   225 230 235 240
   Asn Phe Phe Leu Thr Arg Lys Glu Ser Val Ile Leu Asn Glu Val Leu
   245 250 255
   Asn Arg Phe Val Asp Ala Leu Thr Asn Glu Val Arg Tyr Glu Val Ser
   260 265 270
   Gln Asn Trp Leu Asp Thr Gly Asn Leu Ala Phe Leu Asn Lys Pro Leu
   275 280 285
   Glu Leu Thr Glu His Glu Lys Gln Trp Ile Lys Gln His Pro Asn Leu
   290 295 300
   Lys Val Leu Glu Asn Pro Tyr Ser Pro Pro Tyr Ser Met Thr Asp Glu
   305 310 315 320
   Asn Gly Ser Val Arg Gly Val Met Gly Asp Ile Leu Asn Ile Ile Thr
   325 330 335
   Leu Gln Thr Gly Leu Asn Phe Ser Pro Ile Thr Val Ser His Asn Ile
   340 345 350
   His Ala Gly Thr Gln Leu Ser Pro Gly Gly Trp Asp Ile Ile Pro Gly
   355 360 365
   Ala Ile Tyr Ser Glu Asp Arg Glu Asn Asn Val Leu Phe Ala Glu Ala
   370 375 380
   Phe Ile Thr Thr Pro Tyr Val Phe Val Met Gln Lys Ala Pro Asp Ser
   385 390 395 400
   Glu Gln Thr Leu Lys Lys Gly Met Lys Val Ala Ile Pro Tyr Tyr Tyr
   405 410 415
   Glu Leu His Ser Gln Leu Lys Glu Met Tyr Pro Glu Val Glu Trp Ile
   420 425 430
   Gln Val Asp Asn Ala Ser Ala Ala Phe His Lys Val Lys Glu Gly Glu
   435 440 445
   Leu Asp Ala Leu Val Ala Thr Gln Leu Asn Ser Arg Tyr Met Ile Asp
   450 455 460
   His Tyr Tyr Pro Asn Glu Leu Tyr His Phe Leu Ile Pro Gly Val Pro
   465 470 475 480
   Asn Ala Ser Leu Ser Phe Ala Phe Pro Arg Gly Glu Pro Glu Leu Lys
   485 490 495
   Asp Ile Ile Asn Lys Ala Leu Asn Ala Ile Pro Pro Ser Glu Val Leu
   500 505 510
   Arg Leu Thr Glu Lys Trp Ile Lys Met Pro Asn Val Thr Ile Asp Thr
   515 520 525
   Trp Asp Leu Tyr Ser Glu Gln Phe Tyr Ile Val Thr Thr Leu Ser Val
   530 535 540
   Leu Leu Val Gly Ser Ser Leu Leu Trp Gly Phe Tyr Leu Leu Arg Ser
   545 550 555 560
   Val Arg Arg Arg Lys Val Ile Gln Gly Asp Leu Glu Asn Gln Ile Ser
   565 570 575
   Phe Arg Lys Ala Leu Ser Asp Ser Leu Pro Asn Pro Thr Tyr Val Val
   580 585 590
   Asn Trp Gln Gly Asn Val Ile Ser His Asn Ser Ala Phe Glu His Tyr
   595 600 605
   Phe Thr Ala Asp Tyr Tyr Lys Asn Ala Met Leu Pro Leu Glu Asn Ser
   610 615 620
   Asp Ser Pro Phe Lys Asp Val Phe Ser Asn Ala His Glu Val Thr Ala
   625 630 635 640
   Glu Thr Lys Glu Asn Arg Thr Ile Tyr Thr Gln Val Phe Glu Ile Asp
   645 650 655
   Asn Gly Ile Glu Lys Arg Cys Ile Asn His Trp His Thr Leu Cys Asn
   660 665 670
   Leu Pro Ala Ser Asp Asn Ala Val Tyr Ile Cys Gly Trp Gln Asp Ile
   675 680 685
   Thr Glu Thr Arg Asp Leu Ile Asn Ala Leu Glu Val Glu Lys Asn Lys
   690 695 700
   Ala Ile Lys Ala Thr Val Ala Lys Ser Gln Phe Leu Ala Thr Met Ser
   705 710 715 720
   His Glu Ile Arg Thr Pro Ile Ser Ser Ile Met Gly Phe Leu Glu Leu
   725 730 735
   Leu Ser Gly Ser Gly Leu Ser Lys Glu Gln Arg Val Glu Ala Ile Ser
   740 745 750
   Leu Ala Tyr Ala Thr Gly Gln Ser Leu Leu Gly Leu Ile Gly Glu Ile
   755 760 765
   Leu Asp Val Asp Lys Ile Glu Ser Gly Asn Tyr Gln Leu Gln Pro Gln
   770 775 780
   Trp Val Asp Ile Pro Thr Leu Val Gln Asn Thr Cys His Ser Phe Gly
   785 790 795 800
   Ala Ile Ala Ala Ser Lys Ser Ile Ala Leu Ser Cys Ser Ser Thr Phe
   805 810 815
   Pro Glu His Tyr Leu Val Lys Ile Asp Pro Gln Ala Phe Lys Gln Val
   820 825 830
   Leu Ser Asn Leu Leu Ser Asn Ala Leu Lys Phe Thr Thr Glu Gly Ala
   835 840 845
   Val Lys Ile Thr Thr Ser Leu Gly His Ile Asp Asp Asn His Ala Val
   850 855 860
   Ile Lys Met Thr Ile Met Asp Ser Gly Ser Gly Leu Ser Gln Glu Glu
   865 870 875 880
   Gln Gln Gln Leu Phe Lys Arg Tyr Ser Gln Thr Ser Ala Gly Arg Gln
   885 890 895
   Gln Thr Gly Ser Gly Leu Gly Leu Met Ile Cys Lys Glu Leu Ile Lys
   900 905 910
   Asn Met Gln Gly Asp Leu Ser Leu Glu Ser His Pro Gly Ile Gly Thr
   915 920 925
   Thr Phe Thr Ile Thr Ile Pro Val Glu Ile Ser Gln Gln Val Ala Thr
   930 935 940
   Val Glu Ala Lys Ala Glu Gln Pro Ile Thr Leu Pro Glu Lys Leu Ser
   945 950 955 960
   Ile Leu Ile Ala Asp Asp His Pro Thr Asn Arg Leu Leu Leu Lys Arg
   965 970 975
   Gln Leu Asn Leu Leu Gly Tyr Asp Val Asp Glu Ala Thr Asp Gly Val
   980 985 990
   Gln Ala Leu His Lys Val Ser Met Gln His Tyr Asp Leu Leu Ile Thr
   995 1000 1005
   Asp Val Asn Met Pro Asn Met Asp Gly Phe Glu Leu Thr Arg Lys
   1010 1015 1020
   Leu Arg Glu Gln Asn Ser Ser Leu Pro Ile Trp Gly Leu Thr Ala
   1025 1030 1035
   Asn Ala Gln Ala Asn Glu Arg Glu Lys Gly Leu Ser Cys Gly Met
   1040 1045 1050
   Asn Leu Cys Leu Phe Lys Pro Leu Thr Leu Asp Val Leu Lys Thr
   1055 1060 1065
   His Leu Ser Gln Leu His Gln Val Ala His Ile Ala Pro Gln Tyr
   1070 1075 1080
   Arg His Leu Asp Ile Glu Ala Leu Lys Asn Asn Thr Ala Asn Asp
   1085 1090 1095
   Leu Gln Leu Met Gln Glu Ile Leu Met Thr Phe Gln His Glu Thr
   1100 1105 1110
   His Lys Asp Leu Pro Ala Ala Phe Gln Ala Leu Glu Ala Gly Asp
   1115 1120 1125
   Asn Arg Thr Phe His Gln Cys Ile His Arg Ile His Gly Ala Ala
   1130 1135 1140
   Asn Ile Leu Asn Leu Gln Lys Leu Ile Asn Ile Ser His Gln Leu
   1145 1150 1155
   Glu Ile Thr Pro Val Ser Asp Asp Ser Lys Pro Glu Ile Leu Gln
   1160 1165 1170
   Leu Leu Asn Ser Val Lys Glu His Ile Ala Glu Leu Asp Gln Glu
   1175 1180 1185
   Ile Ala Val Phe Cys Gln Lys Asn Asp
   1190 1195
<210> 27
   <211> 528
   <212> DNA
   <213> Escherichia coli
<220>
   <221> CDS
   <222> (1)..(528)
<400> 27
   atg att ttt ctc atg acg aaa gat tct ttt ctt tta cag ggc ttt tgg      48
   Met Ile Phe Leu Met Thr Lys Asp Ser Phe Leu Leu Gln Gly Phe Trp
   1               5                   10                  15
   cag ttg aaa gat aat cac gaa atg ata aaa atc aat tcc ctg tca gag      96
   Gln Leu Lys Asp Asn His Glu Met Ile Lys Ile Asn Ser Leu Ser Glu
               20                  25                  30
   atc aaa aaa gta ggc aat aaa ccc ttc aag gtt atc att gat acc tat     144
   Ile Lys Lys Val Gly Asn Lys Pro Phe Lys Val Ile Ile Asp Thr Tyr
           35                  40                  45
   cac aat cat atc ctt gat gaa gaa gcg att aaa ttt ctg gag aaa tta     192
   His Asn His Ile Leu Asp Glu Glu Ala Ile Lys Phe Leu Glu Lys Leu
       50                  55                  60
   gat gcc gag aga att att gtt ttg gca cct tat cac atc agt aaa cta     240
   Asp Ala Glu Arg Ile Ile Val Leu Ala Pro Tyr His Ile Ser Lys Leu
   65                  70                  75                  80
   aaa gct aaa gcg cct att tat ttt gtt agc cgc aaa gaa agt atc aaa     288
   Lys Ala Lys Ala Pro Ile Tyr Phe Val Ser Arg Lys Glu Ser Ile Lys
                   85                  90                  95
   aat ctt ctt gag att act tat ggt aaa cac ttg ccc cat aag aat tca     336
   Asn Leu Leu Glu Ile Thr Tyr Gly Lys His Leu Pro His Lys Asn Ser
               100                 105                 110
   caa tta tgt ttt tca cat aat cag ttc aaa att atg caa ctg att ctg     384
   Gln Leu Cys Phe Ser His Asn Gln Phe Lys Ile Met Gln Leu Ile Leu
           115                 120                 125
   aaa aat aaa aat gaa agc aat atc acg tcg acg ctc aat att tcg caa     432
   Lys Asn Lys Asn Glu Ser Asn Ile Thr Ser Thr Leu Asn Ile Ser Gln
       130                 135                 140
   caa aca tta aag att cag aaa ttc aac att atg tac aag ctg aaa cta     480
   Gln Thr Leu Lys Ile Gln Lys Phe Asn Ile Met Tyr Lys Leu Lys Leu
   145                 150                 155                 160
   aga cgt atg agc gac atc gtc acc ctg ggt atc aca tct tat ttt tag     528
   Arg Arg Met Ser Asp Ile Val Thr Leu Gly Ile Thr Ser Tyr Phe
                   165                 170                 175
<210> 28
   <211> 175
   <212> PRT
   <213> Escherichia coli
<400> 28
   Met Ile Phe Leu Met Thr Lys Asp Ser Phe Leu Leu Gln Gly Phe Trp
   1 5 10 15
   Gln Leu Lys Asp Asn His Glu Met Ile Lys Ile Asn Ser Leu Ser Glu
   20 25 30
   Ile Lys Lys Val Gly Asn Lys Pro Phe Lys Val Ile Ile Asp Thr Tyr
   35 40 45
   His Asn His Ile Leu Asp Glu Glu Ala Ile Lys Phe Leu Glu Lys Leu
   50 55 60
   Asp Ala Glu Arg Ile Ile Val Leu Ala Pro Tyr His Ile Ser Lys Leu
   65 70 75 80
   Lys Ala Lys Ala Pro Ile Tyr Phe Val Ser Arg Lys Glu Ser Ile Lys
   85 90 95
   Asn Leu Leu Glu Ile Thr Tyr Gly Lys His Leu Pro His Lys Asn Ser
   100 105 110
   Gln Leu Cys Phe Ser His Asn Gln Phe Lys Ile Met Gln Leu Ile Leu
   115 120 125
   Lys Asn Lys Asn Glu Ser Asn Ile Thr Ser Thr Leu Asn Ile Ser Gln
   130 135 140
   Gln Thr Leu Lys Ile Gln Lys Phe Asn Ile Met Tyr Lys Leu Lys Leu
   145 150 155 160
   Arg Arg Met Ser Asp Ile Val Thr Leu Gly Ile Thr Ser Tyr Phe
   165 170 175
<210> 29
   <211> 762
   <212> DNA
   <213> Escherichia coli
<220>
   <221> CDS
   <222> (1)..(762)
<400> 29
   atg tcg ctc gtt tgt tct gtt ata ttt att cat cat gcc ttc aac gct 48
   Met Ser Leu Val Cys Ser Val Ile Phe Ile His His Ala Phe Asn Ala
   1 5 10 15
   aac att tta gat aaa gat tac gcc ttc tct gac ggc gag atc ctg atg 96
   Asn Ile Leu Asp Lys Asp Tyr Ala Phe Ser Asp Gly Glu Ile Leu Met
   20 25 30
   gta gat aac gct gtt cgt acg cat ttt gaa cct tat gag cgg cat ttt    144
   Val Asp Asn Ala Val Arg Thr His Phe Glu Pro Tyr Glu Arg His Phe
           35                  40                  45
   aaa gag atc gga ttt act gaa aat acc att aaa aaa tat cta caa tgc    192
   Lys Glu Ile Gly Phe Thr Glu Asn Thr Ile Lys Lys Tyr Leu Gln Cys
       50                  55                  60
   act aac atc cag aca gtg acg gtg cct gtt cct gcg aag ttt tta cgt    240
   Thr Asn Ile Gln Thr Val Thr Val Pro Val Pro Ala Lys Phe Leu Arg
   65                  70                  75                  80
   gct tca aat gta ccg act gga ttg ctt aat gaa atg att gct tat ctc    288
   Ala Ser Asn Val Pro Thr Gly Leu Leu Asn Glu Met Ile Ala Tyr Leu
                   85                  90                  95
   aac tcg gaa gaa cgc aat cat cat aat ttt tca gaa ctt ttg ctt ttt    336
   Asn Ser Glu Glu Arg Asn His His Asn Phe Ser Glu Leu Leu Leu Phe
               100                 105                 110
   tct tgc ctg tct att ttt gcc gca tgc aaa ggt ttc att aca cta tta    384
   Ser Cys Leu Ser Ile Phe Ala Ala Cys Lys Gly Phe Ile Thr Leu Leu
           115                 120                 125
   act aac ggt gtg cta tcc gtt tct ggg aaa gtg aga aat att gtc aac    432
   Thr Asn Gly Val Leu Ser Val Ser Gly Lys Val Arg Asn Ile Val Asn
       130                 135                 140
   atg aag ccg gcg cac cca tgg aag ctg aaa gat att tgt gac tgc ctg    480
   Met Lys Pro Ala His Pro Trp Lys Leu Lys Asp Ile Cys Asp Cys Leu
   145                 150                 155                 160
   tac atc agt gaa agc ctg ttg aag aaa aaa ctt aag caa gag caa acg    528
   Tyr Ile Ser Glu Ser Leu Leu Lys Lys Lys Leu Lys Gln Glu Gln Thr
                   165                 170                 175
   aca ttc tca cag att ctt tta gat gca aga atg cag cac gca aaa aat    576
   Thr Phe Ser Gln Ile Leu Leu Asp Ala Arg Met Gln His Ala Lys Asn
               180                 185                 190
   ttg ata cgc gta gaa ggt tca gtc aat aaa att gcc gaa caa tgt ggt    624
   Leu Ile Arg Val Glu Gly Ser Val Asn Lys Ile Ala Glu Gln Cys Gly
           195                 200                 205
   tat gcc agt aca tct tat ttt att tat gcg ttc cgc aaa cat ttc ggc    672
   Tyr Ala Ser Thr Ser Tyr Phe Ile Tyr Ala Phe Arg Lys His Phe Gly
   210 215 220
   aac agt ccg aag aga gtt tct aag gag tac cgt tgt caa agt cac acg 720
   Asn Ser Pro Lys Arg Val Ser Lys Glu Tyr Arg Cys Gln Ser His Thr
   225 230 235 240
   ggt atg aat acg ggc aac acg atg aat gct tta gct att tga 762
   Gly Met Asn Thr Gly Asn Thr Met Asn Ala Leu Ala Ile
   245 250
<210> 30
   <211> 253
   <212> PRT
   <213> Escherichia coli
<400> 30
   Met Ser Leu Val Cys Ser Val Ile Phe Ile His His Ala Phe Asn Ala
   1 5 10 15
   Asn Ile Leu Asp Lys Asp Tyr Ala Phe Ser Asp Gly Glu Ile Leu Met
   20 25 30
   Val Asp Asn Ala Val Arg Thr His Phe Glu Pro Tyr Glu Arg His Phe
   35 40 45
   Lys Glu Ile Gly Phe Thr Glu Asn Thr Ile Lys Lys Tyr Leu Gln Cys
   50 55 60
   Thr Asn Ile Gln Thr Val Thr Val Pro Val Pro Ala Lys Phe Leu Arg
   65 70 75 80
   Ala Ser Asn Val Pro Thr Gly Leu Leu Asn Glu Met Ile Ala Tyr Leu
   85 90 95
   Asn Ser Glu Glu Arg Asn His His Asn Phe Ser Glu Leu Leu Leu Phe
   100 105 110
   Ser Cys Leu Ser Ile Phe Ala Ala Cys Lys Gly Phe Ile Thr Leu Leu
   115 120 125
   Thr Asn Gly Val Leu Ser Val Ser Gly Lys Val Arg Asn Ile Val Asn
   130 135 140
   Met Lys Pro Ala His Pro Trp Lys Leu Lys Asp Ile Cys Asp Cys Leu
   145 150 155 160
   Tyr Ile Ser Glu Ser Leu Leu Lys Lys Lys Leu Lys Gln Glu Gln Thr
   165 170 175
   Thr Phe Ser Gln Ile Leu Leu Asp Ala Arg Met Gln His Ala Lys Asn
   180 185 190
   Leu Ile Arg Val Glu Gly Ser Val Asn Lys Ile Ala Glu Gln Cys Gly
   195 200 205
   Tyr Ala Ser Thr Ser Tyr Phe Ile Tyr Ala Phe Arg Lys His Phe Gly
   210 215 220
   Asn Ser Pro Lys Arg Val Ser Lys Glu Tyr Arg Cys Gln Ser His Thr
   225 230 235 240
   Gly Met Asn Thr Gly Asn Thr Met Asn Ala Leu Ala Ile
   245 250
<210> 31
   <211> 2148
   <212> DNA
   <213> Escherichia coli
<220>
   <221> CDS
   <222> (1)..(2145)
<400> 31
   atg aac gtt att gca ata ttg aat cac atg ggg gtt tat ttt aaa gaa 48
   Met Asn Val Ile Ala Ile Leu Asn His Met Gly Val Tyr Phe Lys Glu
   1 5 10 15
   gaa ccc atc cgt gaa ctt cat cgc gcg ctt gaa cgt ctg aac ttc cag 96
   Glu Pro Ile Arg Glu Leu His Arg Ala Leu Glu Arg Leu Asn Phe Gln
   20 25 30
   att gtt tac ccg aac gac cgt gac gac tta tta aaa ctg atc gaa aac 144
   Ile Val Tyr Pro Asn Asp Arg Asp Asp Leu Leu Lys Leu Ile Glu Asn
   35 40 45
   aat gcg cgt ctg tgc ggc gtt att ttt gac tgg gat aaa tat aat ctc 192
   Asn Ala Arg Leu Cys Gly Val Ile Phe Asp Trp Asp Lys Tyr Asn Leu
   50 55 60
   gag ctg tgc gaa gaa att agc aaa atg aac gag aac ctg ccg ttg tac 240
   Glu Leu Cys Glu Glu Ile Ser Lys Met Asn Glu Asn Leu Pro Leu Tyr
   65 70 75 80
   gcg ttc gct aat acg tat tcc act ctc gat gta agc ctg aat gac ctg 288
   Ala Phe Ala Asn Thr Tyr Ser Thr Leu Asp Val Ser Leu Asn Asp Leu
   85 90 95
   cgt tta cag att agc ttc ttt gaa tat gcg ctg ggt gct gct gaa gat 336
   Arg Leu Gln Ile Ser Phe Phe Glu Tyr Ala Leu Gly Ala Ala Glu Asp
   100 105 110
   att gct aat aag atc aag cag acc act gac gaa tat atc aac act att 384
   Ile Ala Asn Lys Ile Lys Gln Thr Thr Asp Glu Tyr Ile Asn Thr Ile
   115 120 125
   ctg cct ccg ctg act aaa gca ctg ttt aaa tat gtt cgt gaa ggt aaa 432
   Leu Pro Pro Leu Thr Lys Ala Leu Phe Lys Tyr Val Arg Glu Gly Lys
   130 135 140
   tat act ttc tgt act cct ggt cac atg ggc ggt act gca ttc cag aaa 480
   Tyr Thr Phe Cys Thr Pro Gly His Met Gly Gly Thr Ala Phe Gln Lys
   145 150 155 160
   agc ccg gta ggt agc ctg ttc tat gat ttc ttt ggt ccg aat acc atg 528
   Ser Pro Val Gly Ser Leu Phe Tyr Asp Phe Phe Gly Pro Asn Thr Met
                   165                 170                 175
   aaa tct gat att tcc att tca gta tct gaa ctg ggt tct ctg ctg gat      576
   Lys Ser Asp Ile Ser Ile Ser Val Ser Glu Leu Gly Ser Leu Leu Asp
               180                 185                 190
   cac agt ggt cca cac aaa gaa gca gaa cag tat atc gct cgc gtc ttt      624
   His Ser Gly Pro His Lys Glu Ala Glu Gln Tyr Ile Ala Arg Val Phe
           195                 200                 205
   aac gca gac cgc agc tac atg gtg acc aac ggt act tcc act gcg aac      672
   Asn Ala Asp Arg Ser Tyr Met Val Thr Asn Gly Thr Ser Thr Ala Asn
       210                 215                 220
   aaa att gtt ggt atg tac tct gct cca gca ggc agc acc att ctg att      720
   Lys Ile Val Gly Met Tyr Ser Ala Pro Ala Gly Ser Thr Ile Leu Ile
   225                 230                 235                 240
   gac cgt aac tgc cac aaa tcg ctg acc cac ctg atg atg atg agc gat      768
   Asp Arg Asn Cys His Lys Ser Leu Thr His Leu Met Met Met Ser Asp
                   245                 250                 255
   gtt acg cca atc tat ttc cgc ccg acc cgt aac gct tac ggt att ctt      816
   Val Thr Pro Ile Tyr Phe Arg Pro Thr Arg Asn Ala Tyr Gly Ile Leu
               260                 265                 270
   ggt ggt atc cca cag agt gaa ttc cag cac gct acc att gct aag cgc      864
   Gly Gly Ile Pro Gln Ser Glu Phe Gln His Ala Thr Ile Ala Lys Arg
           275                 280                 285
   gtg aaa gaa aca cca aac gca acc tgg ccg gta cat gct gta att acc      912
   Val Lys Glu Thr Pro Asn Ala Thr Trp Pro Val His Ala Val Ile Thr
       290                 295                 300
   aac tct acc tat gat ggt ctg ctg tac aac acc gac ttc atc aag aaa      960
   Asn Ser Thr Tyr Asp Gly Leu Leu Tyr Asn Thr Asp Phe Ile Lys Lys
   305                 310                 315                 320
   aca ctg gat gtg aaa tcc atc cac ttt gac tcc gcg tgg gtg cct tac     1008
   Thr Leu Asp Val Lys Ser Ile His Phe Asp Ser Ala Trp Val Pro Tyr
                   325                 330                 335
   acc aac ttc tca ccg att tac gaa ggt aaa tgc ggt atg agc ggt ggc     1056
   Thr Asn Phe Ser Pro Ile Tyr Glu Gly Lys Cys Gly Met Ser Gly Gly
               340                 345                 350
   cgt gta gaa ggg aaa gtg att tac gaa acc cag tcc act cac aaa ctg     1104
   Arg Val Glu Gly Lys Val Ile Tyr Glu Thr Gln Ser Thr His Lys Leu
           355                 360                 365
   ctg gcg gcg ttc tct cag gct tcc atg atc cac gtt aaa ggt gac gta     1152
   Leu Ala Ala Phe Ser Gln Ala Ser Met Ile His Val Lys Gly Asp Val
       370                 375                 380
   aac gaa gaa acc ttt aac gaa gcc tac atg atg cac acc acc act tct     1200
   Asn Glu Glu Thr Phe Asn Glu Ala Tyr Met Met His Thr Thr Thr Ser
   385                 390                 395                 400
   ccg cac tac ggt atc gtg gcg tcc act gaa acc gct gcg gcg atg atg     1248
   Pro His Tyr Gly Ile Val Ala Ser Thr Glu Thr Ala Ala Ala Met Met
                   405                 410                 415
   aaa ggc aat gca ggt aag cgt ctg atc aac ggt tct att gaa cgt gcg     1296
   Lys Gly Asn Ala Gly Lys Arg Leu Ile Asn Gly Ser Ile Glu Arg Ala
   420 425 430
   atc aaa ttc cgt aaa gag atc aaa cgt ctg aga acg gaa tct gat ggc 1344
   Ile Lys Phe Arg Lys Glu Ile Lys Arg Leu Arg Thr Glu Ser Asp Gly
   435 440 445
   tgg ttc ttt gat gta tgg cag ccg gat cat atc gat acg act gaa tgc 1392
   Trp Phe Phe Asp Val Trp Gln Pro Asp His Ile Asp Thr Thr Glu Cys
   450 455 460
   tgg ccg ctg cgt tct gac agc acc tgg cac ggc ttc aaa aac atc gat 1440
   Trp Pro Leu Arg Ser Asp Ser Thr Trp His Gly Phe Lys Asn Ile Asp
   465 470 475 480
   aac gag cac atg tat ctt gac ccg atc aaa gtc acc ctg ctg act ccg 1488
   Asn Glu His Met Tyr Leu Asp Pro Ile Lys Val Thr Leu Leu Thr Pro
   485 490 495
   ggg atg gaa aaa gac ggc acc atg agc gac ttt ggt att ccg gcc agc 1536
   Gly Met Glu Lys Asp Gly Thr Met Ser Asp Phe Gly Ile Pro Ala Ser
   500 505 510
   atc gtg gcg aaa tac ctc gac gaa cat ggc atc gtt gtt gag aaa acc 1584
   Ile Val Ala Lys Tyr Leu Asp Glu His Gly Ile Val Val Glu Lys Thr
   515 520 525
   ggt ccg tat aac ctg ctg ttc ctg ttc agc atc ggt atc gat aag acc 1632
   Gly Pro Tyr Asn Leu Leu Phe Leu Phe Ser Ile Gly Ile Asp Lys Thr
   530 535 540
   aaa gca ctg agc ctg ctg cgt gct ctg act gac ttt aaa cgt gcg ttc 1680
   Lys Ala Leu Ser Leu Leu Arg Ala Leu Thr Asp Phe Lys Arg Ala Phe
   545 550 555 560
   gac ctg aac ctg cgt gtg aaa aac atg ctg ccg tct ctg tat cgt gaa 1728
   Asp Leu Asn Leu Arg Val Lys Asn Met Leu Pro Ser Leu Tyr Arg Glu
   565 570 575
   gat cct gaa ttc tat gaa aac atg cgt att cag gaa ctg gct cag aat 1776
   Asp Pro Glu Phe Tyr Glu Asn Met Arg Ile Gln Glu Leu Ala Gln Asn
   580 585 590
   atc cac aaa ctg att gtt cac cac aat ctg ccg gat ctg atg tat cgc 1824
   Ile His Lys Leu Ile Val His His Asn Leu Pro Asp Leu Met Tyr Arg
   595 600 605
   gca ttt gaa gtg ctg ccg acg atg gta atg act ccg tat gct gca ttc 1872
   Ala Phe Glu Val Leu Pro Thr Met Val Met Thr Pro Tyr Ala Ala Phe
   610 615 620
   cag aaa gag ctg cac ggt atg acc gaa gaa gtt tac ctc gac gaa atg 1920
   Gln Lys Glu Leu His Gly Met Thr Glu Glu Val Tyr Leu Asp Glu Met
   625 630 635 640
   gta ggt cgt att aac gcc aat atg atc ctt ccg tac ccg ccg gga gtt 1968
   Val Gly Arg Ile Asn Ala Asn Met Ile Leu Pro Tyr Pro Pro Gly Val
   645 650 655
   cct ctg gta atg ccg ggt gaa atg atc acc gaa gaa agc cgt ccg gtt 2016
   Pro Leu Val Met Pro Gly Glu Met Ile Thr Glu Glu Ser Arg Pro Val
   660 665 670
   ctg gag ttc ctg cag atg ctg tgt gaa atc ggc gct cac tat ccg ggc     2064
   Leu Glu Phe Leu Gln Met Leu Cys Glu Ile Gly Ala His Tyr Pro Gly
           675                 680                 685
   ttt gaa acc gat att cac ggt gca tac cgt cag gct gat ggc cgc tat     2112
   Phe Glu Thr Asp Ile His Gly Ala Tyr Arg Gln Ala Asp Gly Arg Tyr
       690                 695                 700
   acc gtt aag gta ttg aaa gaa gaa agc aaa aaa taa                     2148
   Thr Val Lys Val Leu Lys Glu Glu Ser Lys Lys
   705                 710                 715
<210> 32
   <211> 715
   <212> PRT
   <213> Escherichia coli
<400> 32
   Met Asn Val Ile Ala Ile Leu Asn His Met Gly Val Tyr Phe Lys Glu
   1               5                   10                  15
   Glu Pro Ile Arg Glu Leu His Arg Ala Leu Glu Arg Leu Asn Phe Gln
               20                  25                  30
   Ile Val Tyr Pro Asn Asp Arg Asp Asp Leu Leu Lys Leu Ile Glu Asn
           35                  40                  45
   Asn Ala Arg Leu Cys Gly Val Ile Phe Asp Trp Asp Lys Tyr Asn Leu
       50                  55                  60
   Glu Leu Cys Glu Glu Ile Ser Lys Met Asn Glu Asn Leu Pro Leu Tyr
   65                  70                  75                  80
   Ala Phe Ala Asn Thr Tyr Ser Thr Leu Asp Val Ser Leu Asn Asp Leu
                   85                  90                  95
   Arg Leu Gln Ile Ser Phe Phe Glu Tyr Ala Leu Gly Ala Ala Glu Asp
               100                 105                 110
   Ile Ala Asn Lys Ile Lys Gln Thr Thr Asp Glu Tyr Ile Asn Thr Ile
           115                 120                 125
   Leu Pro Pro Leu Thr Lys Ala Leu Phe Lys Tyr Val Arg Glu Gly Lys
       130                 135                 140
   Tyr Thr Phe Cys Thr Pro Gly His Met Gly Gly Thr Ala Phe Gln Lys
   145                 150                 155                 160
   Ser Pro Val Gly Ser Leu Phe Tyr Asp Phe Phe Gly Pro Asn Thr Met
                   165                 170                 175
   Lys Ser Asp Ile Ser Ile Ser Val Ser Glu Leu Gly Ser Leu Leu Asp
               180                 185                 190
   His Ser Gly Pro His Lys Glu Ala Glu Gln Tyr Ile Ala Arg Val Phe
           195                 200                 205
   Asn Ala Asp Arg Ser Tyr Met Val Thr Asn Gly Thr Ser Thr Ala Asn
       210                 215                 220
   Lys Ile Val Gly Met Tyr Ser Ala Pro Ala Gly Ser Thr Ile Leu Ile
   225                 230                 235                 240
   Asp Arg Asn Cys His Lys Ser Leu Thr His Leu Met Met Met Ser Asp
   245 250 255
   Val Thr Pro Ile Tyr Phe Arg Pro Thr Arg Asn Ala Tyr Gly Ile Leu
   260 265 270
   Gly Gly Ile Pro Gln Ser Glu Phe Gln His Ala Thr Ile Ala Lys Arg
   275 280 285
   Val Lys Glu Thr Pro Asn Ala Thr Trp Pro Val His Ala Val Ile Thr
   290 295 300
   Asn Ser Thr Tyr Asp Gly Leu Leu Tyr Asn Thr Asp Phe Ile Lys Lys
   305 310 315 320
   Thr Leu Asp Val Lys Ser Ile His Phe Asp Ser Ala Trp Val Pro Tyr
   325 330 335
   Thr Asn Phe Ser Pro Ile Tyr Glu Gly Lys Cys Gly Met Ser Gly Gly
   340 345 350
   Arg Val Glu Gly Lys Val Ile Tyr Glu Thr Gln Ser Thr His Lys Leu
   355 360 365
   Leu Ala Ala Phe Ser Gln Ala Ser Met Ile His Val Lys Gly Asp Val
   370 375 380
   Asn Glu Glu Thr Phe Asn Glu Ala Tyr Met Met His Thr Thr Thr Ser
   385 390 395 400
   Pro His Tyr Gly Ile Val Ala Ser Thr Glu Thr Ala Ala Ala Met Met
   405 410 415
   Lys Gly Asn Ala Gly Lys Arg Leu Ile Asn Gly Ser Ile Glu Arg Ala
   420 425 430
   Ile Lys Phe Arg Lys Glu Ile Lys Arg Leu Arg Thr Glu Ser Asp Gly
   435 440 445
   Trp Phe Phe Asp Val Trp Gln Pro Asp His Ile Asp Thr Thr Glu Cys
   450 455 460
   Trp Pro Leu Arg Ser Asp Ser Thr Trp His Gly Phe Lys Asn Ile Asp
   465 470 475 480
   Asn Glu His Met Tyr Leu Asp Pro Ile Lys Val Thr Leu Leu Thr Pro
   485 490 495
   Gly Met Glu Lys Asp Gly Thr Met Ser Asp Phe Gly Ile Pro Ala Ser
   500 505 510
   Ile Val Ala Lys Tyr Leu Asp Glu His Gly Ile Val Val Glu Lys Thr
   515 520 525
   Gly Pro Tyr Asn Leu Leu Phe Leu Phe Ser Ile Gly Ile Asp Lys Thr
   530 535 540
   Lys Ala Leu Ser Leu Leu Arg Ala Leu Thr Asp Phe Lys Arg Ala Phe
   545 550 555 560
   Asp Leu Asn Leu Arg Val Lys Asn Met Leu Pro Ser Leu Tyr Arg Glu
   565 570 575
   Asp Pro Glu Phe Tyr Glu Asn Met Arg Ile Gln Glu Leu Ala Gln Asn
   580 585 590
   Ile His Lys Leu Ile Val His His Asn Leu Pro Asp Leu Met Tyr Arg
   595 600 605
   Ala Phe Glu Val Leu Pro Thr Met Val Met Thr Pro Tyr Ala Ala Phe
   610 615 620
   Gln Lys Glu Leu His Gly Met Thr Glu Glu Val Tyr Leu Asp Glu Met
   625 630 635 640
   Val Gly Arg Ile Asn Ala Asn Met Ile Leu Pro Tyr Pro Pro Gly Val
   645 650 655
   Pro Leu Val Met Pro Gly Glu Met Ile Thr Glu Glu Ser Arg Pro Val
   660 665 670
   Leu Glu Phe Leu Gln Met Leu Cys Glu Ile Gly Ala His Tyr Pro Gly
   675 680 685
   Phe Glu Thr Asp Ile His Gly Ala Tyr Arg Gln Ala Asp Gly Arg Tyr
   690 695 700
   Thr Val Lys Val Leu Lys Glu Glu Ser Lys Lys
   705 710 715
<210> 33
   <211> 2142
   <212> DNA
   <213> Escherichia coli
<220>
   <221> CDS
   <222> (1)..(2139)
<400> 33
   atg aac atc att gcc att atg gga ccg cat ggc gtc ttt tat aaa gat 48
   Met Asn Ile Ile Ala Ile Met Gly Pro His Gly Val Phe Tyr Lys Asp
   1 5 10 15
   gag ccc atc aaa gaa ctg gag tcg gcg ctg gtg gcg caa ggc ttt cag 96
   Glu Pro Ile Lys Glu Leu Glu Ser Ala Leu Val Ala Gln Gly Phe Gln
   20 25 30
   att atc tgg cca caa aac agc gtt gat ttg ctg aaa ttt atc gag cat 144
   Ile Ile Trp Pro Gln Asn Ser Val Asp Leu Leu Lys Phe Ile Glu His
   35 40 45
   aac cct cga att tgc ggc gtg att ttt gac tgg gat gag tac agt ctc 192
   Asn Pro Arg Ile Cys Gly Val Ile Phe Asp Trp Asp Glu Tyr Ser Leu
   50 55 60
   gat tta tgt agc gat atc aat cag ctt aat gaa tat ctc ccg ctt tat 240
   Asp Leu Cys Ser Asp Ile Asn Gln Leu Asn Glu Tyr Leu Pro Leu Tyr
   65 70 75 80
   gcc ttc atc aac acc cac tcg acg atg gat gtc agc gtg cag gat atg 288
   Ala Phe Ile Asn Thr His Ser Thr Met Asp Val Ser Val Gln Asp Met
   85 90 95
   cgg atg gcg ctc tgg ttt ttt gaa tat gcg ctg ggg cag gcg gaa gat 336
   Arg Met Ala Leu Trp Phe Phe Glu Tyr Ala Leu Gly Gln Ala Glu Asp
   100 105 110
   atc gcc att cgt atg cgt cag tac acc gac gaa tat ctt gat aac att 384
   Ile Ala Ile Arg Met Arg Gln Tyr Thr Asp Glu Tyr Leu Asp Asn Ile
   115 120 125
   aca ccg ccg ttc acg aaa gcc ttg ttt acc tac gtc aaa gag cgg aag 432
   Thr Pro Pro Phe Thr Lys Ala Leu Phe Thr Tyr Val Lys Glu Arg Lys
   130 135 140
   tac acc ttt tgt acg ccg ggg cat atg ggc ggc acc gca tat caa aaa 480
   Tyr Thr Phe Cys Thr Pro Gly His Met Gly Gly Thr Ala Tyr Gln Lys
   145 150 155 160
   agc ccg gtt ggc tgt ctg ttt tat gat ttt ttc ggc ggg aat act ctt 528
   Ser Pro Val Gly Cys Leu Phe Tyr Asp Phe Phe Gly Gly Asn Thr Leu
   165 170 175
   aag gct gat gtc tct att tcg gtc acc gag ctt ggt tcg ttg ctc gac 576
   Lys Ala Asp Val Ser Ile Ser Val Thr Glu Leu Gly Ser Leu Leu Asp
   180 185 190
   cac acc ggg cca cac ctg gaa gcg gaa gag tac atc gcg cgg act ttt 624
   His Thr Gly Pro His Leu Glu Ala Glu Glu Tyr Ile Ala Arg Thr Phe
   195 200 205
   ggc gcg gaa cag agt tat atc gtt acc aac gga aca tcg acg tcg aac 672
   Gly Ala Glu Gln Ser Tyr Ile Val Thr Asn Gly Thr Ser Thr Ser Asn
   210 215 220
   aaa att gtg ggt atg tac gcc gcg cca tcc ggc agt acg ctg ttg atc 720
   Lys Ile Val Gly Met Tyr Ala Ala Pro Ser Gly Ser Thr Leu Leu Ile
   225 230 235 240
   gac cgc aat tgt cat aaa tcg ctg gcg cat ctg ttg atg atg aac gat 768
   Asp Arg Asn Cys His Lys Ser Leu Ala His Leu Leu Met Met Asn Asp
   245 250 255
   gta gtg cca gtc tgg ctg aaa ccg acg cgt aat gcg ttg ggg att ctt 816
   Val Val Pro Val Trp Leu Lys Pro Thr Arg Asn Ala Leu Gly Ile Leu
   260 265 270
   ggt ggg atc ccg cgc cgt gaa ttt act cgc gac agc atc gaa gag aaa 864
   Gly Gly Ile Pro Arg Arg Glu Phe Thr Arg Asp Ser Ile Glu Glu Lys
   275 280 285
   gtc gct gct acc acg caa gca caa tgg ccg gtt cat gcg gtg atc acc 912
   Val Ala Ala Thr Thr Gln Ala Gln Trp Pro Val His Ala Val Ile Thr
   290 295 300
   aac tcc acc tat gat ggc ttg ctc tac aac acc gac tgg atc aaa cag 960
   Asn Ser Thr Tyr Asp Gly Leu Leu Tyr Asn Thr Asp Trp Ile Lys Gln
   305 310 315 320
   acg ctg gat gtc ccg tcg att cac ttc gat tct gcc tgg gtg ccg tac 1008
   Thr Leu Asp Val Pro Ser Ile His Phe Asp Ser Ala Trp Val Pro Tyr
   325 330 335
   acc cat ttt cat ccg atc tac cag ggt aaa agt ggt atg agc ggc gag 1056
   Thr His Phe His Pro Ile Tyr Gln Gly Lys Ser Gly Met Ser Gly Glu
   340 345 350
   cgt gtt gcg gga aaa gtg atc ttc gaa acg caa tcg acc cac aaa atg 1104
   Arg Val Ala Gly Lys Val Ile Phe Glu Thr Gln Ser Thr His Lys Met
   355 360 365
   ctg gcg gcg tta tcg cag gct tcg ctg atc cac att aaa ggc gag tat 1152
   Leu Ala Ala Leu Ser Gln Ala Ser Leu Ile His Ile Lys Gly Glu Tyr
   370 375 380
   gac gaa gag gcc ttt aac gaa gcc ttt atg atg cat acc acc acc tcg 1200
   Asp Glu Glu Ala Phe Asn Glu Ala Phe Met Met His Thr Thr Thr Ser
   385 390 395 400
   ccc agt tat ccc att gtt gct tcg gtt gag acg gcg gcg gcg atg ctg 1248
   Pro Ser Tyr Pro Ile Val Ala Ser Val Glu Thr Ala Ala Ala Met Leu
   405 410 415
   cgt ggt aat ccg ggc aaa cgg ctg att aac cgt tca gta gaa cga gct 1296
   Arg Gly Asn Pro Gly Lys Arg Leu Ile Asn Arg Ser Val Glu Arg Ala
   420 425 430
   ctg cat ttt cgc aaa gag gtc cag cgg ctg cgg gaa gag tct gac ggt 1344
   Leu His Phe Arg Lys Glu Val Gln Arg Leu Arg Glu Glu Ser Asp Gly
   435 440 445
   tgg ttt ttc gat atc tgg caa ccg ccg cag gtg gat gaa gcc gaa tgc 1392
   Trp Phe Phe Asp Ile Trp Gln Pro Pro Gln Val Asp Glu Ala Glu Cys
   450 455 460
   tgg ccc gtt gcg cct ggc gaa cag tgg cac ggc ttt aac gat gcg gat 1440
   Trp Pro Val Ala Pro Gly Glu Gln Trp His Gly Phe Asn Asp Ala Asp
   465 470 475 480
   gcc gat cat atg ttt ctc gat ccg gtt aaa gtc act att ttg aca ccg 1488
   Ala Asp His Met Phe Leu Asp Pro Val Lys Val Thr Ile Leu Thr Pro
   485 490 495
   ggg atg gac gag cag ggc aat atg agc gag gag ggg atc ccg gcg gcg 1536
   Gly Met Asp Glu Gln Gly Asn Met Ser Glu Glu Gly Ile Pro Ala Ala
   500 505 510
   ctg gta gca aaa ttc ctc gac gaa cgt ggg atc gta gta gag aaa acc 1584
   Leu Val Ala Lys Phe Leu Asp Glu Arg Gly Ile Val Val Glu Lys Thr
   515 520 525
   ggc cct tat aac ctg ctg ttt ctc ttt agt att ggc atc gat aaa acc 1632
   Gly Pro Tyr Asn Leu Leu Phe Leu Phe Ser Ile Gly Ile Asp Lys Thr
   530 535 540
   aaa gca atg gga tta ttg cgt ggg ttg acg gaa ttc aaa cgc tct tac 1680
   Lys Ala Met Gly Leu Leu Arg Gly Leu Thr Glu Phe Lys Arg Ser Tyr
   545 550 555 560
   gat ctc aac ctg cgg atc aaa aat atg cta ccc gat ctc tat gca gaa 1728
   Asp Leu Asn Leu Arg Ile Lys Asn Met Leu Pro Asp Leu Tyr Ala Glu
   565 570 575
   gat ccc gat ttc tac cgc aat atg cgt att cag gat ctg gca caa ggg 1776
   Asp Pro Asp Phe Tyr Arg Asn Met Arg Ile Gln Asp Leu Ala Gln Gly
   580 585 590
   atc cat aag ctg att cgt aaa cac gat ctt ccc ggt ttg atg ttg cgg 1824
   Ile His Lys Leu Ile Arg Lys His Asp Leu Pro Gly Leu Met Leu Arg
   595 600 605
   gca ttc gat act ttg ccg gag atg atc atg acg cca cat cag gca tgg 1872
   Ala Phe Asp Thr Leu Pro Glu Met Ile Met Thr Pro His Gln Ala Trp
       610                  615                  620
   caa cga caa att aaa ggc gaa gta gaa acc att gcg ctg gaa caa ctg      1920
   Gln Arg Gln Ile Lys Gly Glu Val Glu Thr Ile Ala Leu Glu Gln Leu
   625                 630                 635                 640
   gtc ggt aga gta tcg gca aat atg atc ctg cct tat cca ccg ggc gta      1968
   Val Gly Arg Val Ser Ala Asn Met Ile Leu Pro Tyr Pro Pro Gly Val
                   645                 650                 655
   ccg ctg ttg atg cct gga gaa atg ctg acc aaa gag agc cgc aca gta      2016
   Pro Leu Leu Met Pro Gly Glu Met Leu Thr Lys Glu Ser Arg Thr Val
               660                 665                 670
   ctc gat ttt cta ctg atg ctt tgt tcc gtc ggg caa cat tac ccc ggt      2064
   Leu Asp Phe Leu Leu Met Leu Cys Ser Val Gly Gln His Tyr Pro Gly
           675                 680                 685
   ttt gaa acg gat att cac ggc gcg aaa cag gac gaa gac ggc gtt tac      2112
   Phe Glu Thr Asp Ile His Gly Ala Lys Gln Asp Glu Asp Gly Val Tyr
       690                 695                 700
   cgc gta cga gtc cta aaa atg gcg gga taa                              2142
   Arg Val Arg Val Leu Lys Met Ala Gly
   705                 710
<210> 34
   <211> 713.
   <212> PRT
   <213> Escherichia coli
<400> 34
   Met Asn Ile Ile Ala Ile Met Gly Pro His Gly Val Phe Tyr Lys Asp
   1               5                   10                  15
   Glu Pro Ile Lys Glu Leu Glu Ser Ala Leu Val Ala Gln Gly Phe Gln
               20                  25                  30
   Ile Ile Trp Pro Gln Asn Ser Val Asp Leu Leu Lys Phe Ile Glu His
           35                  40                  45
   Asn Pro Arg Ile Cys Gly Val Ile Phe Asp Trp Asp Glu Tyr Ser Leu
       50                  55                  60
   Asp Leu Cys Ser Asp Ile Asn Gln Leu Asn Glu Tyr Leu Pro Leu Tyr
   65                  70                  75                  80
   Ala Phe Ile Asn Thr His Ser Thr Met Asp Val Ser Val Gln Asp Met
                   85                  90                  95
   Arg Met Ala Leu Trp Phe Phe Glu Tyr Ala Leu Gly Gln Ala Glu Asp
               100                 105                 110
   Ile Ala Ile Arg Met Arg Gln Tyr Thr Asp Glu Tyr Leu Asp Asn Ile
           115                 120                 125
   Thr Pro Pro Phe Thr Lys Ala Leu Phe Thr Tyr Val Lys Glu Arg Lys
       130                 135                 140
   Tyr Thr Phe Cys Thr Pro Gly His Met Gly Gly Thr Ala Tyr Gln Lys
   145                 150                 155                 160
   Ser Pro Val Gly Cys Leu Phe Tyr Asp Phe Phe Gly Gly Asn Thr Leu
   165 170 175
   Lys Ala Asp Val Ser Ile Ser Val Thr Glu Leu Gly Ser Leu Leu Asp
   180 185 190
   His Thr Gly Pro His Leu Glu Ala Glu Glu Tyr Ile Ala Arg Thr Phe
   195 200 205
   Gly Ala Glu Gln Ser Tyr Ile Val Thr Asn Gly Thr Ser Thr Ser Asn
   210 215 220
   Lys Ile Val Gly Met Tyr Ala Ala Pro Ser Gly Ser Thr Leu Leu Ile
   225 230 235 240
   Asp Arg Asn Cys His Lys Ser Leu Ala His Leu Leu Met Met Asn Asp
   245 250 255
   Val Val Pro Val Trp Leu Lys Pro Thr Arg Asn Ala Leu Gly Ile Leu
   260 265 270
   Gly Gly Ile Pro Arg Arg Glu Phe Thr Arg Asp Ser Ile Glu Glu Lys
   275 280 285
   Val Ala Ala Thr Thr Gln Ala Gln Trp Pro Val His Ala Val Ile Thr
   290 295 300
   Asn Ser Thr Tyr Asp Gly Leu Leu Tyr Asn Thr Asp Trp Ile Lys Gln
   305 310 315 320
   Thr Leu Asp Val Pro Ser Ile His Phe Asp Ser Ala Trp Val Pro Tyr
   325 330 335
   Thr His Phe His Pro Ile Tyr Gln Gly Lys Ser Gly Met Ser Gly Glu
   340 345 350
   Arg Val Ala Gly Lys Val Ile Phe Glu Thr Gln Ser Thr His Lys Met
   355 360 365
   Leu Ala Ala Leu Ser Gln Ala Ser Leu Ile His Ile Lys Gly Glu Tyr
   370 375 380
   Asp Glu Glu Ala Phe Asn Glu Ala Phe Met Met His Thr Thr Thr Ser
   385 390 395 400
   Pro Ser Tyr Pro Ile Val Ala Ser Val Glu Thr Ala Ala Ala Met Leu
   405 410 415
   Arg Gly Asn Pro Gly Lys Arg Leu Ile Asn Arg Ser Val Glu Arg Ala
   420 425 430
   Leu His Phe Arg Lys Glu Val Gln Arg Leu Arg Glu Glu Ser Asp Gly
   435 440 445
   Trp Phe Phe Asp Ile Trp Gln Pro Pro Gln Val Asp Glu Ala Glu Cys
   450 455 460
   Trp Pro Val Ala Pro Gly Glu Gln Trp His Gly Phe Asn Asp Ala Asp
   465 470 475 480
   Ala Asp His Met Phe Leu Asp Pro Val Lys Val Thr Ile Leu Thr Pro
   485 490 495
   Gly Met Asp Glu Gln Gly Asn Met Ser Glu Glu Gly Ile Pro Ala Ala
   500 505 510
   Leu Val Ala Lys Phe Leu Asp Glu Arg Gly Ile Val Val Glu Lys Thr
   515 520 525
   Gly Pro Tyr Asn Leu Leu Phe Leu Phe Ser Ile Gly Ile Asp Lys Thr
   530 535 540
   Lys Ala Met Gly Leu Leu Arg Gly Leu Thr Glu Phe Lys Arg Ser Tyr
   545 550 555 560
   Asp Leu Asn Leu Arg Ile Lys Asn Met Leu Pro Asp Leu Tyr Ala Glu
   565 570 575
   Asp Pro Asp Phe Tyr Arg Asn Met Arg Ile Gln Asp Leu Ala Gln Gly
   580 585 590
   Ile His Lys Leu Ile Arg Lys His Asp Leu Pro Gly Leu Met Leu Arg
   595 600 605
   Ala Phe Asp Thr Leu Pro Glu Met Ile Met Thr Pro His Gln Ala Trp
   610 615 620
   Gln Arg Gln Ile Lys Gly Glu Val Glu Thr Ile Ala Leu Glu Gln Leu
   625 630 635 640
   Val Gly Arg Val Ser Ala Asn Met Ile Leu Pro Tyr Pro Pro Gly Val
   645 650 655
   Pro Leu Leu Met Pro Gly Glu Met Leu Thr Lys Glu Ser Arg Thr Val
   660 665 670
   Leu Asp Phe Leu Leu Met Leu Cys Ser Val Gly Gln His Tyr Pro Gly
   675 680 685
   Phe Glu Thr Asp Ile His Gly Ala Lys Gln Asp Glu Asp Gly Val Tyr
   690 695 700
   Arg Val Arg Val Leu Lys Met Ala Gly
   705 710
<210> 35
   <211> 54
   <212> DNA
   <213> Artificial
<220>
   <223> 5'-primer for cadA
<400> 35
   tttgctttct tctttcaata ccttaacggt atagcgtgaa gcctgctttt ttat 54
<210> 36
   <211> 54
   <212> DNA
   <213> Artificial
<220>
   <223> 3'-primer for cadA
<400> 36
   agatatgact atgaacgtta ttgcaatatt gaatcacgct caagttagta taaa 54
<210> 37
   <211> 54
   <212> DNA
   <213> Artificial
<220>
   <223> 5'-primer for ldc
<400> 37
   ggaggaacac atgaacatca ttgccattat gggacctgaa gcctgctttt ttat 54
<210> 38
   <211> 53
   <212> DNA
   <213> Artificial
<220>
   <223> 3'-primer for ldc
<400> 38
   cgccattttt aggactcgta cgcggtaaac gccgtccgtc aagttagtat aaa 53

## Description

### Technical Field

The present invention relates to a method for producing an L-amino acid using a microorganism, and more specifically, to a method for producing an L-amino acid, such as L-lysine, L-threonine, L-tryptophan, etc.. L-lysine, L-threonine, and L-tryptophan are used in industry as animal feed additives, health food ingredients, and amino acid infusions.

### Bacground Art

To produce a target substance, such as an L-amino acid, etc., by fermentation using a microorganism, there are methods which use a wild-type microorganism (wild-type strain), an auxotrophic strain derived from a wild-type strain, a metabolic regulation mutant strain as one of various types of drug-resistant mutant strains derived from a wild-type strain, a strain which has the characteristics of both the auxotrophic strain and metabolic regulation mutant strain, and so forth.

In recent years, recombinant DNA technology has been used to produce target substances by fermentation. For example, the ability of a microorganism to produce an L-amino acid has been improved by enhancing the expression of a gene that encodes an L-amino acid biosynthesis enzyme (U.S. Patent No. 5168056, U.S. Patent No. 5776736), or enhancing the influx of the carbon source to the L-amino acid biosynthesis system (U.S. Patent No. 5906925).

As for the two-component system EvgAS which has been found in bacteria of *Enterobacteriaceae,* such as *Escherichia coli,* etc., the function of EvgS, which is a sensor kinase, is unknown, but EvgA which is a response regulator transcription factor, is known to regulate the transcription of many genes (Masuda, N. and Church, G. M., J. Bacteriol. 2002. 184(22):6225-6234. *Escherichia coli* gene expression responsive to levels of the response regulator EvgA.). It is also known that the transcription factor EvgA positively regulates the transcription of the *ydeO* and *gadE* genes which encode two transcription factors, YdeO and GadE, and that YdeO positively regulates the transcription of the *gadE* gene (Masuda, N. and Church, G. M., Mol. Microbiol. 2003. 48(3):699-712. Regulatory network of acid resistance genes in *Escherichia coli.;* Ma, Z., Masuda, N., and Foster, J. W., J. Bacteriol. 2004. 186(21):7378-7389. Characterization of EvgAS-YdeO-GadE branched regulatory circuit governing glutatnate-dependent acid resistance in *Escherichia coli*.). However, the production of an amino acid using a microorganism in which the expression of the *evgA, gadE,* or *ydeO* gene has been increased has not been previously reported.

### Disclosure of the Invention

An object of the present invention is to provide a method for efficiently producing an L-amino acid using a bacterial strain of the family *Enterobacteriaceae* which is capable of efficiently producing an L-amino acid.

The inventors of the present invention extensively studied in order to resolve the above-mentioned problem, resulting in the discovery that the L-amino acid-producing ability can be improved by modifying a microorganism to increase the expression of one or more of the *evgA* gene, *gadE* gene, or *ydeO* genes. These genes encode transcription factors involved in the EvgAS two-component system.

A method for producing an L-amino acid selected from the group consisting of basic amino acids, hydroxymonoaminocarboxylic acids and aromatic L-amino acids, comprising culturing in a medium a bacterium of the family *Enterobacteriaceae* which has an ability to produce an L-amino acid and which has been modified so as to increase the amount of expression of a gene selected from the group consisting of evgA gene, gadE gene, ydeO gene, and combinations thereof, wherein said genes encode transcription factors involved in the EvgAS two-component system regulon, wherein expression of at least one of the said genes has been increased by increasing the number of copies of each gene, modifying the expression regulatory sequence of said gene, or modifying the bacterium so that the expression of an evgS gene that encodes a sensor kinase is increased by increasing the number of copies of the evgS gene or modifying the expression regulatory sequence of the evgS gene, and collecting the L-amino acid from the medium, wherein the basic amino acid is selected from the group consisting of L-lysine, L-ornithine, L-arginine, L-histidine and L-citrulline, wherein the hydroxymonoaminocarboxylic acid is selected from the group consisting of L-threonine and L-serine, and wherein the aromatic L-amino acid is selected from the group consisting of L-phenylalanine, L-tyrosine, and L-tryptophan.

In one embodiment of the method as described above, the *evgA* gene is a DNA selected from the group consisting of:(a) a DNA which comprises the nucleotide sequence of SEQ ID NO:23, and (b) a DNA which hybridizes with a nucleotide sequence complementary to the nucleotide sequence of SEQ ID NO:23 under stringent conditions, comprising washing at 60°C at a salt concentration of 0.1 x SSC and 0.1% SDS, and which encodes a protein having transcription factor activity.

In one embodiment of the method as described above, the gadE gene is a DNA selected from the group consisting of: (c) a DNA which comprises the nucleotide sequence of SEQ ID NO: 27, and (d) a DNA which hybridizes with a nucleotide sequence complementary to the nucleotide sequence of SEQ ID NO:27 under stringent conditions, comprising washing at 60°C at a salt concentration of 0.1 x SSC and 0.1% SDS, and which encodes a protein having transcription factor activity.

In one embodiment of the method as described above, the ydeO gene is a DNA selected from the group consisting of: (e) a DNA which comprises the nucleotide sequence of SEQ ID NO:29, and (f) a DNA which hybridizes with a nucleotide sequence complementary to the nucleotide sequence of SEQ ID NO:29 under stringent conditions, comprising washing at 60°C at a salt concentration of 0.1 x SSC and 0.1% SDS, and which encodes a protein having transcription factor activity.

In one embodiment of the method as described above, the evgS gene is a DNA selected from the group consisting of: (g) a DNA which comprises the nucleotide sequence of SEQ ID:25, and (h) a DNA which hybridizes with a nucleotide sequence complementary to the nucleotide sequence of SEQ ID NO:25 under stringent conditions, comprising washing at 60°C at a salt concentration of 0.1 x SSC and 0.1% SDS, and which encodes a protein having phosphotransfer activity.

In one embodiment of the method as described above, the evgA gene encodes a protein selected from the group consisting of: (A) a protein comprising the amino acid sequence of SEQ ID NO:24, (B) a protein which comprises the amino acid sequence of SEQ ID NO:24 but includes substitution, deletion, insertion, addition, or inversion of 1 to 20 amino acid residues and has transcription factor activity.

In one embodiment of the method as described above, the *gadE* gene encodes a protein selected from the group consisting of: (C) a protein comprising the amino acid sequence of SEQ ID NO:28, and (D) a protein which comprises the amino acid sequence of SEQ ID NO: 28 but includes substitution, deletion, insertion, addition, or inversion of 1 to 20 amino acid residues and has transcription factor activity.

In one embodiment of the method as described above, the *ydeO* gene encodes a protein selected from the group consisting of: E) a protein comprising the amino acid sequence of SEQ ID NO:30; F) a protein which comprises the amino acid sequence of SEQ ID NO:30 but includes substitution, deletion, insertion, addition, or inversion of 1 to 20 amino acid residues and has transcription factor activity.

In one embodiment of the method as described above, the *evgS* gene encodes a protein selected from the group consisting of: (G) a protein comprising the amino acid sequence of SEQ ID NO:26; H) a protein which comprises the amino acid sequence of SEQ ID NO:26 but includes substitution, deletion, insertion, addition, or inversion of 1 to 20 amino acid residues and has phosphotransfer activity.

The method of the present invention in particular relates to the bacterium belonging to the family Enterobacteriaceae and is selected from the group consisting of the genera *Escherichia, Enterobacter, Pantoea, Klebsiella,* and Serratia. Moreover, in one embodiment of the method according to the present invention the L-amino acid is selected from the group consisting of L-lysine, L-threonine, L-tryptophan, and combinations thereof.

### Brief Description of the Drawing

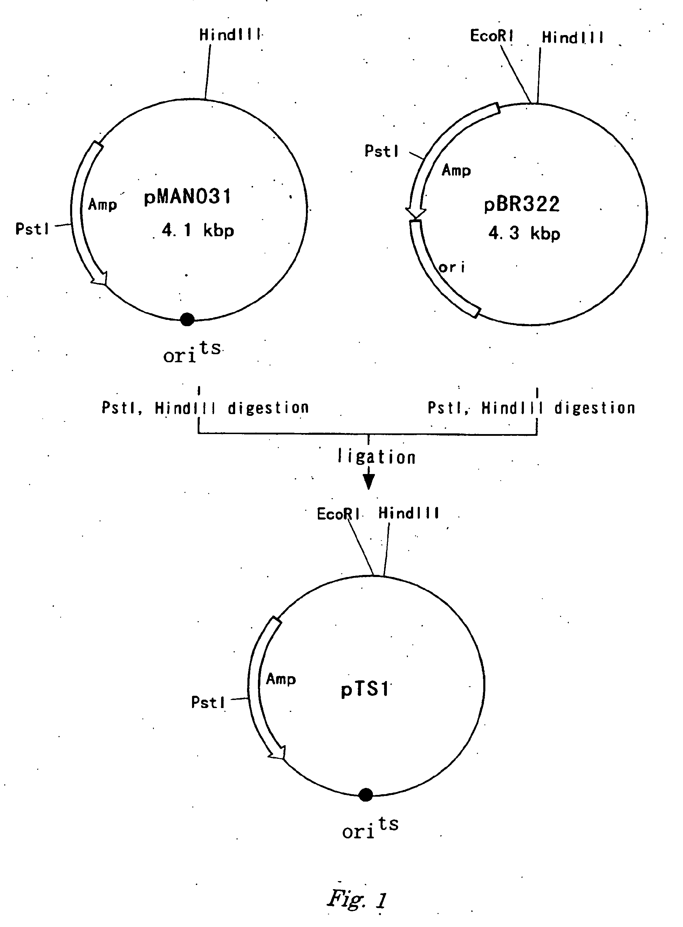

Figure 1 shows construction of plasmid vector pTS1, which has a temperature-sensitive replication origin.

### Detailed Description of the Preferred Embodiments

Hereinafter, the present invention will be explained in detail.

### 1. The microorganism for use in the present invention

The microorganism for use in the present invention is a microorganism of the family *Enterobacteriaceae* which has an ability to produce an L-amino acid and which has been modified so as to increase the expression of one or more of the *evgA, gadE,* or *ydeO* genes, which encode the transcription factor involved in the EvgAS two-component system regulon. Here, "an ability to produce an L-amino acid" means an ability to produce an L-amino acid and cause accumulation of it to a level at which it can be collected from a medium or the cells of the microorganism when the microorganism of the present invention is cultured in the medium. The microorganism for use in the present invention may have the ability to produce multiple L-amino acids. The microorganism may inherently possess the ability to produce an L-amino acid, or may be a microorganism that has been modified by mutagenesis or recombinant DNA techniques to impart the ability to produce an L-amino acid, as described below.

Also, the phrase "increase the expression of a gene" means that the transcription and/or translation of the gene is increased.

L-amino acids include basic amino acids such as L-lysine, L-ornithine, L-arginine, L-histidine, and L-citrulline; L-amino acids which are hydroxymonoaminocarboxylic acids such as L-threonine and L-serine; and aromatic L-amino acids such as L-phenylalanine, L-tyrosine, and L-tryptophan; In particular, L-lysine, L-threonine, and L-tryptophan are preferred. The microorganism for use in the present invention may be able to produce two or more amino acids.

### 1-1. Imparting L-amino acid-producing ability

The following includes a description of the method for imparting L-amino acid-producing ability to a microorganism, along with examples of microorganisms imparted with the L-amino acid-producing ability which can be used in the present invention, but the invention is not limited to these as long as they have an L-amino acid-producing ability.

The microorganism which is used in the present invention is not particularly limited, as long as it belongs to the family *Enterobacteriaceae,* such as the genera *Escherichia, Enterobacter, Pantoea, Klebsiella, Serratia, Erwinia, Salmonella, Morganella,* etc., and it has an L-amino acid-producing ability. Specifically, any microorganism belonging to the family *Enterobacteriaceae* with its classification described in the NCBI (National Center for Biotechnology Information) database may be used (http://www.ncbi.ntm.nih.gov/htbin-post/Taxonomy/wgetorg?mode=Tree&id=1236&1v1=3&keep=1 &srchmode=1&unlock). As the parent strain from the family *Enterobacteriaceae* which can be used to derive the microorganism of the present invnetion, it is particularly desirable to use bacteria which belong to the genera *Escherichia, Enterobacter,* or *Pantoea.*

There is no particular parent strain of the bacteria of the genus *Escherichia* which must be used in order to obtain the bacteria of the present invention, but those listed in Neidhardt et a!., may be used (Backmann, B. J. 1996. Derivations and Genotypes of some mutant derivatives of Escherichia coli K-12, p. 2460-2488. Table 1. In F. D. Neidhardt (ed.), Escherichia coli and Salmonella Cellular and Molecular Biology/Second Edition, American Society for Microbiology Press, Washington, D.C.). One example is *Escherichia coli.* Specific examples of *Escherichia coli* are *Escherichia coli* W3110 (ATCC 27325). *Escherichia coli* MG 1655 (ATCC 47076), etc., which are prototypes derived from wild-type strains of K 12.

These are available, for example, from the American Type Culture Collection (address: P.O. Box 1549, Manassas, VA 30108, United States of America). They are available through this organization's web site (see http:/www.atcc.org) via the use of accession numbers given to each bacterial strain. The accession numbers which correspond to each bacterial strain are given in the American Type Culture Collection's catalogue.

Examples of bacteria of the genus *Enterobacter* include *Enterobacter agglomerans* and *Enterobacter aerogenes.* An example of a bacterium of the genus *Pantoea* includes *Pantoea ananatis.* In recent years, based on 16S rRNA nucleotide sequence analysis, *Enterobacter agglomerans* has on occasion been reclassified as *Pantoea agglomerans, Pantoea ananatis,* and *Pantoea stewartii.* For the present invention, any bacterium belonging to the genus *Enterobacter* or *Pantoea* may be used as long as the bacterium is classified in the family *Enterobacteriaceae.* When *Pantoea ananatis* is bred by genetic engineering, the strains *Pantoea ananalis* AJ 13355 (FERM BP-6614), AJ13356 (FERM BP-6615), AJ 13601 (FERM BP-7207), or any derivative thereof may be employed. When isolated, these strains were identified and deposited as *Enterobacter agglomerans.* As stated above, analysis by 16S rRNA nucleotide sequence has caused them to be reclassified as *Pantoea ananatis.*

The following is a description of methods for imparting L-amino acid-producing ability to microorganisms which belong to the family *Enterobacteriaceae* and methods for increasing the L-amino-acid-producing ability in these microorganisms.

To impart the ability to produce an L-amino acid, methods conventionally employed in the breeding of the coryneform bacteria or bacteria of the genus *Escherichia* (see "Amino Acid Fermentation", Gakkai Shuppan Center (Ltd.), 1st Edition, published May 30, 1986, pp. 77-100) can be applied. Such methods include acquisition of an auxotrophic mutant, an analog-resistant strain, or a metabolic regulation mutant, or construction of a recombinant strain having enhanced expression of an L-amino acid biosynthesis enzyme. Here, in the breeding of an L-amino acid-producing bacteria, one or more properties, such as auxotrophic mutation, analog resistance, or metabolic regulation mutation may be imparted. The expression of one or more L-amino acid biosynthesis enzymes can be enhanced singly or in combinations of two or more. Furthermore, the technique of imparting properties such as auxotrophic mutation, analog resistance, or metabolic regulation mutation may be combined with the technique of enhancing the biosynthesis enzymes.

An auxotrophic mutant strain, L-amino acid analog-resistant strain, or metabolic regulation mutatant strain with the ability to produce an L-amino acid can be obtained by subjecting a parent strain or wild-type strain to a conventional mutation treatment, such as exposure to X-rays or UV irradiation, or treatment with a mutagenic agent such as N-methyl-N'-nitro-N-nitrosoguanidine (NTG) or ethyl methane sulfonate (EMS), etc., then selecting those which exhibit an autotrophic mutation, analog resistance, or metabolic regulation mutation and which also have the ability to produce an L-amino acid.

The following are examples of L-lysine-producing bacteria and their construction methods.

For example, bacteria which have L-lysine-producing ability include an L-lysine analog-resistant strain or a metabolic regulation mutant strain. Examples of the L-lysine analogs include, but are not limited to, oxalysine, lysine hydroxamate, S-(2-aminoethyl)-cysteine (AEC), γ-methyl lysine, α-chtorocaprotactam, etc.. Mutant strains which have resistance to these lysine analogs may be obtained by subjecting bacteria belonging to the genus *Escherichia* to a conventional artificial mutagenesis treatment. Examples of L-lysine-producing bacteria include *Escherichia coli* AJ11442 strain (FERM BP-1543, NRRL B-12185; see JP56-18596A and U.S. Patent No. 4,346,110); *Escherichia coli* VL611 strain (EP1016710), etc. The *Escherichia coli* WC196 strain (see WO96/17930) may also be used as an L-lysine producing bacterium. The WC196 strain was bred by imparting AEC resistance to the W3110 strain derived from *Escherichia coli* K-12. This strain was named *Escherichia coli* AJ 13069, and was deposited on December 6, 1994 at the National Institute of Bioscience and Human Technology of the Agency of Industrial Science and Technology (currently, International Patent Organism Depositary, National Institute of Advanced Industrial Science and Technology; Chuo 6, 1-1, Higashi 1-chome, Tsukuba-shi, Ibarakiken 305-8566, Japan) under Accession No. FERM P-14690. Then, it was converted to an international deposit under the Budapest Treaty on September 29, 1995, and given Accession. No. FERM BP-5252.

L-lysine-producing bacteria can also be constructed by increasing an L-lysine biosynthetic enzyme activity. Increasing the activity of these enzymes can be achieved by increasing the number of copies of a gene encoding the enzyme in the cells, or by modifying an expression regulatory sequence.

Examples of genes encoding L-lysine biosynthesis enzymes include, but are not limited to, genes encoding dihydrodipicolinate synthase (*dapA*), aspartokinase (*lysC*), dihydrodipicolinate reductase (*dapB*), diaminopimelate decarbonylase (*lysA*), diaminopimelate dehydrogenase (*ddh*) (WO96/40934 U.S. Patent No. 6,040,160), phosphoenolpyruvate carboxylase (*ppc*) (60-87788A), aspartate aminotransferase (*aspC*) (JP6-102028)B, diaminopimelate epimerase (*dapF*) (JP2003-135066A), aspartate semialdehyde dehydrogenase (*asd*) (WO00/61723), and other genes encoding enzymes of the diaminopimelate pathway; as well as a gene encoding homoaconitate hydratase (JP2000-157276A) and other genes encoding enzymes of aminoadipate pathway.

Furthermore, it is known that wild-type dihydrodipicolinate synthase (DDPS) and aspartokinase (AK) are suppressed by feedback inhibition by L-lysine; therefore, when dapA and lysC are used, it is preferable to use mutant genes encoding dihydrodipicotinate synthase and aspartokinase, respectively, that are resistant to the feedback inhibition by L-lysine (EP 0733710, US5,932,453).

Examples of the DNA encoding mutant dihydrodipicolinate synthase that is resistant to feedback inhibition by L-lysine include a DNA encoding DDPS having an amino acid sequence wherein the 118th histidine residue is substitued with tyrosine. (U.S. 5,661,012 and 6,040,160). Furthermore, examples of the DNA encoding a mutant AK that is resistant to feedback inhibition by L-lysine include a DNA encoding AK having the amino acid sequence wherein the 352-threonine residue is substituted with isoleucine. (U.S. 5,661,412 and 6,040,160). These mutant DNAs can be obtained by site-directed mutagenesis using PCR, or the like.

The following is an example of a technique for imparting an L-lysine-producing ability by introducing a gene encoding an L-lysine biosynthesis enzyme into a host. That is, recombinant DNA is prepared by ligating a gene fragment that encodes the L-lysine biosynthesis gene with a vector that functions in the chosen host microorganism, preferably a multi copy vector, and transforming the host with the recombinant vector. Due to the transformation, the copy number of the gene encoding the L-lysine biosynthesis enzyme in the host cell increases, increasing the expression and consequently increasing the enzymatic activity.

The genes encoding L-lysine biosynthesis enzymes are not particularly specified, as long as they can be expressed in the host microorganism. Examples include genes derived from *Escherichia coli,* and genes derived from the coryneform group of bacteria. Because the entire genome sequences of *Escherichia coli* and *Corynebacterium glutamicum* have been reported, it is possible to synthesize primers based on the nucleotide sequences of these genes and obtain these genes by PCR in which the genomic DNA of a microorganism, such as *Escherichia coli* K12, etc., is used as the template.

In order to clone the genes, plasmids that autonomously replicate in the family *Enterobacteriaceae* can be used. Examples include pBR322, pTWV228 (Takara Bio Inc.), pMW119 (Nippon Gene Co., Ltd.), pUC19, pSTV29 (Takara Bio Inc.), RSF1010 (Gene, 75:271-288 (1989)), etc. In addition to these, a phage vector may also be used.

To ligate the target gene to the above-mentioned vector, the vector is digested with a restriction enzyme matched to the end of the DNA fragment containing the target gene. The ligation is usually conducted with a ligase such as T4 DNA ligase. Target genes may be located on separate vectors, respectively, or located on the same vector. The usual methods known to those skilled in the art can be employed for digesting and ligating a DNA, as well as for preparing genomic DNA, performing PCR, preparing plasmid DNA, transformation, designing oligonucleotide primers, etc.. These methods are described in Sambrook, J., and Russell, D. W. Molecular Cloning A Laboratory Manual/Third Edition. New York: Cold Spring Harbor Laboratory Press (2001), etc.. Any method which achieves adequate transformation efficiency may be employed to introduce the recombinant DNA that has been prepared as described above into the host microorganism. An example includes electroporation (Can. J. Microbiol. 43:197-201 (1997)). An example ofa plasmid prepared using this method includes the Lys-producing plasmid pCABD2 which contains the *dapA, dapB,* and *LysC* genes (WO 01/53459).

Enhancing the expression of genes encoding L-lysine biosynthesis enzymes can also be achieved by introducing multiple copies of the target gene into the genomic DNA of a microorganism. Multiple copies of the target gene can be introduced into the genomic DNA of the microorganism by using a sequence which is present in multiple copies on the genomic DNA as a target in homologous recombination. Such site-specific introduction of mutations based on gene substitution using homologous recombination has been described. Either a linear DNA or a plasmid containing a temperature-sensitive replication origin can be used in such methods (U.S. Patent 6,303,383 and JP05-007491A). Repetitive DNA and inverted repeats present on the ends of transposable elements can be employed as sequences which are present in multiple copies on genomic DNA. An L-lysine biosynthesis gene may be ligated in tandem with a gene which is nativeto the genome, or it may be introduced into a non essential region on the genome or a gene region in which the L-lysine yield will be improved by deletion. Or, as disclosed in U.S. Patent 5,595,889, the target gene may also be located on a transposon, which is then transferred to introduce multiple copies onto the genomic DNA. With either method, the number of copies of the target gene in the transformant increases, so that the enzymatic activity of the L-lysine biosynthesis increases.

In addition to the above-described genetic amplification, an increase in the L-lysine biosynthesis enzyme activity can be achieved by replacing an expression regulatory sequence of the target gene such as a promoter, etc., with a stronger one (see JP1-215280A). For example, *lac* promoter, *trp* promoter, *trc* promoter, *tac* promoter, *araBA* promoter, lambda phage PR promoter, PL promoter, *tet* promoter, T7 promoter, ϕ10 promoter, etc., are known as strong promoters. Substitution with these promoters enhances expression of the target gene, thus increasing the enzymatic activity. Examples of strong promoters and methods for evaluating strength of promoters are described in Goldstein et al. (Biotechnol. Annu. Rev., 1, 105-128, (1995) Prokaryotic promoters in biotechnology.), etc.

An increase in the activity of the target enzyme can also be achieved by modifying an element which is involved in the regulation of expression of the gene that encodes the target enzyme, for example, an operator or repressor (Hamilton et al, J. Bacteriol. 171:4617-4622 (1989)). As disclosed in WO 00/18935, it is possible to substitute several bases in the promoter region of a target gene to modify and strengthen it. Furthermore, substituting several nucleotides in the spacer between the ribosome binding site (RBS) and the start codon, particularly in the sequence immediately upstream of the start codon, is known to have a strong effect on mRNA translation efficiency. Therefore, these regions may be modified to increase transcription efficiency. The expression regulatory regions of the target gene such as a promoter, etc., can be determined by promoter probe vectors and gene analysis software such as GENETYX (GENETYX CORPORATION), etc.. Expression of the target gene can be increased by substituting or modifying these promoters. Substitution of expression regulatory sequences can be conducted, for example, in the same manner as in the above-described gene substitution employing temperature-sensitive plasmids. The Red-driven.integration method (W02005/010175) may also be used.

Furthermore, in L-amino-acid-producing bacteria, the activity of an enzyme catalyzing a reaction to produce a compound other than the target L-amino acid, which branches off from the biosynthesis pathway of the L-amino acid, or the activity of an enzyme has a negative effect on the synthesis or accumulation of the L-amino acid, may be reduced or deleted. In the L-lysine production, such enzymes include homoserine dehydrogenase(*thrA*), lysine decarboxylase (*cadA, ldcC*), and malic enzyme(*sfcA, b2463*). The strains with said reduced or deficient enzymatic activity are disclosed in WO 95/23864, WO96/17930, WO2005/010175, etc..

To reduce or delete the activities of these enzymes, a mutant which reduces or deletes said enzyme activity in a cell may be introduced into the gene of the above-mentioned enzyme on the genome, using an ordinary mutatagenisis method or genetic recombination techniques. This approach to the introduction of a mutant can be achieved, for example, by deleting a gene that encodes an enzyme on the genome by genetic recombination, or by modifying an expression regulatory sequence such as a promoter or a Shine-Dalgamo (SD) sequence, etc.. This can also be achieved by introducing an amino acid substitution (missense mutation) or stop codon (nonsense mutation) in the region encoding the enzyme on the genome, by introducing a frameshift mutation to add or delete 1-2 bases, or by deleting a part of the gene or the entire region (J. Biol. Chem. 272:8611-8617 (1997)). Also, the enzyme activity can be reduced or deleted by constructing a gene that encodes the mutant enzyme in which a part or the entire coding region has been deleted, and then substituting the normal gene on the genome with said gene by homologous recombination, etc., or introducing a transposon or IS element into said gene. The following methods may be used to introduce a mutation which reduces or deletes the above-mentioned enzyme activity by genetic recombination. An isolated DNA containing the target gene is mutated so that the resulting mutant gene does not produce an enzyme that functions normally. Then a microorganism which belongs to the family *Enterobacteriaceae* is transformed with the DNA containing the mutated gene to cause recombination between the mutated gene and the target gene on the genome. Thus, the target gene on the genome may be substituted with the mutated gene. For gene substitution using this kind of homologous recombination, there are methods which employ linear DNA, such as the method called "Red-driven integration" (Datsenko, K. A, and Wanner, B. L. Proc. Natl. Acad. Sci. U S A. 97:6640-6645 (2000)), a method combining the Red-driven integration method and the λ phage excisive system (Cho, E. H., Gumport, R. I., Gardner, J. F. J. Bacteriol. 184: 5200-5203 (2002)) (see WO2005/010175), etc.; and there are methods which employ a plasmid containing a temperature-sensitive replication origin (U.S. Patent No. 6,303,383; U.S. Patent No. 5,616,480). Such site-specific introduction of mutations by gene substitution using homologous recombination as described above may also be performed using a plasmid which does not have replication ability in the chosen host.

The above-mentioned method for increasing the activity of the enzyme involving L-lysine biosynthesis and the method for lowering the enzyme activity may likewise be used in breeding other L-amino acid-producing bacteria. The following is a description of methods for breeding other L-amino acid bacteria.

The L-tryptophan-producing bacteria preferably used in the present invention include bacteria in which the activity of one or more of the following enzymes anthranilate synthase, phosphoglycerate dehydrogenase, or tryptophan synthase has been enhanced. Since anthranilate synthase and phosphoglycerate dehydrogenase both are subject to feedback inhibition by L-tryptophan and L-serine, the activities of these enzymes can be increased by retaining the desensitizing mutant enzyme. For instance, it is possible to obtain a bacterium which has a desensitizing enzyme by causing a mutation of the anthranilate synthase gene (*trpE*) and/or the phosphoglycerate dehydrogenase gene (*serA*) to prevent the feedback inhibition, then introducing the mutant gene into a microorganism belonging to the family *Enterobacteriaceae.* (U.S. Patent No. 5,618,716, U.S. Patent No. 6,180,373) A specific example of this kind of bacteria includes a transformant obtained by introducing plasmid pGH5 having a mutant *serA* that encodes desensitized phosphoglycerate dehydrogenase into *Escherichia coli* SV164 which retains desensitized anthranilate synthase (WO94/08301). Stains SV164 is obtained by introducing a gene that encodes desensitized anthranilate synthase into a *trpE* deficient strain of *Escherichia coli* KB862 (DSM7196) (See WO94/08031).

Bacteria transformed with recombinant DNA containing a tryptophan operon are also preferable. A specific example includes *Escherichia coli* transformed with a tryptophan operon containing a gene encoding desensitized anthranilate synthase (JP57-71397A, JP62-244382A, U.S. Patent No. 4,371;614). Furthermore, it is possible to enhance or impart an ability to produce L-tryptophan by enhancing the expression of a gene encoding tryptophan synthase (*trpBA*). Tryptophan synthase consists of α and β subunits that are encoded by *trpA* and *trpB,* respectively. The nucleotide sequence of *trpA* is shown in SEQ ID NO: 13 and the nucleotide sequence of *trpB* is shown in SEQ ID NO: 15.

A strain with deficient *trpR,* tryptophan operon repressor, and a strain with a mutant *trpR* are also preferable. (U.S. Patent No. 4,371,614, W02005/056776).

Another preferable L-tryptophan-producing bacterium is one in which malate synthase, isocitrate lyase, isocitrate dehydrogenase/phosphatase operon (*ace* operon) is structurally expressed or the expression of said operon has been enhanced. Specifically, it is preferable that the promoter of the *ace* operon is not suppressed by the repressor *iclR* or that the suppression by *iclR* has been removed. Such a bacterium can be obtained by disrupting the *iclR* gene or modifying the expression regulatory sequence of the *ace* operon. The *iclR* gene of *Escherichia coli* is shown in SEQ ID NO: 5. A bacterium with enhanced expression of the *ace* operon can be obtained by ligating a DNA containing the *ace* operon to a strong promoter and introducing the recombinant DNA into cells using a plasmid or by homologous recombination, or amplifying the copy number of the *ace* operon using a transposon. Genes contained in the *ace* operon include *aceB, aceA,* and *aceK.* The nucleotide sequences of *aceB, aceA* and *aceK* are shown in SEQ ID NOS: 9, 7, and 11, respectively.

Examples of L-tryptophan-producing bacteria include *Escherichia coli* AGX17 (pGX44) [NRRL B-12263], which is L-phenylalanine and L-tyrosine auxotroph, and AGX6 (pGX50) aroP [NRRL B-12264], which contains the tryptophan operon-containing plasmid pGX50 (see U:S. Patent No. 4,371,614, for both). These strains are available from the Agricultural Research Service Culture Collection, National Center for Agricultural Utilization Research (address: Peoria, Illinois 61604, USA).

L-tryptophan, L-phenylalanine, and L-tyrosine are all aromatic amino acids and share the common biosynthesis pathway. Examples of the genes encoding biosynthesis enzymes for these aromatic amino acids include deoxyarabino-heptulosonate phosphate synthase (*aroG*), 3-dehydroquinate synthase (*aroB*), shikimate dehydratase, shikimate kinase (*aroL*), 5-enolpyruvylshikimate-3-phosphate synthase (*aroA*), and chorismate synthase (*aroC*) (EP763127). Therefore, by multi-copying the gene encoding these enzymes on a plasmid or genome, the aromatic amino acid-producing ability can be improved. It is known that these genes can be controlled by a tyrosine repressor (*tyrR*), so the enzyme activity of an aromatic amino acid biosynthesis may also be increased by deleting the *tyrR* gene (See EP763127).

Examples of L-phenylalanine-producing, bacteria include *Escherichia coli* AJ12739 (tyrA::Tn10, tyrR) (VKPM B-8197) which ia *tyrA, tyrR* deficient, and strains with amplified genes encoding a phenylalanine excreting protein such as *yddG* and *yedA* (WO03/044192; US2003/0148473A1).

L- threonine-producing bacteria used in the present patent are preferably microorganisms belonging to the family *Enterobacteriaceae* wherein an L-threonine biosynthesis enzyme has been enhanced. Examples of such genes include genes encoding aspartokinase III (*lysC*), aspartate-semialdehyde dehydrogenase (*asd*), aspartokinase I (thrA included in *thr* operon), homoserine kinase (*thrB*), and threonine synthase (*thrC*). More than two of these genes may be introduced. The L-threonine biosynthesis gene may be introduced into a bacterium of the genus *Escherichia* wherein threonine degradation has been suppressed. Examples such bacteria include the TDH6 strain wherein the threonine dehydrogenase activity has been deleted (US Pat No.6,960,455), and so forth.

Activities of some of the enzymes of the L-threonine biosynthesis pathway are suppressed by L-threonine. Therefore, in order to construct an L-threonine-producing bacterium, it is preferable to modify the L-threonine biosynthesis enzyme so that the enzyme is not subject to feedback inhibition by L-threonine. The above-mentioned *thrA, thrB,* and *thrC* genes constitute a threonine operon which contains an attenuator structure. Their expression is suppressed by this attenuation. In addition, the expression of the threonine operon is inhibited by isoleucine and threonine present during the culture. The modification of the threonine operon can be achieved by removing the leader sequence in the attenuation region or the attenuator. (See Lynn, S. P., Burton, W. S.; Donohue, T. J., Gould, R. M., Gumport, R. I., and Gardner, J. F. J. Mol. Biol. 194:59-69 (1987); WO02/26993; W02005/049808).

An native promoter is present upstream of the threonine operon. The promoter may be substituted with a non-native promoter (see WO 98/04715). Alternatively, a threonine operon may be constructed so that the expression of the gene involved in threonine biosynthesis is controlled by a repressor and a promoter of lambda phage (See EP0593792). Also, to prevent feedback inhibition by L-threonine, the bacteria of the genus *Escherichia* may be modified by selecting an α-amino-β-hydroxyvaleric acid (AHV) resistant strain.

It is preferred that the copy number of the threonine operon which is modified to prevent feedback inhibition by L-threonine is increased in the host, or is ligated to a strong promoter so that the expression of the operon is enhanced. In addition to amplifying the copy number using a plasmid, the copy number can be increased by introducing the threonine operon on the genome using a transposon, Mu-phage, etc..

For the aspartokinase III gene (*lysC*), it is desirable to use a gene which is modified to prevent feedback inhibition by L-lysine. A *lysC* gene which has been modified to prevent feedback inhibition can be obtained using the method described in the U.S. Patent No. 5,932,453.

Aside from the L-threonine biosynthesis enzyme, it is desirable to enhance a gene related to the glycolytic system, TCA cycle, and respiratory chain, a gene which controls expression of these gene, and a gene which induces uptake of sugar. Examples of these genes which are effective in L-threonine production include genes encoding transhydrogenase (*pntAB*) (EP733712), phosphoenolpyruvate carboxylase (*ppc*) (WO95/06114), phosphoenolpyruvate synthase gene (pps) (EP877090), and a gene encoding pyruvate carboxylase of a coryneform bacterium or a bacterium of the genus *Bacillus* (WO99/18228, EP1092776).

It is also preferable to enhance the expression of a gene that imparts resistance to L-threonine and/or a gene that imparts resistance to L-homoserine, or to impart the L-threonine resistance and/or L-homoserine resistance to the host. Examples of such genes include the *rhtA* gene (Res. Microbiol. 154:123-135 (2003)), the *rhtB* gene (EP0994190), the *rhtC* gene (EP 1013765), and the *yfiK* and *yeaS* genes (EP 1016710). For methods of imparting L-threonine resistance to the host, refer to EP0994190, WO90/04636.

Another example of an L-threonine-producing bacterium is the *Escherichia coli* VKPM B-3996 strain (see U.S. Patent No. 5,175,107). This VKPM B-3996 strain was deposited on April 7, 1987, under Accession No. VKPM B-3996, at the Russian National Collection of Industrial Microorganisms (VKPM), GNII Genetika (address: Russia, 117545 Moscow, 1 Dorozhny proezd. 1). In addition, the VKPM B-3996 strain contains the plasmid pVIC40 (WO90/04636), which was obtained by inserting a threonine biosynthesis gene (threonine operon: *thrABC*) into a wide host-range vector plasmid pAY32 which includes a streptomycin-resistant marker (see Chistorerdov, A. Y., and Tsygankov, Y. D. Plasmid, 16, 161-167 (1986)). The *thrA* gene on pVIC40, and the feedback inhibition by the L-threonine of aspartokinase 1-homoserine dehydrogenase I has been desensitized.

A further preferable example of L-threonine-producing bacteria is the *Escherichia coli* VKPM B-5318 strain (see EP0593792). The VKPM B-5318 strain was deposited under Accession No. VKPM B-5318 at the Russian National Collection of Industrial Microorganisms (VKPM), GNII Genetika (address: Russia, 117545 Moscow, 1 Dorozhny proezd. 1) on May 3, 1990. This VKPM B-5318 strain is non-auxotrophic for isoleucine, and contains a recombinant plasmid DNA constructed so that the gene involved in threonine biosynthesis, i.e., the threonine operon wherein the attenuator and inherent transcriptional regulatory regions, has been deleted, is located downstream of the temperature-sensitive C1 repressor, PR promoter, and the N-terminus of the Cro protein of the lambda phage, and expression of the gene involved in the threonine biosynthesis is controlled by the lambda phage repressor and promoter.

Examples of L-glutamic acid-producing bacteria used in the present invention include a microorganism belonging to the family *Enterobacteriaceae* which has been modified to increase the expression of the gene encoding an enzyme that is involved in L-glutamic acid biosynthesis. The enzymes involved in the L-glutamic acid biosynthesis include glutamate dehydrogenase (hereinafter, also referred to as "GDH"), glutamine synthetase, glutamate-synthase, isocitrate dehydrogenase, aconitate hydratase, citrate synthase (hereinafter, also referred to as "CS"), phosphoenolpyruvate carboxylase (hereinafter, also referred to as "PEPC"), pyruvate carboxylase, pyruvate dehydrogenase, pyruvate kinase, phosphoenolpyruvate synthase, enolase, phosphoglycerolmutase, phosphoglycerate kinase, glyceraldehyde-3-phosphate dehydrogenase, triose phosphate isomerase, fructose-bisphosphate aldolase, phosphofructokinase, glucosephosphate isomerase, etc. Of these enzymes, one or more of CS, PEPC, and GDH is preferable, and all three are more preferable.

Examples of microorganisms belonging to the family *Enterobacteriaceae* which have been modified to enhance the expression of the citrate synthase gene, phosphoenolpyruvate carboxylase gene, and/or glutamate dehydrogenase gene using the methods described above are-disclosed in U.S. Patent Nos. 6,197,559 & 6,331,419, EP0999282.

Furthermore, microorganisms belonging to the family *Enterobacteriaceae* which have been modified to increase the activity of either or both of 6-phosphogluconate dehydratase or 2-keto-3-deoxy-6-phosphogluconate aldolasemay also be used (EP1352966).

As the microorganisms of the family *Enterobacteriaceae* having the ability to produce an L-glutamic acid, bacterium in which the activity of an enzyme that catalyzes the reaction to produce a compound other than L-glutamic acid, which branches off from the biosynthesis pathway of L-glutamic acid, has been deleted or reduced may also be used. Examples of such enzymes include 2-oxoglutarate dehydrogenase, isocitrate lyase, phosphate acetyltransferase, acetate kinase, acetohydroxy acid synthase, acetolactate synthase, formate acetyltransferase, lactate dehydrogenase, glutamate decarboxylase, 1-pyrroline dehydrogenase, etc.. Of these, it is especially preferable to reduce or delete the activity of 2-oxoglutarate dehydrogenase.

Methods for deleting or reducing the activity of 2-oxoglutarate dehydrogenase in a microorganism belonging to the family *Enterobacteriaceae* are described in U.S. Patent No. 5,573,945, U.S. Patent No. 6,197,559, and U.S. Patent No. 6,331,419. Specifically, examples of microorganisms belonging to the family *Enterobacteriaceae* wherein the activity of 2-oxoglutarate dehydrogenase has been deleted or reduced include the following:
*Pantoea ananatis* AJ13601 (FERM BP-7207)
*Klebsiella planticola* AJ 13410 strain (FERM BP-6617)
*Escherichia coli* AJ12949 (FERM BP-4881)

Examples of a preferred L-histidine-producing bacterium for use in the present invention include *Escherichia coli* FERM P-5038 and FERM P-5048. These strains contain a vector which contains genetic information involved in L-histidine biosynthesis (JP56-005099A). The other examples includes a bacterial strain which has been transformed with the amino acid expert gene *Rht* (EP1016710), and *Escherichia coli* 80 which has.been made resistant to sulfaguanidine, D, L-1,2,4-triazole-3-alanine, and streptomycin (VKPM B-7270, Russian Patent Publication No. 2119536), etc..

Microorganisms in which expression of the gene encoding an L-histidine biosynthesis pathway enzyme may be used. Examples of such genes includes the genes encoding ATP phosphoribosyltransferase (*hisG*), phosphoribosyl AMP cyclohydrolase gene (*hisI*), phosphoribosyl-ATP pyrophosphohydrolase (*hisIE*), phosphoribosylformimino-5-aminoimidazole carboxamide ribotide isomerase *(hisA),* amidotransferase gene (*hisH*), histidinol phosphate aminotransferase (*hisC*), histidinol phosphatase (*hisB*), and histidinol dehydrogenase gene (*hisD*), etc..

The preferred L-cysteine-producing bacteria for use in the present invention include bacteria in which the activity of the cystathionine β-lyase has been reduced (JP2003-169668A), and bacteria of the genus *Escherichia* that retain serine acetyltransferase, but with reduced or eliminated feedback inhibition by L-cysteine (JP11-15551A).

The preferred L-proline-producing bacteria for use in the present invention include *Escherichia coli* 702 (VKPMB-8011) which is resistant to 3,4-dehydroxyproline and azetidine-3-carboxylate, and the 702 ilvA strain (VKPM-8012 strain) which is deficient in *ilvA,* and is derived from the strain 702 (JP2002-300874A).

Examples of L-arginine-producing bacteria include *Escherichia coli* mutant strains which are resistant to α- methylmethionine, p-fluorophenylalanine, D-arginine, arginine hydroxamic acid, S-(2-aminoethyl)-cysteine, α-methyleserine, β-2-thienylalanine, or sulfaguanidine (see JP56-106598A), etc.. The *Escherichia coli* 237 strain is an L-arginine-producing bacterium that has a mutant which is resistant to feedback inhibition by L-arginine and that retains highly active N-acetyl glutamate synthase, and it is also a preferable L-arginine-producing strain (Russian PatentApplication No. 2000117677). This strain, numbered VKPM B-7925, was deposited with the Russian National Collection of Industrial Microorganisms (VKPM), GNII Genetika on April 10, 2000, and converted to an international deposit under the Budapest Treaty on May 18, 2001. The *Escherichia coli* 382 strain, which is a derivative of the 237 strain and is an L-arginine-producing bacterium with improved acetic acid assimilating ability, may also be used (JP2002-017342A). The *Escherichia coli* 382 strain, numbered VKPM B-7926, was deposited with the Russian National Collection of Industrial Microorganisms (VKPM) on April 10, 2000.

Also, as the microorganisms having L-arginine-producing ability, microorganisms with an improved expression of a gene encoding an enzyme involved in L-arginine biosynthesis may be used. Examples of L-arginine biosynthesis enzymes are one or more of N-acetyl glutamate synthase (argA), N-acetyl-glutamyl-phosphate reductase (argC), ornithine acetyltransferase (argJ), N-acetyl glutamate kinase (argB), acetyl ornithine transaminase (argD), acetyl ornithine deacetylase (argE), ornithine carbamoyl transferase (argF), argininosuccinate synthase (argG), argininosuccinate lyase (argH), and carbamoyl phosphate synthase (carAB). After each enzyme name, the name of the gene encoding it is given in parentheses. It is desirable to employ a mutation of the N-acetyl glutamate synthase gene (argA) in which L-arginine feedback inhibition has been removed by substitution of the amino acid sequence corresponding to positions 15 to 19 in the wild-type (EP1170361).

The L-leucine-producing bacteria which may be used include a bacterium of the genus *Escherichia coli* in which the branched-chain amino-acid transaminase encoded by the ilvE gene has been inactivated, and the activity of the aromatic amino acid transaminase encoded by the tyrB gene has been enhanced (EP1375655A), the *Escherichia coli* H-9068 strain (ATCC21530) which is resistant to 4-azaleucine or 5, 5, 5-trifluoroleucine, the *Escherichia coli* H-9070 strain (FERM BP-4704), the *Escherichia coli* H-9072 strain (FERM BP-4706) (U.S. Patent No. 5,744,331), an *Escherichia coli* strain in which the isopropylmalate synthase feedback inhibition by L-leucine has been desensitized (EP1067191), the *Escherichia coli* AJ11478 strain which is resistant to β-2 thienylalanine and β- hydroxyleucine (U.S. Patent No. 5,763,231), and so on.

L-isoleucine-producing bacteria include a 6-dimethyl aminopurine-resistant *Escherichia coli* mutant strain (JP5-304969A), L-isoleucine hydroxamate-resistant *Escherichia coli* mutant strain (JP5-130882A), thiaisoleucine-resistant *Escherichia coli* mutant strain (JP5-130882A), DL-ethionine-resistant *Escherichia coli* mutant strain (JP5-130882A), and arginine hydroxamate-resistant mutant strain (JP5-130882A). Examples of the recombinant *Escherichia* bacteria are those in which the genes encoding the L-isoleucine biosynthesis enzymes threonine deaminase or acetohydroxy acid synthase have been strengthened by transformation with a plasmid (JP2-458A, JP2-42988A, JP8-47397), etc.

Examples of L-valine-producing bacteria include the *Escherichia coli* VL1970 strain (U.S. Patent No. 5,658,766), etc.. Examples of L-valine-producing bacteria further include a mutant requiring lipoic acid for its growth and/or lacking H⁺-ATPase (WO96/06926), and a bacterium of the genus *Escherichia* transformed with a DNA fragment containing the *ilvGMEDA* operon, and which express at least the *ilvG*, *ilvM*, *ilvE*, and *ilvD* genes. Since the expression of the *ilvGMEDA* operon is regulated (attenuated) by L-valine and/or L-isoleucine and/or L-leucine, it is desirable that the region required for attenuation is removed or mutated in order to remove the suppression of the expression by L-valine (U.S. Patent No. 5,998,178). It is also desirable that the *ilvGMEDA* operon does not express an activity of threonine deamintase encoded by *ilvA* gene. *Escherichia coli* VL1970, having the *ileS17* mutation wherein the attenuation has been removed as described above, was deposited as Accession No. VKPM B-4411 at GNIIgenetika (Russian National Collection of Industrial Microorganisms (VKPM) Depositary, GNIIgenetika) (address: I, Dorozhny Proezd., 1, 113545, Moscow, Russia).

Aside from a gene which encodes an inherent biosynthesis enzyme, a gene which is involved in sugar uptake, sugar metabolism (glycolytic system), and energy metabolism may be enhanced in the L-amino acid-producing bacteria used in the present invention.

Examples of the genes involved in sugar metabolism are genes which encode glycolytic enzymes or proteins which uptake sugar, such as genes encoding glucose-6-phosphate isomerase (*pgi*; WO01/02542), phosphoenolpyruvate synthase *(pps;* EF877090), phosphoglucomutase (*pgm*; WO03/04598), fructose-bisphosphate aldolase (*fba*; WO03/04664), pyruvate kinase (*pykF*; WO03/008609), transaldolase (*talB*; WO03/008611), fumarase (*fum*; WO01/02545), phosphoenolpyruvate synthase (*pps*; EP877090), the non-PTS sucrose uptake systems(*csc*; EP149911), and sucrose-assimilating genes (*scrAB* operon; WO90/04636).

Examples of the genes involved in energy metabolism include the transhydrogenase gene (*pntAB*; U.S. Patent No. 5,830,716) and the cytochromoe bo type oxidase gene (*cyoABCD*; EP1070376)

The microorganism for use in the present invention can be obtained by modifying a microorganism which has the ability to produce an L-amino acid, as described above, so as to increase the expression of one or more genes selected from the *evgA* gene, *gadE* gene, or *ydeO* gene, which are genes encoding the transcription factor involved in the EvgAS two-component system. The ability to produce the target substance may be imparted after modification to increase the amount of expression of the *evgA* gene, *gadE* gene, or *ydeO* gene. An increase in the expression of the *evgA* gene, *gadE* gene, or *ydeO* gene may be an increase in the expression of the endogenous *evgA* gene, *gadE* gene, or *ydeO* gene through modification of the expression regulatory region, including promoter modification, as described later; or this may be an increase of the copy number of the *evgA* gene, *gadE* gene, or *ydeO* gene through introduction of a plasmid containing the *evgA* gene, *gadE* gene, or *ydeO* gene, or amplification of these genes on the chromosome of the bacterium, and so forth. Furthermore, a combination of these techniques may be employed.

The "EvgAS two-component system" in the present invention means a pathway which activates transcription factors via EvgA-EvgA histidine-aspartate phosphorelay regulation. More specifically, the EvgS protein (SEQ ID NO: 26), which is a sensor kinase, autophosphorylates the histidine residue of said protein, and then transfers the phosphate group to aspartate residues specific to the EvgA protein (SEQ ID NO: 24), which is a response regulator and transcription factor. The response regulator transcription factor EvgA is activated by this phosphorylation to regulate the transcription of many genes as well as to activate the transcription of the *ydeO* gene (SEQ ID NO: 29) that encodes the transcription factor YdeO protein (SEQ ID NO: 30) and/or the *gadE* gene (SEQ ID NO: 27) that encodes the transcription factor GadE protein (SEQ ID NO: 28) (J. Bacteriol. 184:6225-6234, 2002; Mol. Microbiol. 48:699-712, 2003). The YdeO protein also activates the transcription of the *gadE* gene. As a result of the transcription of the *gadE* gene being activated by the activated EvgA protein and YdeO protein, the expression of the GadE protein increases, and as a positive transcription factor, it amplifies the transcription of other genes (Microbiology. 150:61-72, 2004; J Bacteriol. 186:7378-89, 2064). Therefore, "the transcription factor involved in the EvgAS two-component regulator" in the present invention refers to the EvgA protein, GadE protein, or YdeO protein.

Here, in the present invention, "sensor kinase" is a protein which plays the role of sensing an environmental factor as a ligand, phosphorylating its own histidine residues using ATP, and then delivering this phosphate group to a response regulator. In the present invention, "response regulator" is a protein which plays the role of conveying the information within cells after being activated via receiving phosphorylation on the specific aspartate from the sensor kinase; and in many cases, it is a transcription factor.

Improvement in the expression of the gene encoding the EvgA protein, GadE protein, or YdeO protein (hereafter, *evgA*, *gadE*, or *ydeO* gene) when comparing it to the parent strain, for example, a wild-type strain or non-modified strain, can be confirmed by comparing the amount of mRNA with that in the wild-type or non-modified strain. Northern hybridization and Reverse-Transcriptase PCR (RT-PCR) can be used to confirm the amount of expression. (Sambrook, J., and Russell, D.W. Molecular Cloning A Laboratory Manual/Third Edition. New York: Cold Spring Harbor Laboratory Press (2001)). The degree of increase in enzymatic activity is not limited as long as the activity is increased as compared to that in the wild-type or non-modified strain, but it is desirable, for example, for it to be 1.5 or more times, preferably 2 or more times, or more preferably 3 or more times that of the wild-type or non-modified strain. An increase in the enzymatic activity can be confirmed if the target protein amount is increased relative to that of the non-modified or wild-type strain. This can be detected, for instance, by Western blot using an antibody. (Sambrook, J., and Russell, D.W. Molecular Cloning A Laboratory Manual/Third Edition. New York: Cold Spring Harbor Laboratory Press (2001)).

The "transcription factor" in the present invention means a transcription factor of a gene in which the expression is controlled by the EvgSA two-component regulator, and corresponds to the EvgA protein, GadE protein, and YdeO protein. The EvgA protein, GadE protein, and YdeO protein positively regulate the transcription of the gene encoding various metabolic enzymes which are under the control of the EvgSA two-component regulator. For example, the transcription of the *gnd* gene that encodes 6-phosphogluconate dehydrogenase (GND) or the *purA* gene that encodes adenylosuccinate synthase is activated by the transcription factor of the EvgSA two-component regulator. That is, by improving the produced amount of the EvgA protein, GadE protein, or YdeO protein compared to the wild-type strain or non-modified strain, the transcription amount of the gene whose expression is controller by the EvgAS two-component regulator increases. The activity of the transcription factor can be measured using the electrophoretic mobility shift assay to measure the ligation with DNA, or an in vitro measurement of transcription activity (Sambrook, J., and Russell, D.W. Molecular Cloning A Laboratory Manual/Third Edition. New York: Cold Spring Harbor Laboratory Press (2001)).

The *evgA* gene of the present invention is from a bacterium of the genus *Escherichia* and its homolog. For example, *evgA* gene of *Escherichia coli* is a gene (SEQ ID NO: 23) that encodes a protein having the amino acid sequence of SEQ ID NO: 24. (GenBank Accession No. NP_416870 [gi:16130301]).

The homologs of the *evgA* gene are derived from other microorganisms, which have a high similarity in structure to the *evgA* gene of a bacterium of the genus *Escherichia,* and which improve the ability to produce L-amino acid and exhibit transcription factor activity when introduced into the host. Examples of *evgA* homologs are the *evgA* genes of the genera *Salmonella*, *Shigella*, and *Yersinia,* etc. registered at GenBank. Furthermore, based on the homology with the genes given in the above examples, *evgA* genes may be cloned from coryneform bacteria, such as *Corynebacterium glulamicum, Brevibacterium lactofermentum*, etc.; the bacteria of the genus *Pseudomonas*, *such* as *Pseudomonas lactofermentum*, etc.; the bacteria of the genus *Pseudomonas*, such as *Pseudomonas aeruginosa*, etc.; bacteria of the genus *Mycobacterium*, such as *Mycobacterium tuberculosis*, etc.; and bacteria of the genus *Bacillus.* Ones with different gene names are acceptable as long as they are highly homologous with the *evgA* of the bacteria of the genus *Escherichia.* For example, *evgA* gene homologs include a gene that can be cloned using the synthetic oligonucleotides of SEQ ID NOS: 17 and 18.

The nucleotide sequences of the *evgA* gene homologs can be obtained by searching for a gene with high homology from a known database, based on the above-mentioned sequence information. The homology of the amino acid sequences and nucleotide sequences can be determined using, for instance, Karlin and Altschul's BLAST algorithm (Pro. Natl. Acad. Sci. USA, 90, 5873 (1993)) or FASTA (Methods Enzymol., 183, 63 (1990)). Based on this BLAST algorithm, programs called BLASTN or BLASTX have been developed (see http://www.ncbi. nih.gov/BLAST/).

The *gadE* gene of the present invention means the *gadE* gene of the bacteria of the *genus Escherichia* and its homologs. An example of *gadE* gene of *Escherichia coli* includes the gene (SEQ ID NO: 27) which encodes a protein having the amino acid sequence of SEQ ID NO: 28. (GenBank Accession No. NP_417969 [gi:16131384]).

The homologs of the *gadE* gene are, as with the *evgA* gene homologs described above, genes which are derived from other microorganisms, which have high similarity in structure to the *gadE* gene of a bacterium of the genus *Escherichia,* and which improve the ability to produce L-amino acid and exhibit transcription factor activity when introduced into the host. The *gadE* gene homologs include a gene that can be cloned using the synthetic oligonucleotides of SEQ ID NOS: 19 and 20.

The *ydeO* gene of the present invention means the *ydeO* gene of a bacterium of the genus *Escherichia* and its homologs. An example of *ydeO* gene of *Escherichia coli* includes a gene (SEQ ID NO: 29) which encodes a protein having the amino acid sequence of SEQ ID NO: 30. (GenBank Accession No. NP*_*416016 [gi:16129458]).

The homologs of *the ydeO* gene are, as with the *ydeO* gene homologs described above, genes which are derived from other microorganisms, which have high similarity in structure to the *ydeO* gene of a bacterium of the genus *Escherichia,* and which improve the ability to produce L-amino acid and exhibit transcription factor activity when introduced into the host. The *ydeO* gene homologs include a gene that can be cloned using the synthetic oligonucleotides of SEQ ID NOS: 21 and 22.

The *evgA* gene, *gadE* gene, or *ydeO* gene used in the present invention is not limited to the wild-type gene; and as long as the function of the protein encoded thereby, i.e., the transcription factor activity, is not impaired, it can also be a mutant or an artificially modified product that encodes a protein having a sequence including one or several amino acid substitutions, deletions, insertions, additions, or the like, at one or multiple positions in the amino acid sequence of SEQ ID NO: 24, 28, or 30. Here, the term "several" differs depending on the position of the amino acid residues in the stereostructure of the protein or the type of the amino acid; specifically, it means 1 to 20, preferably I to 10, and more preferably I to 5. The above substitutions, deletions, insertions, or additions of one or several amino acids are conservative mutations that preserve transcription factor activity. A conservative mutation is a mutation wherein substitution takes place mutually among Phe, Trp, Tyr, if the substitution site is an aromatic amino acid; among Leu, Ile, Val, if the substitution site is a hydrophobic amino acid; between Gln, Asn, if it is a polar amino acid; among Lys, Arg, His, if it is a basic amino acid; between Asp, Glu, if it is an acidic amino acid; and between Ser, Thr, if it is an amino acid having a hydroxyl group. Typical conservative mutations are conservative substitutions. Specific examples of substitutions that are considered to be conservative include: substitution of Ala with Ser or Thr, substitution of Arg with Gln, His, or Lys; substitution ofAsn with Glu, Gln, Lys, His, or Asp; substitution of Asp with Asn, Glu, or Gln; substitution of Cys with Ser or Ala; substitution of Gln with Asn, Glu, Lys, His, Asp, or Arg; substitution of Glu with Gly, Asn, Gln, Lys, or Asp; substitution of Gly with Pro; substitution of His with Asn, Lys, Gln, Arg, or Tyr, substitution of Ile with Leu, Met, Val, or Phe; substitution of Leu with Ile, Met, Val, or Phe; substitution of Lys with Asn, Glu, Gln, His, or Arg; substitution of Met with Ile, Leu, Val, or Phe; substitution of Phe with Trp, Tyr, Met, Ile, or Leu; substitution of Ser with Thr or Ala; substitution of Thr with Ser or Ala; substitution of Trp with Phe or Tyr; substitution of Tyr with His, Phe, or Trp; and substitution of Val with Met, Ile, or Leu. Substitutions, deletions, insertions, additions, or inversions and the like of the amino acids described above include naturally occurred mutations (mutant or variant) depending on differences in species, or individual differences of microorganisms that retain the *evgA, gadE,* and *ydeO* genes. Such genes can be obtained by modifying the nucleotide sequences shown in SEQ ID NOS: 23, 27, and 29 using, for example, site-directed mutagenesis, so that the site-specific amino acid residue in the protein encoded includes substitutions, deletions, insertions, or additions.

Moreover, as the *evgA*, *gadE*, or *ydeO* gene, a sequence that encodes a protein which is a transcription factor and which has at least 80%, preferably at least 90%, more preferably at least 95% or even more preferably at least 97% homology with the entire amino acid sequences of SEQ NOS. 24, 28, or 30. Also, the *evgA, gadE,* or *ydeO* genes in which codons are substituted with other equivalent codons that are more readily utilized by the host into which these genes are respectively introduced. Likewise, as long as the *evgA, gadE*, or *ydeO* gene product maintains the function of the transcription factor, its N terminal or C terminal may be extended or removed. For example, the length of the extension or removal may be 50 or less, preferably 20 or less, more preferably 10 or less, and even more preferably 5 or less amino acid residues. More specifically, a gene which encodes a protein having an extension or removal of from 50 to 5 amino acids of SEQ ID NOS:24, 18, or 30 from the N terminal, an extension or removal of from 50 to 5 amino acids from the C terminal may be used.

Also, the variants of the genes can be obtained by the following conventional mutation treatment. One of the mutation treatment methods is the in vitro mutation of the *evgA*, *gadE*, or *ydeO* gene, using hydroxylamine, etc. Another method employs ultraviolet or a mutation agent normally used in mutation treatment, such as N-methyl-N'-nitro-N-nitrosoguanidine (NTG) or Ethyl Methyl Sulfonate (EMS) to treat a microorganism having said gene, for example, a bacterium of the genus *Escherichia.* Whether or not these genes encode a protein that has transcription factor activity can be confirmed, for example, by expressing these genes in the appropriate cells, and investigating if transcription factor activity is present.

The *evgA*, *gadE,* or *ydeO* gene can also be a DNA which hybridizes under stringent conditions with a nucleotide sequence complemetary to the nucleotide sequence of SEQ ID NO: 23, 27, or 29, respectively, or with a probe prepared from these sequences, and which encodes proteins having transcription activity. Here, the term "stringent conditions" refers to conditions under which so-called specific hybrids are formed and nonspecific hybrids are not formed. Although it is difficult to clearly express such conditions in numbers, these can be exemplified as conditions under which highly homologous fragments of DNA, for example, DNAs having homology no less than 70 %, hybridizes each other and DNAs having homology lower than the above do not hybridize each other. Alternatively, stringent conditions are exemplified by conditions in which washing is performed once or preferably two to three times with washing conditions typical of usual Southern hybridization, that is, at a temperature and salt concentration corresponding to 60 °C, 1 x SSC, 0.1 % SDS, preferably 0.1 x SSC, 0.1 % SDS, and more preferably, 68 °C, 0.1 x SSC, 0.1 % SDS.

As a probe, a nucleotide sequence of SEQ ID NO:23, 27 or 29 or a part of these sequences may also be used. Such a probe can be prepared employing PCR in which a DNA fragment containing one of these nucleotide sequences is used as the template, with an oligonucleotide prepared based on the nucleotide sequence of SEQ ID NO: 23, 27, or 29 as the primer. For example, when a DNA fragment of a length of about 300 bp is used as the probe, the conditions of washing for the hybridization are 50 °C, 2 x SSC, and 0.1% SDS.

A modification to enhance the expression of the *evgA*, *gadE*, or *ydeO* gene can be made by employing, for example, a genetic recombination techniques to increase the number of copies of the gene in the cell. For example, a DNA fragment containing the *evgA, gadE*, or *ydeO* gene is ligated with a vector, preferably a multicopy type vector, functioning in the host microorganism to prepare the recombinant DNA, which is then introduced to the microorganism to transform it.

When the *evgA*, *gadE*, or *ydeO* gene of *Escherichia coli* is used, these genes can be obtained by PCR (PCR: polymerase chain reaction; see White, T.J. et al., Trends Genet. 5, 185 (1989)) in which the genomic DNA of *Escherichia coli* is used as the template, and using a primer prepared based on the nucleotide sequence of SEQ ID NO: 23, 27, or 29, for example, the primers shown in SEQ ID NOS: 17 and 18 for the *evgA* gene, the primers shown in SEQ ID NOS: 19 and 20 for the *gadE* gene, or the primers shown in SEQ ID NOS: 21 and 22 for the *ydeO* gene. The *evgA, gadE,* and *ydeO* genes of other microorganisms belonging to the family *Enterobacteriaceae* can also be obtained from known *evgA*, *gadE*, and *ydeO* genes in those microorganisms or *evgA*, *gadE,* and *ydeO* genes in microorganisms of other species, or the genomic DNA or genomic DNA library of microorganisms, using PCR wherein the oligonucleotide prepared based on other sequence information of the transcription factor is used as the primer, or the hybridization method wherein the oligonucleotide prepared based on the above-mentioned sequence information is used as the probe. Incidentally, the genomic DNA can be prepared from DNA donor microorganisms. For example, Saito and Miura's method, etc., (see H. Saito and K. Miura, Biochem. Biophys. Acta, 72, 619 (1963), Seibutsu Kogaku Jikkensho [Bioengineering Experiments], edited by The Society of Biotechnology, Japan, pp. 97-98, Baifukan, 1992), may be used.

Next, the recombinant DNA is prepared by ligating the *evgA, gadE,* or *ydeO* gene amplified by PCR with a DNA vector capable of functioning in the cells of the host microorganism. A vector which can function in the cells of a host microorganism is a vector which is autonomously replicable in the cells of the host microorganism. Examples of autonomously replicable vectors in cells of *Escherichia coli* include pUC19, pUC18, pHSG299, pHSG399, pHSG398, pACYC184, (pHSG and pACYC are available from Takara Bio Inc.), RSF1010, pBR322, pMW219 (pMW is available from Nippon Gene Co., Ltd.), pSTV29 (available from Takara Bio Inc.), etc..

Recombinant DNA prepared as described above may be introduced to a microorganism in accordance with any of the transformation methods which have been reported to date. For example, one method is to increase the permeability of the DNA by treating the recipient bacteria with calcium chloride, as reported with regards to *Escherichia coli* K-12 (Mandel, M. and Higa, A., J. Mol. Biol., 53, 159 (1970)). Another method is to introduce the DNA after preparing competent cells from the cells at the growth phase, as reported with regards to *Bacillus subtilis* (Duncan, C. H., Wilson, G A. and Young, F. E., Gene, 1, 153 (1977)). Also, another method which may be applied is one in which the DNA recipient microorganism, as known in relation to *Bacillus subtilis,* actinomycetes and yeasts, is changed into the protoplast or spheroplast state that can easily uptake the recombinant DNA, which is then introduced into the host (Chang, S. and Choen, S. N., Molec. Gen. Genet., 168, 111 (1979); Bibb, M. J., Ward, J. M. and Hopwood, O. A., Nature, 274, 398 (1978); Hinnen, A., Hicks, J. B. and Fink, G R., Proc. Natl. Acad. Sci. USA, 75 1929 (1978)).

The number of copies of the *evgA*, *gadE*, or *ydeO* gene can be increased by integrating multiple copies of the *evgA*, *gadE*, or *ydeO* gene as described above on a genomic DNA of the microorganism. Introduction of multiple copies of the *evgA*, *gadE*, or *ydeO* gene onto the genomic DNA of the microorganism is performed by homologous recombination, using a sequence, of which multiple copies are present on the genomic DNA, as the target. Repetitive DNA and inverted repeats present on the ends of transposable elements can be used as a sequence which exists on a chromosomal DNA in multiple copies. Also, these genes may be ligated in tandem, with the *evgA*, *gadE*, or *ydeO* gene exisiting on the genome or incorporated in multiple copies into unnecessary genes on the genome. These genes can be introduced using a temperature-sensitive vector or integration vector.

As disclosed in JP2-109985A, is is also possible to incorporate the *evgA*, *gadE*, or *ydeO* gene into a transposon, and transfer it so that multiple copies of the gene are integrated into the chromosomal DNA. Integration of the *evgA*, *gadE,* or *ydeO* gene into the genome can be confirmed by Southern hybridization using a probe having a part of the *evgA*, *gadE*, or *ydeO* gene.

Aside from increasing the copy number of the gene described above, expression of the *evgA*, *gadE*, or *ydeO* gene can also be enhanced employing the methods described in WO00/18935, for example, substituting the expression regulatory sequence such as a promoter of the *evgA*, *gadE*, or *ydeO* gene, etc., on the genomic DNA or plasmid with a stronger one, approximating the -35, -10 regions of each gene to the consensus sequence, amplifying a regulator which can enhance the expression of the *evgA*, *gadE*, or *ydeO* gene, and deleting or weakening a regulator which would decrease the expression of the *evgA*, *gadE*, or *ydeO* gene. For example, the *lac* promoter, *trp* promoter, *trc* promoter, *tac* promoter, *araBA* promoter, lambda phage PR promoter, PL promoter, *tet* promoter, T7 promoter, ϕ10 promoter, etc., are all known as strong promoters. It is also possible to introduce a nucleotide substitution, etc., into the promoter region or SD region of the *evgA, gadE*, or *ydeO* gene so to increase the promoter strength. Examples of methods for evaluating the strength of promoters and examples of strong promoters are described in articles by Goldstein et al. (Prokaryotic promoters in biotechnology. Biotechnol. Annu. Rev., 1, 105-128 (1995)), etc. Furthermore, substitution of several nucleotides in the spacer region between the ribosome binding site (RBS) and the start codon, particularly in the sequence immediately upstream of a start codon, is known to have a strong effect on mRNA translation efficiency. The expression regulatory region such as promoter etc. of the *evgA, gadE,* or *ydeO* gene, can be identified by a promoter-probe vector and gene analysis software such as GENETYX, etc. Expression of the *evgA*, *gadE*, or *ydeO* gene can be enhanced by substitutions or modifications of these promoters. Substitution of expression regulatory sequences can be conducted, for example, employing temperature-sensitive plasmids or the Red-driven integration method (WO2005/010175).

A mutation which increases transcription of a gene whose expression is regulated by the *evgA*, *gadE,* or *ydeO* gene may also be introduced to the *evgA*, *gadE*, or *ydeO* gene. Examples of mutations which increase the activity of the protein encoded by the *evgA*, *gadE,* or *ydeO* gene are a mutation of the promoter sequence, which increases the transcription amount of the *evgA*, *gadE,* or *ydeO* gene, and a mutation within the encoded region of the gene, which increases the specific activity of the transcription.

The protein encoded by the *evgA*, *gadE*, or *ydeO* gene is activated by histidine kinase EvgS. Therefore, a microorganism for use in the present invention may be one in which the amount of expression of the *evgA*, *gadE,* or *ydeO* gene has been increased by increasing the amount of expression of the *evgS* gene that encodes EvgS. Here, the *evgS* gene of the present invention means an evgS gene of a bacterium of the genus *Escherichia* and its homologs. For example, the *evgS* gene of *Escherichia coli* is exemplified by a gene (SEQ ID NO: 25) that encodes a protein having the amino acid sequence of SEQ ID NO: 26. (GenBank Accession No. NP_416871 [gi:16130302]).

The homologs of the *evgS* gene means genes which are derived from other microorganisms, which have high similarity in structure to the *evgS* gene of a bacterium of the genus *Escherichia*, and which improve the ability to produce L-amino acid and exhibit transcription factor activity when introduced into the host. Examples of *evgS* homologs include *evgS* genes of the genera *Salmonella. Shigella,* and *Yersinia* registered at GenBank. Furthermore, based on the homology with the genes given in the above examples, *evgS* genes may be cloned from coryneform bacteria, such as *Corynebacterium giuramicum. Brevibacterium lactofermentum,* etc.; bacteria of the genus *Pseudomonas,* such as *Pseudomonas aeruginosa*, etc.; bacteria of the genus *Mycobacterium,* such as *Mycobacterium tuberculosis,* etc.; and bacteria of the genus *Bacillus.* Ones with different gene names are acceptable as long as they are highly homologous with the *evgS* of the bacteria of the genus *Escherichia.* For example, in *Escherichia coli,* the *evgS* gene forms an operon with the *evgA* gene, and can be obtained along with the *evgA* gene by a PCR using the synthetic oligonucleotides of SEQ ID NOS: 17 and 18. With other microorganisms, it is exptected that these genes can be obtained likewise using the oligonucleotides of SEQ ID NOS: 17 and 18.

The *evgS* gene used in the present invention is not limited to a wild-type gene; and as long as the function of the protein, i.e., the transcription factor activity, is not impaired, it can also be a mutant or an artificially modified variant encoding a protein that has a sequence including one or several amino acid substitutions, deletions, insertions, additions, or the like at one or multiple positions in the amino acid sequence of SEQ ID NO: 26, i.e., a protein that has conservative mutation: The terms "several" and "conservative mutation" are the same as those described above regarding the *evgA* gene, *gadE* gene, and *ydeO* gene:

Increasing the amount of expression of *evgS* can be performed using the same method used for increasing the amount of expression of the *evgA*, *gadE,* and *ydeO* genes as described above. Increasing the amount of expression of the *evgA*, *gadE,* or *ydeO* gene can be performed using one of the above-mentioned means for all of the genes or a different means for each gene.

### 2. Method for producing L-amino acid

The method for producing L-amino acid of the present invention comprises culturing the microorganism for use in the present invention in a medium, producing and accumulating the L-amino acid in said medium or in the cells of the microorganism, and collecting the L-amino acid from said medium or the cells.

A medium conventionally used in the fermentation and production of L-amino acid using microorganisms may be used in the present invention. That is, an ordinary medium containing a carbon source, nitrogen source, inorganic ions, and other organic components as needed may be used. Carbon sources include a sugar such as glucose, sucrose, lactose, galactose, fructose, a starch hydrolysase, etc., an alcohol such as glycerol, solbitol, etc., an organic acid such as fumaric acid, citric acid, succinic acid, etc.. Of these, glucose, fructose, and sucrose may be preferably used. Nitrogen sources include an inorganic ammonium salt such as ammonium sulfate, ammonium chloride, ammonium phosphate, etc., an organic nitrogen such as a soybean hydrolysate, etc., ammonia gas, ammonia water, etc. As the organic micronutrient source, it is preferable that an appropriate amount of the required substances, such as vitamin B1, L-homoserine, etc., or yeast extract, etc are in the medium. In addition to these, according to necessity, small amounts of potassium phosphate, magnesium sulfate, iron ions, manganese ions, etc., can be added. The medium used in the present invention may be either a natural or synthetic medium as long as it contains a carbon source, nitrogen source, inorganic ions, and, as needed, other organic micronutrients.

An amino acid which improves growth of a microorganism or productivity of target substance may be added to the medium: For example, it is preferable that L-threonine, L-homoserine or L-isoleucine is added for L-lysine fermentation. L-isoleucine, L-lysine, L-glutamic acid or L-homoserine is added for L-threonine fermentation, and L-phenylalanine or L-tyrosine or the like is added for L-tryptophan fermentation. Concentration of added L-amino acid is around 0.01-10g/L.

It is recommended that the culturing be performed under aerobic conditions for 1-7 days at a culture temperature of 24 °C-37 °C, with a pH during the culture of 5-9. To adjust the pH, an inorganic or organic acidic or alkali substance, and ammonia gas and the like may be used. L-amino acids can be collected from the fermentation broth using a combination of a conventional ion-exchange resin method, precipitation method, and other known methods. If the L-amino acid accumulates inside the cells, the cells can be disrupted by ultrasonication, etc. Then, cell debris can be removed by centrifugal separation to obtain the supernatant, from which the L-amino acid can be recollected using an ion-exchange resin method, etc..

### Examples

The present invention will be explained more specifically below with reference to the following non limiting Examples.

### Reference example 1: Construction of L-tryptophan-producing bacteria

### 1-1. Introduction of the serA gene

The phosphoglycerate dehydrogenase gene (*serA*) exists in the plasmid pGH5 (WO9408031) and was inserted into the genome of a bacterium using transposon Mud. Plasmid pCE1134 (JP2-109985A) which contains MudII1734 was digested with *Bam*HI to remove a DNA fragment containing the *lac* operon, and the termini were blunt-ended, and a *Sma*I linker was inserted. This plasmid was redigested with *Sma*I, and self-closed to form a circular plasmid and named pMu1134. A DNA fragment containing *serA* was excised from plasmid pGH5 by cleaving with *Sca*I and *Sal*I, and the termini of the fragment were blunt-ended. Then the fragment was inserted into the *Sma*I site of the above-mentioned pMu 1134. Thus, the plasmid pMudserA which contains Mud into which the pGH5-derived *serA* gene had been inserted (named MudserA) was constructed.

Using the L-tryptophan-producing bacterial strain SV164 (WO9408031) having desensitized anthranilate synthase as a recipient bacterium, the bacterial strain L1 in which MudserA was transferred onto the genome was obtained, in accordance with an ordinary method by using acquisition of kanamycin resistance as an index. From the results of a Southern hybridization experiment, it was inferred that there was only one location where the MudserA was inserted in the L1 strain. Also, with the cloning of a genomic DNA fragment containing MudserA by PCR and the determination of its nucleotide sequence, it was determined that the insertion was at the position No. 240, 950 on the *E. coli* K-12 genome (GenBank Accession No. U00096).

### 1-2. Introduction of the trp operon

Next, the copy number of the *trp* operon was increased by inserting the *trp* operon into the genome using a transposon. The *trp* operon genes were excised from the plasmid pGX100. pGX100 was constructed by inserting into a plasmid pBR313 a DNA fragment derived from the *E. coli* MTR#2 strain (U.S. Patent No. 4,371,614) having a desensitized *trpE* gene. A DNA fragment containing the approx. 7.6 kb *trp* operon can be excised from the plasmid by cleaving with *Xho*I and *Sma*I. A DNA fragment containing the *trp* operon was excised from pGX100 by cleaving with XhoI and *SmaI,* and the termini of the fragment were blunt-ended. Then, the fragment was inserted into the *SmaI* site of the above-mentioned pCE1134. The same DNA fragment containing the *trp* operon can also be cloned directly from the *E. coli* MTR#2 strain genomic DNA, employing PCR, using primers of SEQ ID NOS: 1 and 2 shown in sequence listing. As described above, plasmid pMudtrpG' lac which contains Mud into which the *trp* operon genes derived from the MTR#2 strain had been inserted (named MudtrpG' lac) was constructed.

Prior to increasing the copy number of MudtrpG' lac by inserting MudtrpG' lac into the genome, lactose-assimilation deficiency was imparted to the host strain for the purpose of using complementation of lactose-assimilating ability as the selection marker for the strain. The *ilvG* gene of the L-threonine-producing bacterium VKPM B-3996 (U.S. Patent No. 5,175,107) was P1-tranduced into the L1 strain to impart L-valine resistance to the L1 strain (See W02005/103228). The P1 transduction was conducted in accordance with an ordinary method, transductants were spread on an M9 minimum medium (4 g/L glucose, 12.8 g/L Na₂HPO₄·7H2O, 3 g/L KH₂PO4, 0.5 g/L NaCl, 1 g/L NH₄Cl, 5 mM MgSO₄, 0.1 mM CaCl₂, 1 mg/L thiamine, 20 mg/l L-Phe, 20 mg/L L-Tyr, 20 mg/L L-Met, 3 mg/L pyridoxine, 20 mg/L L-Val, 20 mg/L tetracycline), and a Val-resistant colonies were obtained, and named L1 ValR.

lacZ98::Tn10 was P1-transduced into the L1ValR from the ME8581strain (HfrH (valS←uxuAB):lacZ98::Tn10relA1 thi-1, deposited withat National Institute of Genetics) in accordance with an ordinary method, with Tn10-derived tetracycline resistance as the indicator. The strain obtained was lactose-assimilation deficient, as expected. Next, in order to obtain a strain with deficient lactose-assimilating ability, but without Tn10, a tetracycline-sensitive strain 14-1-lac-tets was obtained from the transduced strains through the replica method. The lactose-assimilating ability was still deficient in the 14-1-lac-tets strain. When confirming the Tn10 status of said strain by Southern hybridization, no band hybridizing with the *tet* gene was detected, but a band hybridizing with the IS10 region of Tn10 was detected, whereby it was believed that IS10 remains on the *lacZ* gene.

Using pMudtrpG' lac, with the 14-1-lac-tets strain as the recipient bacterium, bacterial strain No.202 in which MudtrpG' lac was transferred into the genome was obtained, in accordance with an ordinary method, with complementation of lactose-assimilating ability as the indicator. If the inserted transposon or the gene on transposon tends to drop off from the transposon-inserted strain, a subculture may be performed on a nutrient medium to select a bacterial strain which stably retains kanamycin resistance, lactose-assimilating ability, etc. As a result of Southern hybridization, it was inferred that there was only one location where MudtrpG' lac was inserted into the No. 202 strain. Also, with the cloning of a genomic DNA fragment containing MudtrpG' lac by PCR and the determination of its nucleotide sequence, it was determined that the insertion was at the position No. 530,249 on the *E*. *coli* K-12 genome (GenBank Accession No. U00096).

Next, the scrK, scrY, scrA, scrB, and scrR genes involved in sucrose-assimilating characteristic were introduced into No.202 by P1 transduction, and the resulting bacterial strain was designated No.202 scr (see WO90/04636).

### 1-3. Construction of a plasmid for disrupting iclR

The *iclR* fragment was amplified employing a PCR using Pyrobest DNA Polymerase (Takara Shuzo) according to the attached instruction manual. For the PCR reaction, oligonucleotides of SEQ ID NOS: 3 and 4 were used as the primers, with the W3110 genome extracted using an RNA/DNA maxi Kit (QIAGEN) as the template. After the PCR, the amplified DNA fragment was purified using Wizard PCR Preps (Promega). The purified DNA fragment was digested with restriction endonucleases *Eco*RI and *Hind*III (Takara Shuzo), then underwent phenol chloroform treatment and ethanol precipitation to obtain purified DNA. This fragment and pUC18 (Takara Shuzo), which was digested with the same enzymes and then purified, were ligated using DNA ligation Kit Ver.2 (Takara Shuzo). With this ligation reaction solution, the JM109 competent cells (Takara Shuzo) were transformed, which were then spread onto an LB agar plate containing 50 µg/mL ampicillin (Amp) (Meiji Seika) (LB+Amp plate), and colonies were selected at 37 °C. The colonies were cultured in a test tube using an LB medium containing 50 µg/mL Amp at 37 °C, and the plasmid was extracted using the automatic plasmid extractor PI-50 (Kurabo).

The plasmid pUCiclR obtained was digested with restriction endonuclease EcoO65I (Takara Shuzo), and was blunt-ended and ligated using a BKL kit (Takara Shuzo). With the ligation reaction solution, JM109 was transformed, and colonies were selected as described above, and the plasmid was extracted. The plasmid obtained was digested with *Eco*RI and *Hind*III and then purified, and ligated with the temperdture-sensitive plasmid pTS1, which had been digested with the same enzymes and purified. pTS 1 was obtained by replacing a *Pst*I*-Hind*III fragment of pMAN031 (see J. Bacteriol. 162, 1196-1202 (1985), Figure 1) with a *Pst*I*-Hind*III fragment of pBR322 (Takara Shuzo). With the ligation reaction solution, JM109 was transformed, and colonies were selected on the LB+Amp plate at 30 °C. The colonies were cultured at 30 °C in a test tube using the LB medium containing 50 µg/mL Amp, and the plasmid was extracted as described above. The plasmid which generates a fragment with an expected length by digestion with *Eco*RI and *Hind*III was designated as the *iclR* disrupting plasmid pTSΔiclR.

### 1-4. Obtaining an iclR-disrupted strain

No.202scr was transformed with pTSΔiclR, and colonies were selected on the LB+Amp plate at 30 °C. After liquid culture was performed overnight at 30 °C, the culture was diluted by 10⁻³ and spread on the LB+Amp plate, and the colonies were selected at 42 °C. Specifically, the culture was spread on the LB+Amp plate and cultured at 30 °C, cells which covered 1/8 of the plate were suspended in a 2 mL LB medium, and cultured with shaking for 4-5 hours at 42 °C. The cells diluted by 10⁻⁵ were spread on the LB plate and colonies were obtained, of which some one hundred colonies were spread on the LB plate and LB+Amp plate, respectively, and their growth was confirmed, by which the Amp sensitivity and resistance were confirmed. Amp-sensitive strains were examined by colony PRC using the oligonucleotides of SEQ ID NOS: 3 and 4 as the primers. As a result, a strain in which an amplified fragment could not be cut with *Eco*065I was obtained as the *iclR*-deficient strain (No.202ΔiclR).

### Example 1: Construction of a plasmid for enhancing the evgAS, gadE, and ydeO genes

### 1-1. Construction of a plasmid for enhancing evgA and evgS genes

The entire nucleotide sequence of the genome of *Escherichia coli* (*Escherichia coli* K-12 strain) has already been reported (Science, 277, 1453-1474 (1997)), and it is known that *evgAS* forms an operon structure. Based on the nucleotide sequences of the *evgAS* genes (GenBank Accession Nos. AAC75428 and AAC75429) which were reported in the above report, an oligonucleotide shown in SEQ ID NO:17 having a *Hind*III site as a 5' primer, and an oligonucleotide shown in SEQ ID NO:18 having an *Eco*RI site as a 3' primer were synthesized. Using these primers, PCR was performed with the genomic DNA of the *Escherichia coli* W3110 strain as the template, and the amplified product was treated with restriction endonucleases *Hind*III and *Eco*RI to obtain a fragment containing the *evgAS* genes. The purified PCR product was ligated to vector pMW118 (Nippon Gene Co., Ltd.) which had been digested with *Hind*III and *Eco*RI, thus constructing the plasmid pMW-evgAS for amplifying the *evgAS* operon*.*

### 1-2. Construction of a plasmid for enhancing gadE

The entire nucleotide sequence of the genome of *Escherichia coli* (*Escherichia coli* K-12 strain) has already been reported (Science, 277, 1453-1474 (1997)). Based on the nucleotide sequence of the *gadE* gene (GenBank Accession No. AAC76537) which was reported in the above report, an oligonucleotide shown in SEQ ID NO:19 having a *BamHI* site as a 5' primer, and an oligonucleotide shown in SEQ ID NO:20 having a *Hin*dII site as a 3' primer were synthesized. Using these primers, PCR was performed with the genomic DNA of the *Escherichia coli* W3110 strain as the template, and the amplified product was treated with restriction endonucleases *Hin*dIII and *Bam*HI to obtain a fragment containing the *gadE* gene. The purified PCR product was ligated to vector pMW118 (Nippon Gene Co., Ltd.), which had been digested with *Hin*dIII and *Bam*HI, thus constructing the plasmid pMW-gadE for amplifying *gadE.*

### 1-3. Construction of a plasmid for enhancing ydeO

The entire nucleotide sequence of the genome of *Escherichia coli* (*Escherichia coli* K-12 strain) have already been reported (Science, 277, 1453-1474 (1997)). Based on the nucleotide sequence of the *ydeO* gene (GenBank Accession No. AAC74572) which was reported in the above report, an oligonucleotide shown in SEQ ID NO:21 having a *Hin*dIII site as a 5' primer, and an oligonucleotide shown in SEQ ID NO:22 having a *Eco*RI site as a 3' primer were synthesized. Using these primers, PCR was performed with the genomic DNA of the *Escherichia coli* W3110 strain as the template, and the amplified product was treated with restriction endonucleases *Hin*dIII and *Eco*RI to obtain a fragment containing the *ydeO* gene. The purified PCR product was ligated to vector pMW 118 (Nippon Gene Co., Ltd.), which had been digested with *Hin*dIII and *Eco*RI, thus constructing the plasmid pMW-ydeO for amplifying *ydeO*.

### Example 2: Effect of evgAS, ydeO, and gadE gene amplification on an L-tryptophan-producing strain of a bacterium of the genus Escherichia

The L-tryptophan-producing strain No.202ΔiclR constructed in Reference Example 1 was transformed with the *gadE*-amplifying plasmid pMW-gadE, *evgAS*-amplifying plasmid pMW-evgAS, and the *ydeO*-amplifying plasmid pMW-ydeO created in Example 1, thus obtaining ampicillin-resistant strains. After confirming that these plasmids had been introduced, a strain into which the *gadE*-amplifying plasmid pMW-gadEwas introduced was designated the No.202ΔiclR/gadE strain; a strain into which the *evgAS*-amplifying plasmid pMW-evgAS was introduced was designated the No.202ΔiclR/evgAS strain; and a strain into which the *ydeO*-amplifying plasmid pMW-ydeO was introduced was designated the No.202ΔiclR/ydeO strain.

*evgAS*, *ydeO*, or *gadE* may be amplified in the same manner using known L-tryptophan-producing bacteria other than the No.202ΔiclR strain.

The strains created as above were cultured in an LB medium containing 50 mg/L ampicillin at 37 °C until the OD600 became approx. 0.6. Then, an equal volume of 40% glycerol solutionwas added to the culture broth and stirred, then appropriate amounts were dispensed and stored at -80 °C to make the glycerol stock.

After melting the glycerol stock of these strains, 100 mL of each was homogeneously spread onto an L plate containing 50 mg/L ampicillin, and the plate was incubated at 37 °C for 24 hours. Approx. 1/8 of the cells on the plate were inoculated into a 20mL fermentation medium with 50 mL ampicillin in a 500 mL Sakaguchi flask, and cultured at 37 °C for 48 hours using a reciprocal shaking incubator. After the culture, the amount of L-tryptophan which had accumulated in the medium was measured using an amino acid-analyzer L-8500 (Hitachi). The medium composition used for the culture was as follows.

**L-tryptophan-producing medium:**

| | |
|---|---|
| Glucose | 40 g/L |
| (NH₄)₂SO₄ | 15 g/L |
| KH₂PO₄ | 1.5 g/L |
| MgSO₄·7H₂O | 0.3 g/L |
| FeSO₄·7H₂O | 0.01 g/L |
| MnSO₄·7H₂O | 0.0 1 g/L |
| Yeast Extract | 2.0 g/L |
| ThiaminHCl | 0.005 g/L |
| Pyridoxine | 0.03 g/L |
| L-Met | 0.05 g/L |
| L-Phe | 0.1 g/L |
| L-Tyr | 0.1 g/L |
| CaCO₃ | 30 g/L |

Adjusted to pH 7.0 with KOH, autoclaved at 120 °C for 20 min. Glucose and MgSO₄·7H₂O were mixed and sterilized separately from other components. CaCO₃ was added after dry-heat sterilization.

The OD600 and L-tryptophan accumulation at the 48th hour are shown in Table 1.

**Table 1: Effect of evgAS, ydeO, and gadE amplification on Trp-producing bacteria No.202ΔiclR**

| Strain | OD (600 nm) | Trp concentration (g/l) |
|---|---|---|
| No.202ΔiclR | 7.0 | 7.2 |
| No.202ΔiclR /evgAS | 7.2 | 7.6 |
| No.202ΔiclR /ydeO | 7.0 | 7.4 |
| No.202ΔiclR /gadE | 7.4 | 7.5 |

No.202ΔiclR/evgAS, No.202ΔiclR/ydeO, and No.202ΔiclR/gadE, which are the *evgAS, ydeO,* and *gadE* gene-amplified strains, each indicated an increase in the accumulation of L-tryptophan compared to the control No.202ΔiclR.

### Example 3: Effect of evgAS, ydeO, and gadE amplification on an L-threonine-producing strain of a bacterium of the genus Escherichia

*Escherichia coli* VKPM B-5318strain (see EP0593792) was used as an L-threonine-producing strain of a bacterium of the genus *Escherichia*

The VKPM B-5318 strain was transformed with the *gadE*-amplifying plasmid pMW-gadE, *evgAS*-amplifying plasmid pMW-evgAS, and the *ydeO*-amplifying plasmid pMW-ydeO constructed in Example 1, thus obtaining ampicillin-resistant strains. After confirming that these plasmids had been introduced, a strain into which the *gadE-*amplifying plasmid pMW-gadE was introduced was designated the B5318/gadE strain; a strain into which the *evgAS*-amplifying plasmid pMW-evgAS was introduced was designated the B5318/evgAS strain; and a strain into which the *ydeO*-amplifying plasmid pMW-ydeO was introduced was designated the B5318/ydeO strain.

*evgAS*, *ydeO*, or *gadE* may be amplified in the same manner using known L-threonine-producing bacteria other than the VKPM B-5318 strain.

The strains created as above were cultured in an LB medium containing 50 mg/L ampicillin at 37 °C until the OD600 became approx. 0.6. Then, an equal volume of 40% glycerol solution was added to the culture broth and stirred, then appropriate amounts were dispensed and stored at -80 °C to make the glycerol stock.

After melting the glycerol stock of these strains, 100 mL of each was homogeneously spread onto an L plate containing 50 mg/L ampicillin, and the plate was incubated at 37 °C for 24 hours. Approx. 1/8 of the cells on the plate were inoculated into a 20mL fermentation medium described below with 50 mL ampicillin in a 500 mL Sakaguchi flak, and cultured at 37 °C for 48 hours using a reciprocal shaking incubator. After the culture, the amount of L- threonine which had accumulated in the medium was measured using an amino acid-analyzer L-8500 (Hitachi). The medium composition used for the culture was as follows.

**L-threonine-producing medium:**

| | |
|---|---|
| Glucose | 40g/L |
| (NH₄)₂SO₄ | 16g/L |
| KH₂PO₄ | 1.0g/L |
| MgSO₄·7H₂O | 1.0g/L |
| FeSO₄·7H₂O | 0.01g/L |
| MnSO₄·7H₂O | 0.01g/L |
| Yeast Extract | 2.0g/L |
| CaCO₃ (Japanese Pharmacopoeia) | 30g/L |

Adjusted to pH 7.0 with KOH, autoclaved at 120 °C for 20 min. Glucose and MgSO₄·7H₂O were mixed and sterilized separately. CaCO₃ was added after dry-heat sterilization.

The OD600 and L-threonine accumulation at the 48th hour are shown in Table 2.

**Table2: Effect of evgAS, ydeO, and gadE amplification on**

| Strain | OD (600 nm) | Thr concentration (g/l) |
|---|---|---|
| B5318 | 8.1 | 6.7 |
| B5318/evgAS | 8.1 | 6.9 |
| B5318/ydeO | 7.9 | 7.0 |
| B5318/gadE | 8.4 | 7.2 |

B5318/evgAS, B5318/ydeO, and B5318/gadE, which are the *evgAS*, *ydeO*, and *gadE* gene-amplified strains, each indicated an increase in the accumulation of L-threonine compared to the control VKPM B-5318.

### Example 4: Construction of an L-lysine-producing bacterium

### 4-1. Construction of a strain in which the cadA and ldcC genes that encode lysine decarboxylase have been disrupted

First, a lysine decarboxylase non-producing strain was constructed. Lysine decarboxylase isozymes are encoded by the *cadA* gene (GenBank Accession No. NP_418555. SEQ ID NO: 31) and the *ldcC* gene (GenBank Accession No. NP_414728. SEQ ID NO: 33) (see WO96/17930). The WC196 strain (see WO96/17930), which is an AEC (S-(2-aminoethyl)-cysteine)-resistant strain, was used as the parent strain for the *Escherichia coli* L-lysine-producing strain.

The *cadA* and *ldcC* genes encoding lysine decarboxylase were deleted using a method called "Red-driven integration", which was initially developed by Datsenko and Wanner (Proc. Natl. Acad. Sci. USA. 97. 6640-6645 (2000)), and a λ phage excision system (J. Bacteriol. 184. 5200-5203 (2002)). According to the "Red-driven integration" method, it is possible to construct a gene-disrupted strain in a single step, employing a PCR product obtained by using as primers synthetic oligonucleotides for which a part of the target gene was designed at the 5' terminus and a part of the antibiotic-resistant gene was designed at the 3'terminus. Furthermore, in combination with the λ phage excision system, the antibiotic-resistant gene integrated into the gene-disrupted strain can be removed (JP2005-058227A).

### 4-2. Disruption of the codA gene

Plasmid pMW118-attL-Cm-attR (WO2005/010175) was used as the PCR template. pMW118-attL-Cm-attR is a plasmid wherein the *attL* and *attR* genes, which are the attachment sites of the λ phage, and the *cat* gene, which is an antibiotic-resistant gene, had been inserted into pMW 118 (Takara Bio Inc.) in the order of attL-cat-attR.

PCR was conducted using as primers the synthetic oligonucleotides shown in SEQ ID NOS: 35 and 36, wherein each or the primers has a sequence corresponding to each ends of *attL* and *attR* at the 3' end, and has a sequence corresponding to a part of the target *cadA* gene at the 5' end.

The PCR product was purified with agarose gel, then introduced by electroporation to an *Escherichia coli* WC 196 strain containing a plasmid pKD46, which has temperature-sensitive replication ability. Plasmid pKD46 (Proc. Natl. Acad. Sci. USA. 97. 6640-6645 (2000)) contains the λ phage DNA fragments of a total of 2154 bases, which include the gene (γ, β, exo genes) that encode Red recombinase in the λ Red homologous recombination system controlled by the arabinose-induced ParaB promoter (GenBank/EMBL Accession No. J02459, 31088th - 33241 st).

Competent cells for electroporation were prepared as follows. That is, an *Escherichia coli* WC 196 strain cultured overnight at 30 °C in an LB medium containing 100 mg/L ampicillin was diluted 100 times in a 5mL SOB medium containing ampicillin (20 mg/L) and L-arabinose (1 mM) (Molecular Cloning: Lab Manual 2nd edition, Sambrook, J., et al., Cold Spring Harbor Laboratory Press (1989)). The diluted suspension was aerated at 30 °C until the OD600 reached approx. 0.6, and then the suspension was concentrated 100 times and washed three times with 10% glycerol to ready it for use in electroporation. Electroporation was performed using 70µL competent cells and approx. 100 ng PCR product. I mL SOC medium (Molecular Cloning: Lab Manual 2nd edition, Sambrook, J., et al., Cold Spring Harbor Laboratory Press (1989)) was added to the post-electroporation cells, and cultured at 37 °C for 2.5 hours, then cultured on a plate medium of L-agar containing Cm (chloramphenicol) (25 mg/L) at 37 °C, thus selecting the Cm-resistant recombinant. Next, to remove the pKD46 plasmid, the recombinant was subcultured twice on an L-agar medium containing Cm at 42 °C, the ampicillin resistance of the colony obtained was tested, and an ampicillin-sensitive strain from which the pKD46 was omitted was obtained.

Deletion of the *cadA* gene in the mutant identified by the chloramphenicol-resistant gene was confirmed using the PCR. The *cadA* deficient strain obtained was designated the WC 196ΔcadA::att-cat strain.

Next, to remove the *att-cal* gene introduced into the *cadA* gene, a helper plasmid, pMW-intxis-ts (WO2005/010175) was used. pMW-intxis-ts is a plasmid which contains a gene that encodes λ phage integrase (Int) and a gene that encodes excisionase (Xis).

The competent cells of the WC 196ΔcadA::att-cat strain obtained as described above were prepared using an ordinary method, and were transformed with helper plasmid pMW-intxis-ts, cultured on a plate medium of L-agar containing 50 mg/L ampicillin at 30 °C, thus selecting the ampicillin-resistant strain.

Next, to remove the pMW-intxis-ts plasmid, the ampicillin-resistant transformant was subcultured twice on an L-agar medium at 42 °C, the ampicillin resistance and the chloramphenicol resistance of the colony obtained were tested, and a chloramphenicol and ampicillin-sensitive strain from which the *att-cat* and pMW-intxis-ts were removed was obtained. This strain was designated WC196ΔcadA.

### 4-3. Disruption of the ldcC gene in the WC 196ΔcadA strain

The *ldcC* gene in the WC 196ΔcadA strain was deleted in accordance with the technique described above, using primers SEQ ID NOS: 37 and 38, as the *ldcC* disrupting primers. Thus, WC 196ΔcadAΔldcC, which is a *cadA, ldcC*-disrupted strain, was obtained.

### Example 5: Effect of evgAS amplification on an L-lysine-producing strain of a bacterium of the genus Escherichia

### 5-1. Introduction of lysine-producing plasmid into the WC196ΔcadAΔldcC strain

In accordance with an ordinary method, the WC196AcadAΔldcC strain was transformed with Lysine-producing plasmid pCAB1 which contains the *dapA, dapB,* and *lysC* genes (WO01/53459), to obtain the WC196ΔcadAΔldcC/pCAB1 strain (WC196LC/pCAB1).

The WC196LC/pCAB1 strain was transformed with the *evgAS*-amplifying plasmid pMW-evgAS, created in Example 1, thus obtaining an ampicillin-resistant strain. After confirming that the prescribed plasmid had been introduced, the *evgAS*-amplifying plasmid pMW-evgAS-introduced strain was designated the WC 196LC/pCAB1/evgAS strain.

The *evgAS* may be amplified in the same manner using known L-lysine-producing bacteria other than the WC196LC/pCAB1 strain.

The strains created as above were cultured in an LB medium containing 25 mL streptomycin and 50 mg/L ampicillin at 37 °C until the OD600 became approx. 0.6.Then, an equal volume of 40% glycerol solution was added to the culture broth and stirred, then appropriate amounts were dispensed and stored at -80°C to make the glycerol stock.

### 5-2. Lysine-producing culture

After melting the glycerol stock of these strains, 100 mL of each was homogeneously spread onto an L plate containing 50 mg/L ampicillin, and the plate was incubated at 37°C for 24 hours. Approx. 1/8 of the cells on the plate obtained were inoculated into a 20mL fermentation medium described below with 50 mL ampicillin in a 500 mL Sakaguchi flask, and cultured at 37 °C for 48 hours using reciprocal shaking. After the culture, the amount of L-lysine which had accumulated in the medium was measured using a Biotech-analyzer AS210 (Sakura Seiki). The medium composition used for the culture was as follows.

| | |
|---|---|
| L-lysine-producing medium: | |
| Glucose | 40g/L |
| (NH₄)₂SO₄ | 16g/L |
| KH₂PO₄ | 1.0g/L |
| MgSO₄ ·7H₂O | 1.0g/L |
| FeSO₄ ·7H₂O | 0.01g/L |
| MnSO₄·7H₂O | 0.01g/L |
| Yeast Extract | 2.0g/L |
| CaCO₃ (Japanese Pharmacopoeia) | 30g/L |

Adjusted to pH 7.0 with KOH, autoclaved at 120 °C for 20 min. Glucose and MgSO₄·7H₂O were mixed and sterilized separately. CaCO₃ was added after dry-heat steril ization.

The OD600 and L-lysine accumulation at the 48th hour are shown in Table 3.

**Table 3: Effect of evgAS amplification on lysine-producing bacterium WC196LC/pCAB1**

| strains | OD (600 nm) | Lys concentration (g/l) |
|---|---|---|
| WC196LC/pCAB1 | 7.3 | 14.3 |
| WC196LC/pcABllevgAs | 7.5 | 15.3 |

WC196LC/pCAB1/evgAS, which is the *evgAS* gene-amplified strain, indicated a significant increase in the accumulation of L-lysine compared to the control WC196LC/pCAB1.

### Example 6: Effect of gadE amplification on an L-lysine-producing strain of a bacterium of the genus Escherichia

### 6-1. Construction of an L-lysine-producing strain in which the gadE gene has been enhanced

A *gadE* gene fragment was excised from the plasmid pMW-gadE containing the *gadE* gene of the *Escherichia coli* W3110 strain created in Example 1, and the fragment was ligated with vector pUC 18 (Takara Shuzo) digested with *Hind*III and *Bam*HI, thus constructing the *gadE*-amplifying plasmid pUC-gadE.

The WC196LC/pCAB1 strain was transformed with the above-mentioned pUC-gadE, thus obtaining an ampicillin-resistant strain. After confirming that the prescribed plasmid had been introduced, a strain into which the *gadE*-amplifying plasmid pUC-gadE was introduced was designated the WC196LC/pCABI/pUC-gadE strain.

*evgAS, ydeO,* or *gadE* may be amplified in the same manner using known L-lysine-producing bacteria other than WC196LC/pCAB1. Amplification may also be performed in any combination of the *evg4S, ydeO,* or *gadE* gene.

The strains created as above were cultured in an LB medium containing 25 mg/L streptomycin and 50 mg/L ampicillin at 37°C until the OD600 reached approx. 0.6.Then, an equal volume of a 40% glycerol solution was added to the culture broth and stirred, then appropriate amounts were dispensed and stored at -80 °C to make the glycerol stock.

### 6-2. Lysine-producing culture

After melting the glycerol stock of these strains, 100 mL of each was homogeneously spread onto an L plate containing 25 mg/L streptomycin and 50 mg/L ampicillin, and the plate was incubated at 37 °C for 24 hours. Approx. 1/8 of the cells on the plate obtained were inoculated into a 20 mL fermentation medium described below with 25 mg/L streptomycin and 50 mL ampicillin in a 500 mL Sakaguchi flask, and cultured at 37 °C for 48 hours using a reciprocal shaking incubator. After the culture, the amount of L-lysine which had accumulated in the medium was measured using a Biotech-analyzer, AS210 (Sakura Seiki). The medium composition used for the culture was as follows.

| | |
|---|---|
| L-lysine-producing medium: | |
| Glucose or Fructose | 40g/L |
| (NH₄)₂SO₄ | 16g/L |
| KH₂PO₄ | 1.0g/L |
| MgSO₄·7H₂O | 1.0g/L |
| FeSO₄·7H₂O | 0.01g/L |
| MnSO₄·7H₂O | 0.01g/L |
| Isoleucine | 0.1g/L |
| Yeast Extract | 2.0g/L |
| CaCO₃ (Japanese Pharmacopoeia) | 30g/L |

Adjusted to pH 7.0 with KOH, autoclaved at 120 °C for 20 min. Glucose and MgSO₄·7H₂O were mixed and sterilized separately. CaCO₃ was added after dry-heat sterilization.

The OD600 and L-lysine accumulated at the 48th hour are shown in Table 4.

**Table 4: Effect of godE amplification on lysine-producing bacterium WC196LC/pCAB1 in glucose culture and fructose culture**

| Strain | Carbon source | OD (600 nm) | L-lysine concentration (g/l) |
|---|---|---|---|
| WC196LC/pCAB1 | Glucose | 8.8 | 8.9 |
| WC196LC/pCABI/pUcgadE | Glucose | 8.6 | 9.2 |
| WC196LC/pCAB1 | Fructose | 7.4 | 9.1 |
| WC196LC/pCABl/pUCgadE | Fructose | 7.3 | 10.1 |

The *godE* gene-amplified strain WC196LC/pCAB1/pUCgadE indicated an increase in the accumulation of L-lysine in both a glucose culture and fructose culture compared to , the control WC196LC/pCAB1, in particular, a significant increase in the accumulation of L-lysine in the fructose culture.

### Industrial Applicability

By using the microorganism described in the present invention, it is possible to efficiently produce an L-amino acid, specifically, L-lysine, L-threonine, and L-tryptophan by fermentation.

### Explanation of sequence listing

SEQ ID NO: 1: primer for amplifying trp operon
SEQ ID NO: 2: primer for amplifying *trp* operon
SEQ ID NO: 3: primer for amplifying *iclR* gene
SEQ ID NO: 4: primer for amplifying *iclR* gene
SEQ ID NO: 5: nucleotide sequence of the *iclR* gene
SEQ ID NO: 6: sequence of an amino acid encoded by the *iclR* gene
SEQ ID NO: 7: nucleotide sequence of the *acca* gene
SEQ ID NO: 8: sequence of an amino acid encoded by the *aceA* gene
SEQ ID NO: 9: nucleotide sequence of the *aceB* gene
SEQ ID NO: 10: sequence of an amino acid encoded by the *aceB* gene
SEQ ID NO: 11: nucleotide sequence of the *aceK* gene
SEQ ID NO: 12: sequence of an amino acid encoded by the *aceK* gene
SEQ ID NO: 13: nucleotide sequence of the *trpA* gene
SEQ ID NO: 14: sequence of an amino acid encoded by the *trpA* gene
SEQ ID NO: 15: nucleotide sequence of the *trpB* gene
SEQ ID NO: 16: sequence of an amino acid encoded by the *trpB* gene
SEQ ID NO: 17: primer for amplifying *evgAS* operon
SEQ. ID NO: 18: primer for amplifying *evgAS* operon
SEQ ID NO: 19: primer for amplifying *gadE* gene
SEQ ID NO: 20: primer for amplifying *gadE* gene
SEQ ID NO: 21: primer for amplifying *ydeO* gene
SEQ ID NO: 22: primer for amplifying *ydeO* gene
SEQ ID NO: 23: nucleotide sequence of the *evgA* gene
SEQ ID NO: 24: amino acid sequence of EvgA
SEQ ID NO: 25: nucleotide sequence of the *evgS* gene
SEQ ID NO: 26: amino acid sequence of EvgS
SEQ ID NO: 27: nucleotide sequence of the *gadE* gene
SEQ ID NO: 28: amino acid sequence of GadE
SEQ ID NO: 29: nucleotide sequence of the *ydeO* gene
SEQ ID NO: 30: amino acid sequence of YdeO
SEQ ID NO: 31: nucleotide sequence of the *cadA* gene
SEQ ID NO: 32: sequence of an amino acid encoded by the *cadA* gene
SEQ ID NO: 33: nucleotide sequence of the IdcC gene
SEQ ID NO: 34: sequence of an amino acid encoded by the *ldcC* gene
SEQ ID NO: 35: PCR primer for disrupting the *cadA* gene
SEQ ID NO: 36: PCR primer for disrupting the *cadA* gene
SEQ ID NO: 37: PCR primer for disrupting the *ldc* gene
SEQ ID NO: 38: PCR primer for disrupting the *ldc* gene

## Claims

1. A method for producing an L-amino acid selected from the group consisting of basic amino acids, hydroxymonoaminocarboxylic acids and aromatic L-amino acids, comprising culturing in a medium a bacterium of the family *Enterobacteriaceae* which has an ability to produce an L-amino acid and which has been modified so as to increase the amount of expression of a gene selected from the group consisting of evgA gene, gadE gene, ydeO gene, and combinations thereof, wherein said genes encode transcription factors involved in the EvgAS two-component system regulon, wherein expression of at least one of the said genes has been increased by increasing the number of copies of each gene, modifying the expression regulatory sequence of said gene, or modifying the bacterium so that the expression of an evgS gene that encodes a sensor kinase is increased by increasing the number of copies of the evgS gene or modifying the expression regulatory sequence of the evgS gene, and collecting the L-amino acid from the medium, wherein the basic amino acid is selected from the group consisting of L-lysine, L-ornithine, L-arginine, L-histidine and L-citrulline, wherein the hydroxymonoaminocarboxylic acid is selected from the group consisting of L-threonine and L-serine, and wherein the aromatic L-amino acid is selected from the group consisting of L-phenylalanine, L-tyrosine, and L-tryptophan.

2. The method according to claim 1, wherein the evgA gene is a DNA selected from the group consisting of:
(a) a DNA which comprises the nucleotide sequence of SEQ ID NO:23, and
(b) a DNA which hybridizes with a nucleotide sequence complementary to the nucleotide sequence of SEQ ID NO:23 under stringent conditions, comprising washing at 60°C at a salt concentration of 0.1 x SSC and 0.1% SDS, and which encodes a protein having transcription factor activity.

3. The method according to claim 1, wherein the gadE gene is a DNA selected from the group consisting of:
(c) a DNA which comprises the nucleotide sequence of SEQ ID NO:27, and
(d) a DNA which hybridizes with a nucleotide sequence complementary to the nucleotide sequence of SEQ ID NO:27 under stringent conditions, comprising washing at 60°C at a salt concentration of 0.1 x SSC and 0.1% SDS, and which encodes a protein having transcription factor activity.

4. The method according to claim 1, wherein the ydeO gene is a DNA selected from the group consisting of:
(e) a DNA which comprises the nucleotide sequence of SEQ ID NO:29, and
(f) a DNA which hybridizes with a nucleotide sequence complementary to the nucleotide sequence of SEQ ID NO:29 under stringent conditions, comprising washing at 60°C at a salt concentration of 0.1 x SSC and 0.1% SDS, and which encodes a protein having transcription factor activity.

5. The method according to claim 1, wherein the evgS gene is a DNA selected from the group consisting of:
(g) a DNA which comprises the nucleotide sequence of SEQ ID:25, and
(h) a DNA which hybridizes with a nucleotide sequence complementary to the nucleotide sequence of SEQ ID NO:25 under stringent conditions, comprising washing at 60°C at a salt concentration of 0.1 x SSC and 0.1% SDS, and which encodes a protein having phosphotransfer activity.

6. The method according to claim 1, wherein the evgA gene encodes a protein selected from the group consisting of:
(A) a protein comprising the amino acid sequence of SEQ ID NO:24,
(B) a protein which comprises the amino acid sequence of SEQ ID NO:24 but includes substitution, deletion, insertion, addition, or inversion of 1 to 20 amino acid residues and has transcription factor activity.

7. The method according to claim 1, wherein the gadE gene encodes a protein selected from the group consisting of:
(C) a protein comprising the amino acid sequence of SEQ ID NO:28, and
(D) a protein which comprises the amino acid sequence of SEQ ID NO: 28 but includes substitution, deletion, insertion, addition, or inversion of 1 to 20 amino acid residues and has transcription factor activity.

8. The method according to claim 1, wherein the ydeO gene encodes a protein selected from the group consisting of:
(E) a protein comprising the amino acid sequence of SEQ ID NO:30;
(F) a protein which comprises the amino acid sequence of SEQ ID NO:30 but includes substitution, deletion, insertion, addition, or inversion of 1 to 20 amino acid residues and has transcription factor activity.

9. The method according to claim 1, wherein the evgS gene encodes a protein selected from the group consisting of:
(G) a protein comprising the amino acid sequence of SEQ ID NO:26;
(H) a protein which comprises the amino acid sequence of SEQ ID NO:26 but includes substitution, deletion, insertion, addition, or inversion of 1 to 20 amino acid residues and has phosphotransfer activity.

10. The method according to any one of claims 1 to 9, wherein the bacterium belongs to the family Enterobacteriaceae and is selected from the group consisting of the genera *Escherichia, Enterobacter, Pantoea, Klebsiella,* and *Serratia.*

11. The method according to any one of claims 1 to 10, wherein the L-amino acid is selected from the group consisting of L-lysine, L-threonine, L-tryptophan, and combinations thereof.

## Patentansprüche

1. Verfahren zur Herstellung einer L-Aminosäure, die ausgewählt ist aus der Gruppe bestehend aus basischen Aminosäuren, Hydroxymonoaminocarboxylsäuren und aromatischen L-Aminosäuren, umfassend das Kultivieren, in einem Medium, eines Bakteriums der Familie Enterobacteriaceae, welches eine Fähigkeit hat, eine L-Aminosäure herzustellen, und welches modifiziert wurde, um die Menge der Expression eines Gens zu erhöhen, das ausgewählt ist aus der Gruppe bestehend aus dem evgA-Gen, dem gadE-Gen, dem ydeO-Gen und Kombinationen davon, wobei die genannten Gene Transkriptionsfaktoren kodieren, die an dem EvgAS-Zwei-Komponenten-System-Regulon beteiligt sind, wobei die Expression mindestens eines der genannten Gene erhöht wurde, indem die Anzahl der Kopien jedes Gens erhöht wurde, indem die Expressions-regulatorische Sequenz des genannten Gens modifiziert wurde, oder indem das Bakterium modifiziert wurde, so dass die Expression eines evgS-Gens, das eine Sensorkinase kodiert, erhöht wird, indem die Anzahl der Kopien des evgS-Gens erhöht wird oder indem die Expressions-regulatorische Sequenz des evgS-Gens modifiziert wird, und das Gewinnen der L-Aminosäure aus dem Medium, wobei die basische Aminosäure ausgewählt ist aus der Gruppe bestehend aus L-Lysin, L-Ornithin, L-Arginin, L-Histidin und L-Citrullin, wobei die Hydroxymonoaminocarboxylsäure ausgewählt ist aus der Gruppe bestehend aus L-Threonin und L-Serin, und wobei die aromatische L-Aminosäure ausgewählt ist aus der Gruppe bestehend aus L-Phenylalanin, L-Tyrosin und L-Tryptophan.

2. Verfahren gemäß Anspruch 1, wobei das evgA-Gen eine DNA ist, die ausgewählt ist aus der Gruppe bestehend aus:
(a) einer DNA, die die Nukleotidsequenz gemäß SEQ ID NO: 23 umfasst, und
(b) einer DNA, die mit einer Nukleotidsequenz, die zu der Nukleotidsequenz gemäß SEQ ID NO: 23 komplementär ist, unter stringenten Bedingungen hybridisiert, die Waschen bei 60°C und eine Salzkonzentration von 0,1 x SSC und 0,1 % SDS umfassen, und welche ein Protein kodiert, das Transkriptionsfaktor-Aktivität besitzt.

3. Verfahren gemäß Anspruch 1, wobei das gadE-Gen eine DNA ist, die ausgewählt ist aus der Gruppe bestehend aus:
(c) einer DNA, die die Nukleotidsequenz gemäß SEQ ID NO: 27 umfasst, und
(d) einer DNA, die mit einer Nukleotidsequenz, die zu der Nukleotidsequenz gemäß SEQ ID NO: 27 komplementär ist, unter stringenten Bedingungen hybridisiert, die Waschen bei 60°C und eine Salzkonzentration von 0,1 x SSC und 0,1 % SDS umfassen, und welche ein Protein kodiert, das Transkriptionsfaktor-Aktivität besitzt.

4. Verfahren gemäß Anspruch 1, wobei das ydeO-Gen eine DNA ist, die ausgewählt ist aus der Gruppe bestehend aus:
(e) einer DNA, die die Nukleotidsequenz gemäß SEQ ID NO: 29 umfasst, und
(f) einer DNA, die mit einer Nukleotidsequenz, die zu der Nukleotidsequenz gemäß SEQ ID NO: 29 komplementär ist, unter stringenten Bedingungen hybridisiert, die Waschen bei 60°C und eine Salzkonzentration von 0,1 x SSC und 0,1 % SDS umfassen, und welche ein Protein kodiert, das Transkriptionsfaktor-Aktivität besitzt.

5. Verfahren gemäß Anspruch 1, wobei das evgS-Gen eine DNA ist, die ausgewählt ist aus der Gruppe bestehend aus:
(g) einer DNA, die die Nukleotidsequenz gemäß SEQ ID NO: 25 umfasst, und
(h) einer DNA, die mit einer Nukleotidsequenz, die zu der Nukleotidsequenz gemäß SEQ ID NO: 25 komplementär ist, unter stringenten Bedingungen hybridisiert, die Waschen bei 60°C und eine Salzkonzentration von 0,1 x SSC und 0,1 % SDS umfassen, und welche ein Protein kodiert, das Phosphotransfer-Aktivität besitzt.

6. Verfahren gemäß Anspruch 1, wobei das evgA-Gen ein Protein kodiert, das ausgewählt ist aus der Gruppe bestehend aus:
(A) einem Protein, das die Aminosäuresequenz gemäß SEQ ID NO: 24 umfasst,
(B) einem Protein, das die Aminosäuresequenz gemäß SEQ ID NO: 24 umfasst, aber Substitution, Deletion, Insertion, Addition oder Inversion von 1 bis 20 Aminosäureresten einschließt und Transkriptionsfaktor-Aktivität besitzt.

7. Verfahren gemäß Anspruch 1, wobei das gadE-Gen ein Protein kodiert, das ausgewählt ist aus der Gruppe bestehend aus:
(C) einem Protein, das die Aminosäuresequenz gemäß SEQ ID NO: 28 umfasst, und
(D) einem Protein, das die Aminosäuresequenz gemäß SEQ ID NO: 28 umfasst, aber Substitution, Deletion, Insertion, Addition oder Inversion von 1 bis 20 Aminosäureresten einschließt und Transkriptionsfaktor-Aktivität besitzt.

8. Verfahren gemäß Anspruch 1, wobei das ydeO-Gen ein Protein kodiert, das ausgewählt ist aus der Gruppe bestehend aus:
(E) einem Protein, das die Aminosäuresequenz gemäß SEQ ID NO: 30 umfasst;
(F) einem Protein, das die Aminosäuresequenz gemäß SEQ ID NO: 30 umfasst, aber Substitution, Deletion, Insertion, Addition oder Inversion von 1 bis 20 Aminosäureresten einschließt und Transkriptionsfaktor-Aktivität besitzt.

9. Verfahren gemäß Anspruch 1, wobei das evgS-Gen ein Protein kodiert, das ausgewählt ist aus der Gruppe bestehend aus:
(G) einem Protein, das die Aminosäuresequenz gemäß SEQ ID NO: 26 umfasst;
(H) einem Protein, das die Aminosäuresequenz gemäß SEQ ID NO: 26 umfasst, aber Substitution, Deletion, Insertion, Addition oder Inversion von 1 bis 20 Aminosäureresten einschließt und Phosphortransfer-Aktivität besitzt.

10. Verfahren gemäß mindestens einem der Ansprüche 1 bis 9, wobei das Bakterium zu der Familie Enterobacteriaceae gehört und ausgewählt ist aus der Gruppe bestehend aus den Gattungen Escherichia, Enterobacter, Pantoea, Klebsiella und Serratia.

11. Verfahren gemäß mindestens einem der Ansprüche 1 bis 10, wobei die L-Aminosäure ausgewählt ist aus der Gruppe bestehend aus L-Lysin, L-Threonin, L-Tryptophan und Kombinationen davon.

## Revendications

1. Procédé de production d'un L-aminoacide choisi dans le groupe consistant en les aminoacides basiques, les acides hydroxymonoamino-carboxyliques et les L-aminoacides aromatiques, comprenant la culture dans un milieu d'une bactérie de la famille des *Enterobacteriaceae* qui a une aptitude à produire un L-aminoacide et qui a été modifiée de manière à augmenter la quantité d'expression d'un gène choisi dans le groupe consistant en le gène evgA, le gène gadE, le gène ydeO et leurs combinaisons, où lesdits gènes codent des facteurs de transcription impliqués dans le régulon du système à deux composants EvgAS, où l'expression d'au moins l'un desdits gènes a été augmentée en augmentant le nombre de copies de chaque gène, modifiant la séquence régulant l'expression dudit gène ou modifiant la bactérie de sorte que l'expression d'un gène evgS qui code une kinase capteur est augmentée en augmentant le nombre de copies du gène evgS ou modifiant la séquence régulant l'expression du gène evgS, et la récupération du L-aminoacide à partir du milieu, où l'aminoacide basique est choisi dans le groupe consistant en la L-lysine, la L-ornithine, la L-arginine, la L-histidine et la L-citrulline, où l'acide hydroxymonoaminocarboxylique est choisi dans le groupe consistant en la L-thréonine et la L-sérine, et où le L-aminoacide aromatique est choisi dans le groupe consistant en la L-phénylalanine, la L-tyrosine et le L-tryptophane.

2. Procédé selon la revendication 1 où le gène *evgA* est un ADN choisi dans le groupe consistant en :
(a) un ADN qui comprend la séquence nucléotidique de SEQ ID NO : 23, et
(b) un ADN qui s'hybride avec une séquence nucléotidique complémentaire de la séquence nucléotidique de SEQ ID NO: 23 dans des conditions stringentes, comprenant un lavage à 60°C à une concentration de sel de 0,1 x SSC et 0,1 % de SDS, et qui code une protéine ayant une activité de facteur de transcription.

3. Procédé selon la revendication 1 où le gène gadE est un ADN choisi dans le groupe consistant en :
(c) un ADN qui comprend la séquence nucléotidique de SEQ ID NO : 27, et
(d) un ADN qui s'hybride avec une séquence nucléotidique complémentaire de la séquence nucléotidique de SEQ ID NO: 27 dans des conditions stringentes, comprenant un lavage à 60°C à une concentration de sel de 0,1 x SSC et 0,1 % de SDS, et qui code une protéine ayant une activité de facteur de transcription.

4. Procédé selon la revendication 1 où le gène ydeO est un ADN choisi dans le groupe consistant en :
(e) un ADN qui comprend la séquence nucléotidique de SEQ ID NO : 29, et
(f) un ADN qui s'hybride avec une séquence nucléotidique complémentaire de la séquence nucléotidique de SEQ ID NO: 29 dans des conditions stringentes, comprenant un lavage à 60°C à une concentration de sel de 0,1 x SSC et 0,1 % de SDS, et qui code une protéine ayant une activité de facteur de transcription.

5. Procédé selon la revendication 1 où le gène evgS est un ADN choisi dans le groupe consistant en :
(g) un ADN qui comprend la séquence nucléotidique de SEQ ID NO : 25, et
(h) un ADN qui s'hybride avec une séquence nucléotidique complémentaire de la séquence nucléotidique de SEQ ID NO : 25 dans des conditions stringentes, comprenant un lavage à 60°C à une concentration de sel de 0,1 x SSC et 0,1 % de SDS, et qui code une protéine ayant une activité de phosphotransfert.

6. Procédé selon la revendication 1 où le gène evgA code une protéine choisie dans le groupe consistant en :
(A) une protéine comprenant la séquence d'aminoacides de SEQ ID NO : 24,
(B) une protéine qui comprend la séquence d'aminoacides de SEQ ID NO : 24 mais inclut une substitution, une délétion, une insertion, une addition ou une inversion de 1 à 20 résidus d'aminoacides et a une activité de facteur de transcription.

7. Procédé selon la revendication 1 où le gène *gadE* code une protéine choisie dans le groupe consistant en :
(C) une protéine comprenant la séquence d'aminoacides de SEQ ID NO : 28, et
(D) une protéine qui comprend la séquence d'aminoacides de SEQ ID NO : 28 mais inclut une substitution, une délétion, une insertion, une addition ou une inversion de 1 à 20 résidus d'aminoacides et a une activité de facteur de transcription.

8. Procédé selon la revendication 1 où le gène *ydeO* code une protéine choisie dans le groupe consistant en :
(E) une protéine comprenant la séquence d'aminoacides de SEQ ID NO : 30 ;
(F) une protéine qui comprend la séquence d'aminoacides de SEQ ID NO : 30 mais qui inclut une substitution, une délétion, une insertion, une addition ou une inversion de 1 à 20 résidus d'aminoacides et a une activité de facteur de transcription.

9. Procédé selon la revendication 1 où le gène *evgS* code une protéine choisie dans le groupe consistant en :
(G) une protéine comprenant la séquence d'aminoacides de SEQ ID NO : 26 ;
(H) une protéine qui comprend la séquence d'aminoacides de SEQ ID NO : 26 mais inclut une substitution, une délétion, une insertion, une addition ou une inversion de 1 à 20 résidus d'aminoacides et a une activité de phosphotransfert.

10. Procédé selon l'une quelconque des revendications 1 à 9 où la bactérie appartient à la famille des *Enterobacteriaceae* et est choisie dans le groupe consistant en les genres *Escherichia, Enterobacter, Pantoea, Klebsiella* et *Serratia.*

11. Procédé selon l'une quelconque des revendications 1 à 10 où le L-aminoacide est choisi dans le groupe consistant en la L-lysine, la L-thréonine, le L-tryptophane et leurs combinaisons.
